# EUROPEAN PATENT APPLICATION

(11) **EP 4 748 857 A1**
(43) Date of publication of application: **27.05.2026**
(21) Application number: 24805420.7
(22) Date of filing: 20.05.2024
(51) Int. Cl.: C07K 16/28, A61P 37/00, A61K 39/00

(54) **ANTI-CD40L/ANTI-CD28 BISPECIFIC ANTIBODY AND USE THEREOF**

(30) Priority: 18.05.2023 KR 20230064576
(71) Applicant: Seoul National University R&DB Foundation, Seoul 08826 (KR)
(72) Inventor: CHOI, Kyungho, Seoul 03080 (KR); CHUNG, Junho, Seoul 03080 (KR); LEE, Youngjae, Seoul 03080 (KR); KIM, Sang Il, Seoul 03080 (KR); KIM, Sujeong, Seoul 03080 (KR); HAN, Jerome, Seoul 03080 (KR); HWANG, Siwon, Seoul 03080 (KR); SHIN, Nara, Seoul 03080 (KR); SEONG, Jihye, Seoul 03080 (KR); LEE, Hae Nim, Seoul 03080 (KR)
(74) Representative: Ullrich & Naumann PartG mbB
(86) International application number: PCT/KR2024/006813
(87) International publication number: WO 2024/237744

(57) **Abstract**

An anti-CD40L/anti-CD28 bispecific antibody of the present application comprises anti-CD40L scFv and anti-CD28 scFv, and thus exhibits no thromboembolic side effects, can exhibit an excellent effect of treating autoimmune diseases and/or graft-versus-host diseases while preventing a T cell activation effect caused by CD28 dimer formation, and can exhibit excellent bispecific antibody physical properties.

## Description

### FIELD OF THE INVENTION

### Cross-reference to Related Application(s)

The present disclosure claims the benefit of priority based on Korean Patent Application No. 10-2023-0064576 filed on May 18, 2023, the entire disclosure of which is incorporated herein by reference.

The present disclosure relates to anti-CD40L antibodies, anti-CD28 antibodies, anti-CD40L/anti-CD28 bispecific antibodies, and uses thereof.

### BACKGROUND ART

There are approximately 80 autoimmune diseases worldwide, and it is estimated that over 300 million people globally suffer from these diseases. Traditionally, autoimmune diseases have been treated using immunosuppressants or anti-inflammatory agents that broadly inhibit the body's immune cells. However, this is a merely symptomatic treatment that inhibits the final tissue damage; that is, such a treatment fails to eliminate the underlying cause, and thus makes it difficult to halt disease progression. Furthermore, the use of broad-spectrum immunosuppressants reduces immunity against external pathogens, which can lead to adverse effects from infectious diseases, thereby posing a challenge for continuous use.

Therefore, there has been active development of therapies (immune tolerance-inducing agents) that selectively inhibit and eliminate immune responses only against specific autoantigens, instead of broad immunosuppression, thereby maintaining immune responses against external pathogens while suppressing autoimmune responses.

Accordingly, antibody therapeutics targeting CD40L and CD28, which are known as representative T lymphocyte co-stimulatory molecules, have been developed. However, it has been reported that conventional anti-CD40L antibodies crosslink with FcγRIIa located on platelets and promote platelet activation, thereby causing an adverse effect of thromboembolic symptoms. Furthermore, conventional anti-CD28 antibodies have been problematic in that they activate T cells solely through dimerization.

The present inventors newly propose novel anti-CD40L antibodies, anti-CD28 antibodies, bispecific antibodies comprising the aforementioned antibodies, and uses thereof as therapeutic agents for autoimmune diseases and/or graft-versus-host disease, wherein the antibodies address the aforementioned problems.

### DISCLOSURE OF INVENTION

### TECHNICAL PROBLEM

In an aspect, there is provided an anti-CD40L/anti-CD28 bispecific antibody comprising:
(1) an anti-CD40L antibody or an antigen-binding fragment thereof as a CD40L-targeting moiety capable of specifically recognizing and/or binding to CD40L protein; and
(2) an anti-CD28 antibody or an antigen-binding fragment thereof as a CD28-targeting moiety capable of specifically recognizing and/or binding to CD28 protein.

In another aspect, there is provided an anti-CD40L antibody or an antigen-binding fragment thereof.

In yet another aspect, there is provided an anti-CD28 antibody or an antigen-binding fragment thereof.

In still yet another aspect, there is provided a pharmaceutical composition for prevention, amelioration, or treatment of one or more diseases selected from the group consisting of autoimmune diseases and graft-versus-host disease, the composition comprising the anti-CD40L/anti-CD28 bispecific antibody, the anti-CD40L antibody or an antigen-binding fragment thereof, and/or the anti-CD28 antibody or an antigen-binding fragment thereof.

In still yet another aspect, there is provided a use of the anti-CD40L/anti-CD28 bispecific antibody, the anti-CD40L antibody or an antigen-binding fragment thereof, and/or the anti-CD28 antibody or an antigen-binding fragment thereof for prevention, amelioration, or treatment of one or more diseases selected from the group consisting of autoimmune diseases and graft-versus-host disease.

In still yet another aspect, there is provided a use of the anti-CD40L/anti-CD28 bispecific antibody, the anti-CD40L antibody or an antigen-binding fragment thereof, and/or the anti-CD28 antibody or an antigen-binding fragment thereof for manufacture of a pharmaceutical composition for prevention, amelioration, or treatment of one or more diseases selected from the group consisting of autoimmune diseases and graft-versus-host disease.

In still yet another aspect, there is provided a method for prevention, amelioration, or treatment of one or more diseases selected from the group consisting of autoimmune diseases and graft-versus-host disease, the method comprising administering, to a subject in need thereof, the anti-CD40L/anti-CD28 bispecific antibody, the anti-CD40L antibody or an antigen-binding fragment thereof, and/or the anti-CD28 antibody or an antigen-binding fragment thereof. The method may further comprise, prior to the administration step, identifying a subject in need of prevention, amelioration, or treatment of one or more diseases selected from the group consisting of autoimmune diseases and graft-versus-host disease.

In a still yet another aspect, there is provided a polynucleotide that encodes the anti-CD40L/anti-CD28 bispecific antibody, the anti-CD40L antibody or an antigen-binding fragment thereof, and/or the anti-CD28 antibody or an antigen-binding fragment thereof.

In a still yet another aspect, there is provided a recombinant vector comprising the polynucleotide. The recombinant vector may be used as an expression vector for the polynucleotide that encodes the anti-CD40L/anti-CD28 bispecific antibody, the anti-CD40L antibody or an antigen-binding fragment thereof, and/or the anti-CD28 antibody or an antigen-binding fragment thereof.

In a still yet another aspect, there is provided a cell comprising the polynucleotide that encodes the anti-CD40L/anti-CD28 bispecific antibody, the anti-CD40L antibody or an antigen-binding fragment thereof, and/or the anti-CD28 antibody or an antigen-binding fragment thereof. The cell may be a recombinant cell transformed with a recombinant vector comprising the polynucleotide.

In a still yet another aspect, there is provided a method for preparing the anti-CD40L/anti-CD28 bispecific antibody, the anti-CD40L antibody or an antigen-binding fragment thereof, and/or the anti-CD28 antibody or an antigen-binding fragment thereof, the method comprising expressing the polynucleotide in a cell. The step of expressing the polynucleotide may be performed by culturing the cell, which comprises the polynucleotide, under conditions that allow for expression of the polynucleotide (for example, in a recombinant vector).

### SOLUTION TO PROBLEM

### Definition of Terms

In the present specification, the expression "consisting of a sequence," "consisting essentially of a sequence," or "comprising a sequence" is intended to encompass all cases where the sequence is included, and is not intended to exclude cases where other sequences are included in addition to the sequence.

In the present specification, the terms "a protein or polypeptide comprising or consisting of an amino acid sequence represented by SEQ ID NO" and "a gene or polynucleotide comprising or consisting of a nucleotide sequence represented by SEQ ID NO" may refer to a protein (or polypeptide) or a gene (or polynucleotide) which consists essentially of the amino acid sequence or nucleotide sequence, or has a sequence identity of at least 90%, at least 91%, at least 92%, at least 93%, at least 94%, at least 95%, at least 96%, at least 97%, at least 98%, or at least 99% to the amino acid sequence or nucleotide sequence while retaining its inherent activity and/or function.

The term "antibody" as used herein may encompass a wide range of polypeptides that are biochemically distinguishable. Those skilled in the art will understand that heavy chains are classified as gamma, mu, alpha, delta, or epsilon (y, µ, α, δ, ε), some of which have subclasses (for example, γ1 to γ4), and that light chains are classified as kappa or lambda (κ, λ). It is the nature of this heavy chain that determines the "class" of an antibody as IgG, IgM, IgA, IgD, or IgE, respectively. Immunoglobulin subclasses (isotypes), such as IgG1, IgG2, IgG3, IgG4, and the like, are well characterized and known to confer functional specialization.

An intact antibody comprises two full-length light chains and two full-length heavy chains, wherein each light chain may be linked to the heavy chain by a disulfide bond. The antibody has a heavy chain constant region and a light chain constant region. The heavy chain constant region is of the gamma (γ), mu (µ), alpha (α), delta (δ), or epsilon (ε) type, which may be further subtyped as gamma 1 (γ1), gamma 2 (γ2), gamma 3 (γ3), gamma 4 (γ4), alpha 1 (α1), or alpha 2 (α2). The light chain constant region may be of the kappa (κ) or lambda (λ) type.

The term "heavy chain" may refer to a full-length heavy chain or a fragment thereof, comprising a variable region V_{H}, which comprises an amino acid sequence sufficient to confer specificity to an antigen, three constant regions C_{H1}, C_{H2}, and C_{H3}, and a hinge region. The term "light chain" may refer to a full-length light chain or a fragment thereof, comprising a variable region V_{L}, which comprises an amino acid sequence sufficient to confer specificity to an antigen, and a constant region C_{L}.

The term "complementarity-determining region (CDR)" refers to an amino acid sequence found in the hypervariable region of the heavy or light chain of an immunoglobulin. The heavy and light chains may each comprise three CDRs (CDRH1, CDRH2, and CDRH3; and CDRL1, CDRL2, and CDRL3). The CDRs may provide residues that play a crucial role in binding of an antibody to an antigen or epitope. The term "specifically binds" or "specifically recognized" is well known to those skilled in the art and indicates that an antibody and an antigen interact in a specific manner to induce immunological activity.

The antibody may be an animal-derived antibody (for example, mouse-derived antibody or chicken-derived antibody), a chimeric antibody (for example, mouse-human chimeric antibody or chicken-human chimeric antibody), or a humanized antibody. The antibody or antigen-binding fragment may be isolated from a living organism or non-naturally occurring. The antibody or antigen-binding fragment may be produced recombinantly or synthetically (not naturally produced).

In another example, the antibody may be derived (isolated) from any animal, including mammals, such as human, birds and the like. For example, the antibody may be an antibody from human, mouse, donkey, sheep, rabbit, goat, guinea pig, camel, horse, or chicken. Here, a human antibody is an antibody having the amino acid sequence of a human immunoglobulin, and includes antibodies isolated from a human immunoglobulin library or antibodies isolated from an animal that has been transduced to express one or more human immunoglobulins and does not express any endogenous immunoglobulin.

The antibody may be a monoclonal antibody or a polyclonal antibody, for example, it may be a monoclonal antibody. Monoclonal antibodies may be produced by methods widely known in the art. For example, the monoclonal antibodies may be prepared using phage display techniques.

Animal-derived antibodies produced by immunizing animals with a desired antigen may typically induce immune rejection responses when administered to humans for therapeutic purposes. To suppress such immune rejection responses, chimeric antibodies have been developed. Chimeric antibodies are formed by replacing the constant regions of animal-derived antibodies, which cause anti-isotype responses, with human antibody constant regions using genetic engineering methods. Chimeric antibodies exhibit significantly improved anti-isotype responses compared to animal-derived antibodies; however, potential side effects due to anti-idiotype responses still exist since animal-derived amino acids are still present in the variable regions of the chimeric antibodies. Humanized antibodies have been developed to mitigate these side effects. The humanized antibody is produced by grafting CDRs (complementarity-determining regions), which are located in the variable region of a chimeric antibody and play a crucial role in antigen binding, onto a human antibody framework.

As used herein, the term "antigen-binding fragment" refers to a fragment derived from a complete immunoglobulin structure that comprises a portion capable of binding to an antigen, such as CDRs. For example, the antigen-binding fragment may be, but is not limited to, scFv, (scFv)₂, Fab, Fab', or F(ab')₂. In the present disclosure, the antigen-binding fragment may be an antibody-derived fragment that comprises at least one complementarity determining region selected from the group consisting of, for example, scFv, (scFv)₂, scFv-Fc, Fab, Fab', and F(ab')₂.

In an example, the antigen-binding fragment may be an antigen-binding fragment lacking an Fc region, such as, but not limited to, scFv, Fv, (scFv)₂, Fab, Fab', F(ab')₂, or a single domain antibody lacking an Fc region.

In an example, the antigen-binding fragment may be in the form of an antigen-binding fragment in a monovalent antibody form, such as, but not limited to, scFv, Fv, Fab, Fab', or a single domain antibody in a monovalent antibody form.

Among the antigen-binding fragments, Fab has a structure composed of variable regions of light and heavy chains, a constant region of the light chain, and the first constant region (CH1) of the heavy chain, and possesses a single antigen-binding site.

Fab' differs from Fab in that it has a hinge region which comprises one or more cysteine residues at the C terminus of the heavy chain CH1 domain. F(ab')2 antibodies are formed through disulfide bonds between cysteine residues in the hinge regions of Fab'.

Fv is a minimal antibody fragment having only a heavy chain variable region and a light chain variable region. Recombinant techniques for producing Fv fragments are well known in the art. A two-chain Fv has a structure in which a heavy chain variable region and a light chain variable region are connected by non-covalent bonding. A single-chain Fv typically has a structure in which a heavy chain variable region and a light chain variable region are covalently linked via a peptide linker or directly connected at the C terminus, thereby forming a dimer-like structure similar to two-chain Fv.

The antigen-binding fragments may be obtained using proteolytic enzymes (for example, Fab is obtained in a case where a whole antibody is subjected to restricted cleavage with papain, while an F(ab')₂ fragment is obtained in a case where a whole antibody is subjected to restricted cleavage with pepsin), or may be produced by gene recombination techniques.

The term "hinge region" refers to a region included in the heavy chain of an antibody, which exists between CH1 and CH2 regions, and functions to provide flexibility to an antigen-binding site within the antibody.

The immunoglobulin (for example, human immunoglobulin) or antibody molecule of the present disclosure may be of any type (for example, IgG, IgE, IgM, IgD, IgA, IgY, or the like), a class (for example, IgG1, IgG2, IgG3, IgG4, IgA1, IgA2, or the like), or a subclass of an immunoglobulin molecule.

In an antibody or antibody fragment, portions (for example, constant regions) excluding CDRs or variable regions may be derived from human antibodies. In particular, these portions may be derived from, for example, IgG, IgA, IgD, IgE, IgM, or IgY, such as IgG1, IgG2, IgG3, or IgG4.

As used herein, "peptide linker" may refer to an oligopeptide comprising 1 to 100 amino acids, in particular 2 to 50 amino acids, each of which may be any type of amino acid without limitation. Any conventional peptide linker may be used, with or without appropriate modification, to achieve a particular purpose. In certain embodiments, the peptide linker may comprise, for example, Gly, Asn, and/or Ser residues, and/or may comprise neutral amino acid residue(s) such as Thr and/or Ala. Suitable amino acid sequences for the peptide linker may be known in the art. In the related art, the length of the peptide linker may be appropriately determined as long as the function of the antibody and/or scFv is not affected. For example, the peptide linker may be formed of 1 to 100 amino acids, 2 to about 50 amino acids, or 5 to 25 amino acids, each of which may be selected from the group consisting of Gly, Asn, Ser, Thr, and Ala. In an embodiment, the peptide linker may be represented by (GₘSₗ)ₙ (where m, l, and n are the number of "G", "S", and "(GₘSₗ)", respectively, and each may be independently selected from an integer of about 1 to about 10, in particular, 1, 2, 3, 4, 5, 6, 7, 8, 9, or 10). In an example, the peptide linker may comprise the amino acid sequence GQSSRSSGGGGSSGGGGS (SEQ ID NO: 93) or GQSSRSSSGGGSSGGGGS (SEQ ID NO: 118).

### CD40L-targeting moiety

CD40L is a member of the TNF family of molecules which is primarily expressed on activated T cells (including Th0, Th1, and Th2 subtypes) and is known to form homotrimers similarly to other members of this family. Further, CD40L has been found to be expressed on mast cells, and activated basophils and eosinophils. CD40L binds to CD40 receptor (CD40R) on antigen-presenting cells (APCs), which leads to many effects depending on a target cell type. In general, CD40L plays the role of a costimulatory molecule and induces activation in APC in association with T cell receptor stimulation by MHC molecules on the APC.

The term "CD40L" may refer to both full-length CD40L and soluble fragments, for example, extracellular domain forms of CD40L resulting from proteolysis. Amino acid sequences of membrane-bound and soluble forms of human CD40L (Swissprot: P29965) may be obtained from known databases.

CD40L (also known as CD154, CD40 ligand, gp39 or TBAM) may be a 33 kDa, type II membrane glycoprotein (Swiss-ProtAcc-No P29965). In addition, shorter 18 kDa CD40L soluble forms (also known as sCD40L or soluble CD40L) exist. These soluble forms of CD40L are generated by proteolytic processing of a membrane-bound protein, but the cellular activity of the soluble species is weak in the absence of higher order oligomerization (for example, trimerization).

CD40L (Cluster of Differentiation 40 ligand) is a type II transmembrane protein with a variable molecular weight ranging from 32 to 39 kDa due to post-translational modifications. CD40L belongs to the tumor necrosis factor superfamily and exists as a CD40L trimer due to its sandwich extracellular structure. CD40L is primarily expressed on activated T cells, and is also known to be expressed on activated B cells and platelets.

The CD40L may be, but is not limited to, human CD40L, mouse CD40L, monkey CD40L, donkey CD40L, sheep CD40L, rabbit CD40L, goat CD40L, guinea pig CD40L, camel CD40L, horse CD40L, or chicken CD40L.

In an example, the anti-CD40L antibody or an antigen-binding fragment thereof may exhibit an advantageous effect of preventing thrombotic adverse events by not comprising an Fc region.

In an example, the antigen-binding fragment of the anti-CD40L antibody may not comprise an Fc region and/or may be in a monovalent antibody form.

In an example, the antigen-binding fragment of the anti-CD40L antibody may be an antigen-binding fragment lacking an Fc region, such as, but not limited to, scFv, Fv, (scFv)₂, Fab, Fab', F(ab')₂, or a single domain antibody lacking an Fc region.

In an example, the anti-CD40L antibody or an antigen-binding fragment thereof may be an anti-CD40L scFv.

In an aspect, the anti-CD40L antibody or an antigen-binding fragment thereof may comprise:
L-CDR1 comprising the amino acid sequence of SEQ ID NO: 1, 2, or 3;
L-CDR2 comprising the amino acid sequence of SEQ ID NO: 4, 5, or 6;
L-CDR3 comprising the amino acid sequence of SEQ ID NO: 7, 8, or 9;
H-CDR1 comprising the amino acid sequence of SEQ ID NO: 10, 11, or 12;
H-CDR2 comprising the amino acid sequence of SEQ ID NO: 13, 14, or 15; and
H-CDR3 comprising the amino acid sequence of SEQ ID NO: 16, 17, or 18.

The amino acid sequences of the complementarity determining regions (CDRs) of the anti-CD40L antibody or an antigen-binding fragment thereof are exemplified in Table 1 below:

**[Table 1]**

| Region | Sequence | SEQ ID NO |
|---|---|---|
| L-CDR1 | SGGYSSYGIYYYG | 1 |
| L-CDR1 | SGDSSWYG | 2 |
| L-CDR1 | SGGSGSYG | 3 |
| L-CDR2 | NGNKRPS | 4 |
| L-CDR2 | ANTNRPS | 5 |
| L-CDR2 | WDDERPS | 6 |
| L-CDR3 | GSGDSSSDSGI | 7 |
| L-CDR3 | GSADSTYTAA | 8 |
| L-CDR3 | GGYDDSSYAGI | 9 |
| H-CDR1 | GFTFSSYGMN | 10 |
| H-CDR1 | GFDFSSYDMN | 11 |
| H-CDR1 | GFTFSSYGMQ | 12 |
| H-CDR2 | AISNDGSSTSYAPAVKG | 13 |
| H-CDR2 | GIYDDGSGTVYAPAVKG | 14 |
| H-CDR2 | AIDDDGSSTNYGAAVKG | 15 |
| H-CDR3 | ESTNGGWVADDIDA | 16 |
| H-CDR3 | GGGADNIDA | 17 |
| H-CDR3 | DGIIYYWNADAGYIDA | 18 |

For example, the anti-CD40L antibody or an antigen-binding fragment thereof may comprise: (1) L-CDR1 comprising the amino acid sequence of SEQ ID NO: 1, L-CDR2 comprising the amino acid sequence of SEQ ID NO: 4, L-CDR3 comprising the amino acid sequence of SEQ ID NO: 7, H-CDR1 comprising the amino acid sequence of SEQ ID NO: 10, H-CDR2 comprising the amino acid sequence of SEQ ID NO: 13, and H-CDR3 comprising the amino acid sequence of SEQ ID NO: 16;
(2) L-CDR1 comprising the amino acid sequence of SEQ ID NO: 2, L-CDR2 comprising the amino acid sequence of SEQ ID NO: 5, L-CDR3 comprising the amino acid sequence of SEQ ID NO: 8, H-CDR1 comprising the amino acid sequence of SEQ ID NO: 11, H-CDR2 comprising the amino acid sequence of SEQ ID NO: 14, and H-CDR3 comprising the amino acid sequence of SEQ ID NO: 17; or
(3) L-CDR1 comprising the amino acid sequence of SEQ ID NO: 3, L-CDR2 comprising the amino acid sequence of SEQ ID NO: 6, L-CDR3 comprising the amino acid sequence of SEQ ID NO: 9, H-CDR1 comprising the amino acid sequence of SEQ ID NO: 12, H-CDR2 comprising the amino acid sequence of SEQ ID NO: 15, and H-CDR3 comprising the amino acid sequence of SEQ ID NO: 18.

In an aspect, the anti-CD40L antibody or an antigen-binding fragment thereof may comprise:
L-CDR1 comprising the amino acid sequence of SEQ ID NO: 1 or 2;
L-CDR2 comprising the amino acid sequence of SEQ ID NO: 4 or 5;
L-CDR3 comprising the amino acid sequence of SEQ ID NO: 7 or 8;
H-CDR1 comprising the amino acid sequence of SEQ ID NO: 10 or 11;
H-CDR2 comprising the amino acid sequence of SEQ ID NO: 13 or 14; and
H-CDR3 comprising the amino acid sequence of SEQ ID NO: 16 or 17.

For example, the anti-CD40L antibody or an antigen-binding fragment thereof may comprise:
(1) L-CDR1 comprising the amino acid sequence of SEQ ID NO: 1, L-CDR2 comprising the amino acid sequence of SEQ ID NO: 4, L-CDR3 comprising the amino acid sequence of SEQ ID NO: 7, H-CDR1 comprising the amino acid sequence of SEQ ID NO: 10, H-CDR2 comprising the amino acid sequence of SEQ ID NO: 13, and H-CDR3 comprising the amino acid sequence of SEQ ID NO: 16; or
(2) L-CDR1 comprising the amino acid sequence of SEQ ID NO: 2, L-CDR2 comprising the amino acid sequence of SEQ ID NO: 5, L-CDR3 comprising the amino acid sequence of SEQ ID NO: 8, H-CDR1 comprising the amino acid sequence of SEQ ID NO: 11, H-CDR2 comprising the amino acid sequence of SEQ ID NO: 14, and H-CDR3 comprising the amino acid sequence of SEQ ID NO: 17.

The anti-CD40L antibody or an antigen-binding fragment thereof may comprise: a light chain variable region that comprises L-CDR1 comprising the amino acid sequence of SEQ ID NO: 1, 2, or 3; L-CDR2 comprising the amino acid sequence of SEQ ID NO: 4, 5, or 6; and L-CDR3 comprising the amino acid sequence of SEQ ID NO: 7, 8, or 9; and
a heavy chain variable region that comprises H-CDR1 comprising the amino acid sequence of SEQ ID NO: 10, 11, or 12; H-CDR2 comprising the amino acid sequence of SEQ ID NO: 13, 14, or 15; and H-CDR3 comprising the amino acid sequence of SEQ ID NO: 16, 17, or 18.

In an example, the amino acid sequences of the variable region frameworks of the anti-CD40L antibody or an antigen-binding fragment thereof are exemplified in Table 2 below:

**[Table 2]**

| Region | Sequence | SEQ ID NO |
|---|---|---|
| L-FR1 | ALTQPSSVSANLGGTVKITC | 19 |
| L-FR1 | ELTQDPAVSVALGQTVRITC | 20 |
| L-FR1 | LTQASSVSANLGGTVEITC | 21 |
| L-FR1 | LTQPSSVSANPGETVKITC | 22 |
| L-FR2 | WYQQKSPGSAPVTLIY | 23 |
| L-FR2 | WYQQKPGQAPVTVIY | 24 |
| L-FR3 | NIPSRFSGSTSGSTNTLTITGVQVDDEAVYFC | 25 |
| L-FR3 | GIPDRFSGSSSGNTASLTITGAQAEDEADYYC | 26 |
| L-FR3 | DIPSRFSGSTSGSTGTLTITGVQADDEAVYYC | 27 |
| L-FR3 | DIPSRFSGSKSGSTATLTITGVQAEDEAVYFC | 28 |
| L-FR4 | FGAGTTLTVL | 29 |
| L-FR4 | FGGGTKLTVL | 30 |
| L-FR4 | FGAGTTLTVL | 31 |
| H-FR1 | AVTLDESGGGLQTPGGALSLVCKAS | 32 |
| H-FR1 | EVQLVESGGGLVQPGGSLRLSCAAS | 33 |
| H-FR1 | EVQLVESGGGLVQPGGSLRLICAAS | 34 |
| H-FR1 | AVTLDESGGGLQTPGGTLSLVCKAS | 35 |
| H-FR1 | AVTLDESGGGLHTPGGALSLVCKAS | 36 |
| H-FR2 | WVRQAPGKGLEWVG | 37 |
| H-FR2 | WVRQAPGKGLEWVS | 38 |
| H-FR2 | WVRQAPGKGLEYVA | 39 |
| H-FR2 | WVRQAPGKGLEFVA | 40 |
| H-FR3 | RATISRDNGQSTVRLQLNNLRAEDTATYYCAK | 41 |
| H-FR3 | RFTISRDNSKNTLYLQMNSLRAEDTAVYYCAK | 42 |
| H-FR3 | RATISRDNGQSTVRLQLNNLRAEDTGTYYCTR | 43 |
| H-FR3 | RATISRDNGQSTVRLQLNNLRAEDTGTYYCAK | 44 |
| H-FR4 | WGHGTEVIVSS | 45 |
| H-FR4 | WGQGTLVTVSS | 46 |

For example, the anti-CD40L antibody or an antigen-binding fragment thereof may comprise:
a light chain variable region comprising the amino acid sequence of SEQ ID NO: 47, 48, 49, 50, or 128; and
a heavy chain variable region comprising the amino acid sequence of SEQ ID NO: 51, 52, 53, 54, 55, 56, or 57.

For example, the anti-CD40L antibody or an antigen-binding fragment thereof may comprise:
a light chain variable region comprising the amino acid sequence of SEQ ID NO: 47, 48, 49, or 128; and
a heavy chain variable region comprising the amino acid sequence of SEQ ID NO: 51, 52, 53, 54, 55, or 56.

The amino acid sequences of the variable regions of the anti-CD40L antibody or an antigen-binding fragment thereof are exemplified in Table 3 below:

**[Table 3]**

| Region | Sequence | SEQ ID NO |
|---|---|---|
| VL | ALTQPSSVSANLGGTVKITCSGGYSSYGIYYYGWYQQKSPGSA PVTLIYNGNKRPSNIPSRFSGSTSGSTNTLTITGVQVDDEAVY FCGSGDSSSDSGIFGAGTTLTVL | 47 |
| VL | ELTQDPAVSVALGQTVRITCSGGYSSYGIYYYGWYQQKPGQAP VTVIYNGNKRPSGIPDRFSGSSSGNTASLTITGAQAEDEADYY CGSGDSSSDSGIFGGGTKLTVL | 48 |
| VL | ELTQDPAVSVALGQTVRITCSGGYSSYGIYYYGWYQQKPGQAP VLVIYNGNKRPSGIPDRFSGSSSGNTASLTITGAQAEDEADYY CGSGDSSSDSGIFGGGTKLTVL | 128 |
| VL | LTQASSVSANLGGTVEITCSGDSSWYGWYQQKSPGSAPVTLIY ANTNRPSDIPSRFSGSTSGSTGTLTITGVQADDEAVYYCGSAD STYTAAFGAGTTLTVL | 49 |
| VL | LTQPSSVSANPGETVKITCSGGSGSYGWYQQKSPGSAPVTLIY WDDERPSDIPSRFSGSKSGSTATLTITGVQAEDEAVYFCGGYD DSSYAGIFGAGTTLTVL | 50 |
| VH | AVTLDESGGGLQTPGGALSLVCKASGFTFSSYGMNWVRQAPGK GLEWVGAISNDGSSTSYAPAVKGRATISRDNGQSTVRLQLNNL RAEDTATYYCAKESTNGGWVADDIDAWGHGTEVIVSS | 51 |
| VH | EVQLVESGGGLVQPGGSLRLSCAASGFTFSSYGMNWVRQAPGK GLEWVSAISNDGSSTSYAPAVKGRFTISRDNSKNTLYLQMNSL RAEDTAVYYCAKESTNGGWVADDIDAWGQGTLVTVSS | 52 |
| VH | EVQLVESGGGLVQPGGSLRLSCAASGFTFSSYGMNWVRQAPGK GLEWVGAISNDGSSTSYAPAVKGRFTISRDNSKNTLYLQMNSL RAEDTAVYYCAKESTNGGWVADDIDAWGQGTLVTVSS | 53 |
| VH | EVQLVESGGGLVQPGGSLRLICAASGFTFSSYGMNWVRQAPGK | 54 |
| | GLEWVSAISNDGSSTSYAPAVKGRFTISRDNSKNTLYLQMNSL RAEDTAVYYCAKESTNGGWVADDIDAWGQGTLVTVSS | |
| VH | EVQLVESGGGLVQPGGSLRLICAASGFTFSSYGMNWVRQAPGK GLEWVGAISNDGSSTSYAPAVKGRFTISRDNSKNTLYLQMNSL RAEDTAVYYCAKESTNGGWVADDIDAWGQGTLVTVSS | 55 |
| VH | AVTLDESGGGLQTPGGTLSLVCKASGFDFSSYDMNWVRQAPGK GLEYVAGIYDDGSGTVYAPAVKGRATISRDNGQSTVRLQLNNL RAEDTGTYYCTRGGGADNIDAWGHGTEVIVSS | 56 |
| VH | AVTLDESGGGLHTPGGALSLVCKASGFTFSSYGMQWVRQAPGK GLEFVAAIDDDGSSTNYGAAVKGRATISRDNGQSTVRLQLNNL RAEDTGTYYCAKDGIIYYWNADAGYIDAWGHGTEVIVSS | 57 |

In the light chain and heavy chain variable regions of Table 3, the respective complementarity determining regions are underlined.

In an example, the anti-CD40L antibody or an antigen-binding fragment thereof may comprise:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48; and a heavy chain variable region comprising the amino acid sequence of SEQ ID NO: 52, 53, 54, or 55;
(3) a light chain variable region comprising the amino acid sequence of SEQ ID NO: 49; and a heavy chain variable region comprising the amino acid sequence of SEQ ID NO: 56; or
(4) a light chain variable region comprising the amino acid sequence of SEQ ID NO: 50; and a heavy chain variable region comprising the amino acid sequence of SEQ ID NO: 57.

In an example, the anti-CD40L antibody or an antigen-binding fragment thereof may comprise:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48; and a heavy chain variable region comprising the amino acid sequence of SEQ ID NO: 52, 53, 54, or 55; or
(3) a light chain variable region comprising the amino acid sequence of SEQ ID NO: 49; and a heavy chain variable region comprising the amino acid sequence of SEQ ID NO: 56.

In an example, the anti-CD40L antibody or an antigen-binding fragment thereof may be a single-chain variable fragment (scFv) comprising:
a light chain variable region that comprises L-CDR1 comprising the amino acid sequence of SEQ ID NO: 1, 2, or 3; L-CDR2 comprising the amino acid sequence of SEQ ID NO: 4, 5, or 6; and L-CDR3 comprising the amino acid sequence of SEQ ID NO: 7, 8, or 9; and
a heavy chain variable region that comprises H-CDR1 comprising the amino acid sequence of SEQ ID NO: 10, 11, or 12; H-CDR2 comprising the amino acid sequence of SEQ ID NO: 13, 14, or 15; and H-CDR3 comprising the amino acid sequence of SEQ ID NO: 16, 17, or 18.

In an example, the anti-CD40L scFv may comprise:
a light chain variable region that comprises L-CDR1 comprising the amino acid sequence of SEQ ID NO: 1 or 2; L-CDR2 comprising the amino acid sequence of SEQ ID NO: 4 or 5; and L-CDR3 comprising the amino acid sequence of SEQ ID NO: 7 or 8; and
a heavy chain variable region that comprises H-CDR1 comprising the amino acid sequence of SEQ ID NO: 10 or 11; H-CDR2 comprising the amino acid sequence of SEQ ID NO: 13 or 14; and H-CDR3 comprising the amino acid sequence of SEQ ID NO: 16 or 17.

In an example, the anti-CD40L scFv may comprise:
a light chain variable region comprising the amino acid sequence of SEQ ID NO: 47, 48, 49, 50, or 128; and
a heavy chain variable region comprising the amino acid sequence of SEQ ID NO: 51, 52, 53, 54, 55, 56, or 57.

In an example, the anti-CD40L scFv may comprise:
a light chain variable region comprising the amino acid sequence of SEQ ID NO: 47, 48, 49, or 128; and
a heavy chain variable region comprising the amino acid sequence of SEQ ID NO: 51, 52, 53, 54, 55, or 56.

In an example, the anti-CD40L scFv may comprise:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48 or 128; and a heavy chain variable region comprising the amino acid sequence of SEQ ID NO: 52, 53, 54, or 55;
(3) a light chain variable region comprising the amino acid sequence of SEQ ID NO: 49; and a heavy chain variable region comprising the amino acid sequence of SEQ ID NO: 56; or
(4) a light chain variable region comprising the amino acid sequence of SEQ ID NO: 50; and a heavy chain variable region comprising the amino acid sequence of SEQ ID NO: 57.

In an example, the anti-CD40L scFv may comprise:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48 or 128; and a heavy chain variable region comprising the amino acid sequence of SEQ ID NO: 52, 53, 54, or 55; or
(3) a light chain variable region comprising the amino acid sequence of SEQ ID NO: 49; and a heavy chain variable region comprising the amino acid sequence of SEQ ID NO: 56.

Here, the light chain variable region and the heavy chain variable region may be linked to each other, in any order, either directly (for example, without a linker) or via a peptide linker. The peptide linker is as described above.

For example, the anti-CD40L scFv may comprise a light chain variable region and a heavy chain variable region in an N- to C-terminal direction. Alternatively, the anti-CD40L scFv may comprise a heavy chain variable region and a light chain variable region in an N- to C-terminal direction.

In an example, the anti-CD40L scFv may comprise the amino acid sequence of SEQ ID NO: 58, 59, 60, 61, 62, 63, or 64.

In an example, the anti-CD40L scFv may comprise the amino acid sequence of SEQ ID NO: 58, 59, 60, 61, 62, or 63.

### CD28-targeting moiety

The CD28 (Cluster of Differentiation 28) molecule is a 44 kDa glycoprotein specifically expressed on the surface of T cells and is known to be an essential factor for T cell activation and survival. CD28 co-stimulation-mediated cellular signaling is one of the key factors regulating T cell activation and is essential for T cell responses to antigens and T cell-mediated B cell responses.

The CD28 may be human CD28, mouse CD28, monkey CD28, donkey CD28, sheep CD28, rabbit CD28, goat CD28, guinea pig CD28, camel CD28, horse CD28, or chicken CD28, but is not limited thereto.

In an example, the antigen-binding fragment of the anti-CD28 antibody may be in a monovalent antibody form, which can, in turn, prevent CD28 dimerization to inhibit T cell activation.

In an example, the antigen-binding fragment of the anti-CD28 antibody may not comprise an Fc region and/or may be in a monovalent antibody form.

In an example, the antigen-binding fragment of the anti-CD28 antibody may be in the form of an antigen-binding fragment in a monovalent antibody form, such as, but not limited to, scFv, Fv, Fab, Fab', or a single domain antibody in a monovalent antibody form.

In an example, the antigen-binding fragment of the anti-CD28 antibody may be an anti-CD28 scFv.

In another aspect, the anti-CD28 antibody or an antigen-binding fragment thereof may comprise:
L-CDR1 comprising the amino acid sequence of SEQ ID NO: 65;
L-CDR2 comprising the amino acid sequence of SEQ ID NO: 66;
L-CDR3 comprising the amino acid sequence of SEQ ID NO: 67;
H-CDR1 comprising the amino acid sequence of SEQ ID NO: 68 or 69;
H-CDR2 comprising the amino acid sequence of SEQ ID NO: 70; and
H-CDR3 comprising the amino acid sequence of SEQ ID NO: 71.

The amino acid sequences of the complementarity determining regions (CDRs) of the anti-CD28 antibody or an antigen-binding fragment thereof are exemplified in Table 4 below:

**[Table 4]**

| Region | Sequence | SEQ ID NO |
|---|---|---|
| L-CDR1 | SGGGSNNYG | 65 |
| L-CDR2 | YNDKRPS | 66 |
| L-CDR3 | GSRDSSHDGI | 67 |
| H-CDR1 | GFTFSTFNMF | 68 |
| H-CDR1 | GFTFSSYAMH | 69 |
| H-CDR2 | QISSNDGSWTGYGAAVKG | 70 |
| H-CDR3 | AAGGYNVCGYGCGGDYIDA | 71 |

For example, the anti-CD28 antibody or an antigen-binding fragment thereof may comprise:
(1) L-CDR1 comprising the amino acid sequence of SEQ ID NO: 65, L-CDR2 comprising the amino acid sequence of SEQ ID NO: 66, L-CDR3 comprising the amino acid sequence of SEQ ID NO: 67, H-CDR1 comprising the amino acid sequence of SEQ ID NO: 68, H-CDR2 comprising the amino acid sequence of SEQ ID NO: 70, and H-CDR3 comprising the amino acid sequence of SEQ ID NO: 71; or
(2) L-CDR1 comprising the amino acid sequence of SEQ ID NO: 65, L-CDR2 comprising the amino acid sequence of SEQ ID NO: 66, L-CDR3 comprising the amino acid sequence of SEQ ID NO: 67, H-CDR1 comprising the amino acid sequence of SEQ ID NO: 69, H-CDR2 comprising the amino acid sequence of SEQ ID NO: 70, and H-CDR3 comprising the amino acid sequence of SEQ ID NO: 71.

The anti-CD28 antibody or an antigen-binding fragment thereof may comprise: a light chain variable region that comprises L-CDR1 comprising the amino acid sequence of SEQ ID NO: 65, L-CDR2 comprising the amino acid sequence of SEQ ID NO: 66, and L-CDR3 comprising the amino acid sequence of SEQ ID NO: 67; and
a heavy chain variable region that comprises H-CDR1 comprising the amino acid sequence of SEQ ID NO: 68 or 69, H-CDR2 comprising the amino acid sequence of SEQ ID NO: 70, and H-CDR3 comprising the amino acid sequence of SEQ ID NO: 71.

In an example, the amino acid sequences of the variable region frameworks of the anti-CD28 antibody or an antigen-binding fragment thereof are exemplified in Table 5 below:

**[Table 5]**

| Region | Sequence | SEQ ID NO |
|---|---|---|
| L-FR1 | MTQPSSVSANPGETVKITC | 72 |
| L-FR1 | LTQPSSVSANPGETVKITC | 73 |
| L-FR2 | WYQQKSPGSAPVTVIY | 74 |
| L-FR2 | WYQQKPGSAPVTVIY | 75 |
| L-FR3 | GIPSRFSGSTSGSTGTLTITGVRAEDEAVYFC | 76 |
| L-FR4 | FGAGTTLTVL | 77 |
| L-FR4 | FGGGTKLTVL | 78 |
| H-FR1 | AVTLDESGGGLQTPGGALSLVCKAS | 79 |
| H-FR1 | PVKLVESGGGLVTPGGSLRLSCSAS | 80 |
| H-FR2 | WVRQAPGKGLEYVA | 81 |
| H-FR2 | WVRQAPGKGLEYVG | 82 |
| H-FR3 | RATISRDNGQSTVRLQLNNLRAEDTATYYCAK | 83 |
| H-FR3 | RFTISRDNSKSTLYLQMNSLRAEDTAVYYCAK | 84 |
| H-FR4 | WGHGTEVIVSSTS | 85 |
| H-FR4 | WGHGTQVTVSSTS | 86 |

For example, the anti-CD28 antibody or an antigen-binding fragment thereof may comprise:
a light chain variable region comprising the amino acid sequence of SEQ ID NO: 87, 88, 98, or 100; and
a heavy chain variable region comprising the amino acid sequence of SEQ ID NO: 89, 90, 99, or 101.

The amino acid sequences of the variable regions of the anti-CD28 antibody or an antigen-binding fragment thereof are exemplified in Table 6 below:

**[Table 6]**

| Region | Sequence | SEQ ID NO |
|---|---|---|
| VL | MTQPSSVSANPGETVKITCSGGGSNNYGWYQQKSPGSAPVTVI YYNDKRPSGIPSRFSGSTSGSTGTLTITGVRAEDEAVYFCGSR DSSHDGIFGAGTTLTVL | 87 |
| VL | LTQPSSVSANPGETVKITCSGGGSNNYGWYQQKPGSAPVTVIY YNDKRPSGIPSRFSGSTSGSTGTLTITGVRAEDEAVYFCGSRD SSHDGIFGGGTKLTVL | 88 |
| VL | LTQPSSVSVSPGQTARITCSGGGSNNYGWYQQKPGSAPVTVIY YNDKRPSGIPERFSGSTSGSTGTLTISGVQAEDEAVYFCGSRD SSHDGIFGGGTKLTVL | 98 |
| VL | LTQPSSVSVSPGQTARITCSGGGSNNYGWYQQKPGQAPVTVIY YNDKRPSGIPERFSGSTSGSTGTLTISGVQAEDEAVYFCGSRD SSHDGIFGGGTKLTVL | 100 |
| VH | AVTLDESGGGLQTPGGALSLVCKASGFTFSTFNMFWVRQAPGK GLEYVAQISSNDGSWTGYGAAVKGRATISRDNGQSTVRLQLNN LRAEDTATYYCAKAAGGYNVCGYGCGGDYIDAWGHGTEVIVSS TS | 89 |
| VH | PVKLVESGGGLVTPGGSLRLSCSASGFTFSSYAMHWVRQAPGK GLEYVGQISSNDGSWTGYGAAVKGRFTISRDNSKSTLYLQMNS LRAEDTAVYYCAKAAGGYNVCGYGCGGDYIDAWGHGTQVTVSS TS | 90 |
| VH | EVQLVESGGGLVQPGGSLRLSCSASGFTFSSYAMHWVRQAPGK GLEYVGQISSNDGSWTGYGAAVKGRFTISRDNSKSTLYLQMNS LRAEDTAVYYCAKAAGGYNVCGYGCGGDYIDAWGQGTLVTVSS TS | 99 |
| VH | EVQLVESGGGLVQPGGSLRLSCSASGFTFSSYAMHWVRQAPGK GLEYVGQISSNDGSWTGYGAAVKGRFTISRDNSKSTLYLQMNS LRAEDTAVYYCAKAAGGYNVCGYGCGGDYIDAWGQGTLVTVSS TS | 101 |

In the light chain and heavy chain variable regions of Table 6, the respective complementarity determining regions are underlined.

In an example, the anti-CD28 antibody or an antigen-binding fragment thereof may comprise:
(1) a light chain variable region comprising the amino acid sequence of SEQ ID NO: 87; and a heavy chain variable region comprising the amino acid sequence of SEQ ID NO: 89;
(2) a light chain variable region comprising the amino acid sequence of SEQ ID NO: 88; and a heavy chain variable region comprising the amino acid sequence of SEQ ID NO: 90;
(3) a light chain variable region comprising the amino acid sequence of SEQ ID NO: 98; and a heavy chain variable region comprising the amino acid sequence of SEQ ID NO: 99;
(4) a light chain variable region comprising the amino acid sequence of SEQ ID NO: 100; and a heavy chain variable region comprising the amino acid sequence of SEQ ID NO: 99; or
(5) a light chain variable region comprising the amino acid sequence of SEQ ID NO: 98; and a heavy chain variable region comprising the amino acid sequence of SEQ ID NO: 101.

In an example,
the anti-CD28 antibody or an antigen-binding fragment thereof may be an scFv comprising:
a light chain variable region that comprises L-CDR1 comprising the amino acid sequence of SEQ ID NO: 65, L-CDR2 comprising the amino acid sequence of SEQ ID NO: 66, and L-CDR3 comprising the amino acid sequence of SEQ ID NO: 67; and
a heavy chain variable region that comprises H-CDR1 comprising the amino acid sequence of SEQ ID NO: 68 or 69, H-CDR2 comprising the amino acid sequence of SEQ ID NO: 70, and H-CDR3 comprising the amino acid sequence of SEQ ID NO: 71.

In an example, the anti-CD28 scFv may comprise:
a light chain variable region comprising the amino acid sequence of SEQ ID NO: 87 or 88; and
a heavy chain variable region comprising the amino acid sequence of SEQ ID NO: 89 or 90.

In an example, the anti-CD28 scFv may comprise:
(1) a light chain variable region comprising the amino acid sequence of SEQ ID NO: 87; and a heavy chain variable region comprising the amino acid sequence of SEQ ID NO: 89; or
(2) a light chain variable region comprising the amino acid sequence of SEQ ID NO: 88; and a heavy chain variable region comprising the amino acid sequence of SEQ ID NO: 90.

Here, the light chain variable region and the heavy chain variable region may be linked to each other, in any order, either directly (for example, without a linker) or via a peptide linker. The peptide linker is as described above.

For example, the anti-CD28 scFv may comprise a light chain variable region and a heavy chain variable region in an N- to C-terminal direction. Alternatively, the anti-CD28 scFv may comprise a heavy chain variable region and a light chain variable region in an N- to C-terminal direction.

In an example, the anti-CD28 scFv may comprise the amino acid sequence of SEQ ID NO: 91 or 92.

### Anti-CD40L/anti-CD28 bispecific antibody

In another aspect, there is provided an anti-CD40L/anti-CD28 bispecific antibody comprising:
(1) an anti-CD40L antibody or an antigen-binding fragment thereof as a CD40L-targeting moiety capable of specifically recognizing and/or binding to CD40L; and
(2) an anti-CD28 antibody or an antigen-binding fragment thereof as a CD28-targeting moiety capable of specifically recognizing and/or binding to CD28.

The anti-CD40L/anti-CD28 bispecific antibody may comprise:
(1) an antigen-binding fragment of an anti-CD40L antibody, which does not comprise an Fc domain (or Fc region), as a CD40L-targeting moiety capable of specifically recognizing and/or binding to CD40L; and
(2) an antigen-binding fragment of an anti-CD28 antibody in a monovalent antibody form, as a CD28-targeting moiety capable of specifically recognizing and/or binding to CD28.

In an example, the antigen-binding fragment of the anti-CD40L antibody may be in the form of an anti-CD40L scFv, Fv, (scFv)₂, Fab, Fab' or F(ab')₂, or a single domain antibody lacking an Fc region.

In an example, the antigen-binding fragment of the anti-CD28 antibody may be in the form of an anti-CD28 scFv, Fv, Fab or Fab', or a single domain antibody in a monovalent antibody form.

In an example, the anti-CD40L/anti-CD28 bispecific antibody may comprise:
(1) an antigen-binding fragment of an anti-CD40L antibody lacking an Fc region; and
(2) an antigen-binding fragment of an anti-CD28 antibody in a monovalent antibody form.

The antigen-binding fragment of the anti-CD40L antibody lacking an Fc region may be in the form of an anti-CD40L scFv, Fv, (scFv)₂, Fab, Fab' or F(ab')₂, or a single domain antibody lacking an Fc region.

The antigen-binding fragment of the anti-CD28 antibody in a monovalent antibody form may be in the form of an anti-CD28 scFv, Fv, Fab or Fab', or a single domain antibody in a monovalent antibody form.

The antigen-binding fragment of the anti-CD40L antibody included in the bispecific antibody does not comprise an Fc region, which enables it to achieve advantageous effects of exhibiting binding affinity to CD40L antigen and inhibition of co-stimulatory molecules and cytokine secretion, which are equivalent to or greater than conventional anti-CD40L antibodies comprising an Fc region, while not exhibiting thromboembolic adverse events.

Furthermore, the antigen-binding fragment of the anti-CD28 antibody in a monovalent antibody form is an antigen-binding fragment in a monovalent antibody form, and thus can achieve an advantageous effect of preventing T cell activation caused by CD28 dimerization.

In an example, the anti-CD40L/anti-CD28 bispecific antibody may comprise:
(1) an anti-CD40L antibody or an antigen-binding fragment thereof; and
(2) an anti-CD28 antibody or an antigen-binding fragment thereof, which comprises L-CDR1 comprising the amino acid sequence of SEQ ID NO: 65;
   L-CDR2 comprising the amino acid sequence of SEQ ID NO: 66;
   L-CDR3 comprising the amino acid sequence of SEQ ID NO: 67;
   H-CDR1 comprising the amino acid sequence of SEQ ID NO: 68 or 69;
   H-CDR2 comprising the amino acid sequence of SEQ ID NO: 70; and
   H-CDR3 comprising the amino acid sequence of SEQ ID NO: 71.

The anti-CD40L antibody or an antigen-binding fragment thereof and the anti-CD28 antibody or an antigen-binding fragment thereof are as described above.

In an example, the anti-CD40L/anti-CD28 bispecific antibody may comprise:
(1) an antigen-binding fragment of an anti-CD40L antibody lacking an Fc region; and
(2) an antigen-binding fragment of an anti-CD28 antibody in a monovalent antibody form.

The antigen-binding fragment of the anti-CD40L antibody may comprise:
L-CDR1 comprising the amino acid sequence of SEQ ID NO: 1, 2, or 3;
L-CDR2 comprising the amino acid sequence of SEQ ID NO: 4, 5, or 6;
L-CDR3 comprising the amino acid sequence of SEQ ID NO: 7, 8, or 9;
H-CDR1 comprising the amino acid sequence of SEQ ID NO: 10, 11, or 12;
H-CDR2 comprising the amino acid sequence of SEQ ID NO: 13, 14, or 15; and
H-CDR3 comprising the amino acid sequence of SEQ ID NO: 16, 17, or 18.

The antigen-binding fragment of the anti-CD28 antibody may comprise:
L-CDR1 comprising the amino acid sequence of SEQ ID NO: 65;
L-CDR2 comprising the amino acid sequence of SEQ ID NO: 66;
L-CDR3 comprising the amino acid sequence of SEQ ID NO: 67;
H-CDR1 comprising the amino acid sequence of SEQ ID NO: 68 or 69;
H-CDR2 comprising the amino acid sequence of SEQ ID NO: 70; and
H-CDR3 comprising the amino acid sequence of SEQ ID NO: 71.

The antigen-binding fragment of the anti-CD40L antibody and the antigen-binding fragment of the anti-CD28 antibody are as described above.

In an example, the anti-CD40L/anti-CD28 bispecific antibody may be such that:
the anti-CD40L antibody or an antigen-binding fragment thereof is an anti-CD40L scFv, or
the anti-CD28 antibody or an antigen-binding fragment thereof is an anti-CD28 scFv, or
the anti-CD40L antibody or an antigen-binding fragment thereof is an anti-CD40L scFv, and the anti-CD28 antibody or an antigen-binding fragment thereof is an anti-CD28 scFv.

In an example, the anti-CD40L/anti-CD28 bispecific antibody may comprise an anti-CD40L antibody in an scFv form and an anti-CD28 antibody in an scFv form. The anti-CD40L scFv included in the bispecific antibody does not comprise an Fc region, which enables it to achieve advantageous effects of exhibiting binding affinity to CD40L antigen and inhibition of co-stimulatory molecules and cytokine secretion, which are equivalent to or greater than conventional anti-CD40L antibodies comprising an Fc region, while not exhibiting thromboembolic adverse events. Furthermore, the anti-CD28 scFv included in the bispecific antibody is an antibody in a monovalent form, and thus can achieve an advantageous effect of preventing T cell activation caused by CD28 dimerization.

The bispecific antibody may comprise an anti-CD40L scFv and an anti-CD28 scFv, wherein the anti-CD40L scFv may be linked, either directly (for example, without a peptide linker) or via a peptide linker, to the N terminus, C terminus, or both of the anti-CD28 scFv. The anti-CD28 scFv may be linked, either directly (for example, without a peptide linker) or via a peptide linker, to the N terminus, C terminus, or both of the anti-CD40L scFv.

In an example, the anti-CD40L scFv or anti-CD28 scFv included in the bispecific antibody may comprise a heavy chain variable region and a light chain variable region in any order. For example, the scFvs included in the bispecific antibody may comprise a light chain variable region and a heavy chain variable region in an N- to C-terminal direction, and may optionally comprise a peptide linker between them. Alternatively, the scFvs included in the bispecific antibody may comprise a light chain variable region and a heavy chain variable region in a C- to N-terminal direction, and may optionally comprise a peptide linker between them.

The anti-CD40L scFv and the anti-CD28 scFv of the bispecific antibody may be linked to each other via a constant region of an antibody.

The constant region of the antibody may refer to a constant region of a heavy chain (γ, δ, α, µ, or ε) or a light chain (λ or κ) of an antibody (for example, IgG, IgM, IgA, IgG, or IgE). In an example, the constant region of the antibody may refer to a constant region (C kappa) of a light chain of an IgG antibody. In an example, the constant region of the antibody may comprise the amino acid sequence of SEQ ID NO: 95.

The constant region of the antibody may be linked to the anti-CD40L scFv directly (for example, without a peptide linker) or via a peptide linker.

The constant region of the antibody may be linked to the anti-CD28 scFv directly (for example, without a peptide linker) or via a peptide linker.

In an example, the bispecific antibody may comprise, from the N terminus to the C terminus:
an anti-CD40L scFv;
optionally, a first peptide linker;
a constant region of an antibody;
optionally, a second peptide linker; and
an anti-CD28 scFv.

In an example, the bispecific antibody may comprise, from the N terminus to the C terminus:
an anti-CD28 scFv;
optionally, a first peptide linker;
a constant region of an antibody;
optionally, a second peptide linker; and
an anti-CD40L scFv.

The first peptide linker and the second peptide linker may independently be present or absent in the bispecific antibody and may be the same as or different from each other.

In an example, the bispecific antibody may comprise the amino acid sequence of SEQ ID NO: 96 or 97.

The bispecific antibody may achieve significantly superior properties (for example, thermal stability) compared to conventional bispecific antibodies. For example, the bispecific antibody may maintain excellent binding affinity to an antigen even after prolonged exposure to high temperatures, and may exhibit excellent inhibition of co-stimulatory molecules and cytokine secretion.

### Production of polynucleotides, recombinant vectors, and antibodies

In another aspect, there are provided polynucleotides encoding the anti-CD40L/anti-CD28 bispecific antibody, the anti-CD40L antibody or an antigen-binding fragment thereof, and/or the anti-CD28 antibody or an antigen-binding fragment thereof.

In another aspect, there is provided a recombinant vector comprising the polynucleotide. For example, recombinant cells may be cells transfected with the recombinant vector.

In another aspect, there is provided a method for preparing the anti-CD40L/anti-CD28 bispecific antibody, the anti-CD40L antibody or an antigen-binding fragment thereof, and/or the anti-CD28 antibody or an antigen-binding fragment thereof, comprising expressing the polynucleotide in a cell. The step of expressing the polynucleotide may be performed by culturing a cell, which comprises the polynucleotide, under conditions that allow expression of the polynucleotide (for example, in a recombinant vector). The method may further comprise isolating and/or purifying the antibody or an antigen-binding fragment thereof from the cell culture following the expression or culture step.

The term "vector" may refer to a means for expressing a target gene in a host cell, as exemplified by plasmid vectors, cosmid vectors and bacteriophage vectors, as well as viral vectors such as adenovirus vectors, retrovirus vectors and adeno-associated viral vectors. Recombinant vectors may consist of plasmids (for example, pSC101, pGV1106, pACYC177, ColE1, pKT230, pME290, pBR322, pUC8/9, pUC6, pBD9, pHC79, pIJ61, pLAFR1, pIJ61, pLAFR1, pET series, and pUC19), phages (for example, λgt4λB, A-Charon, λΔz1, and M13), or engineered viruses (for example, SV40) commonly used in the art.

In recombinant vectors, a polynucleotide may be operably linked to a promoter. The term "operably linked" is intended to refer to a functional linkage between a nucleotide sequence of interest and an expression regulatory element (for example, a promoter sequence). In a case of being "operably linked," the regulatory element is capable of controlling transcription and/or translation of the nucleotide sequence of interest.

Recombinant vectors may typically be constructed as cloning vectors or expression vectors. For recombinant expression vectors, vectors may be used which are commonly available in the art for expressing foreign proteins in plant, animal, or microbial cells. Various methods well known in the art may be used to construct recombinant vectors.

Recombinant vectors may be constructed accordingly for use in hosts such as prokaryotic or eukaryotic cells.
For example, when a vector is constructed as an expression vector for use in a prokaryotic host, the vector may typically comprise a strong promoter for transcription (for example, pLκλ promoter, CMV promoter, trp promoter, lac promoter, tac promoter, and T7 promoter), a ribosome binding site for translation initiation, and transcription/translation termination sequences. Meanwhile, an expression vector for use in a eukaryotic host may comprise an origin of replication operable in eukaryotic cells such as f1 origin of replication, SV40 origin of replication, pMB1 origin of replication, adenovirus origin of replication, AAV origin of replication, and BBV origin of replication, but is not limited to. Furthermore, the expression vector may typically comprise a promoter derived from the genome of a mammalian cell (for example, metallothionein promoter) or a promoter derived from a mammalian virus (for example, adenovirus late promoter, vaccinia virus 7.5K promoter, SV40 promoter, cytomegalovirus promoter, and HSV tk promoter), and a polyadenylation sequence as a transcription termination sequence.

A recombinant cell may be produced by introducing a recombinant vector into a suitable host cell. Any host cell known in the art may be used in the present disclosure, provided that the recombinant vector can be sequentially cloned thereinto and stably expressed. Examples of prokaryotic host cells available for use in the present disclosure may be selected from *E. coli,* Bacillus spp., such as *Bacillus subtilis* and *Bacillus thuringiensis,* and enterobacterial strains, such as *Salmonella typhimurium, Serratia marcescens,* and various Pseudomonas species. Eukaryotic host cells available for transformation may be selected from, but are not limited to, Saccharomyces cerevisiae, insect cells, and animal cells, such as Sp2/0, CHO (Chinese hamster ovary) K1, CHO DG44, PER.C6, W138, BHK, COS-7, 293, HepG2, Huh7, 3T3, RIN, and MDCK.

The polynucleotide or a recombinant vector comprising the polynucleotide may be introduced (transfected) into a host cell using methods known in the art. For example, in a case where the host cell is a prokaryote, such transfection may be performed using CaCl₂ or electroporation.
For a eukaryotic host cell, gene introduction may be achieved using, but not limited to, microinjection, calcium phosphate precipitation, electroporation, liposome-mediated transfection, or particle bombardment.

To select transformed host cells, a phenotype associated with a selection marker may be utilized according to methods well known in the art. For example, in a case where the selection marker is a gene that confers resistance to a specific antibiotic, the host cells may be grown in the presence of the antibiotic in a medium to select for transformants of interest.

In another aspect, there is provided a method for producing a bispecific antibody, comprising expressing the polynucleotide or recombinant vector in a host cell. In an embodiment, the production method may comprise culturing a recombinant cell harboring the polynucleotide or recombinant vector therein, and optionally isolating and/or purifying the antibody from the culture medium.

### Medicinal Uses

In another aspect, there is provided a medicinal use of the anti-CD40L antibody, the anti-CD28 antibody, or the anti-CD40L/anti-CD28 bispecific antibody for prevention, amelioration, or treatment of one or more diseases selected from the group consisting of autoimmune diseases and graft-versus-host disease.

More specifically, the present disclosure provides a pharmaceutical composition for prevention, amelioration, or treatment of one or more diseases selected from the group consisting of autoimmune diseases and graft-versus-host disease, the composition comprising, as an active ingredient, the anti-CD40L antibody, the anti-CD28 antibody, or the anti-CD40L/anti-CD28 bispecific antibody. The pharmaceutical composition may further comprise a pharmaceutically acceptable carrier.

In another aspect, there is provided a use of the anti-CD40L/anti-CD28 bispecific antibody, the anti-CD40L antibody or an antigen-binding fragment thereof, and/or the anti-CD28 antibody or an antigen-binding fragment thereof for prevention, amelioration, or treatment of one or more diseases selected from the group consisting of autoimmune diseases and graft-versus-host disease.

In another aspect, there is provided a use of the anti-CD40L/anti-CD28 bispecific antibody, the anti-CD40L antibody or an antigen-binding fragment thereof, and/or the anti-CD28 antibody or an antigen-binding fragment thereof for manufacture of a pharmaceutical composition for prevention, amelioration, or treatment of one or more diseases selected from the group consisting of autoimmune diseases and graft-versus-host disease.

In another aspect, there is provided a method for prevention, amelioration, or treatment of one or more diseases selected from the group consisting of autoimmune diseases and graft-versus-host disease, comprising administering, to a subject in need thereof, the anti-CD40L/anti-CD28 bispecific antibody, the anti-CD40L antibody or an antigen-binding fragment thereof, and/or the anti-CD28 antibody or an antigen-binding fragment thereof. The method may further comprise, prior to the administration step, identifying a subject in need of prevention, amelioration, or treatment of one or more diseases selected from the group consisting of autoimmune diseases and graft-versus-host disease.

In the present disclosure, the term "prevention" may refer to any act of inhibiting or delaying onset of a disease (condition) by administration of the composition according to an embodiment; the term "treatment" may refer to any act of improving or beneficially altering symptoms of a subject having or suffering from a disease by administration of the composition according to an embodiment; and the term "amelioration" may refer to any act of at least decreasing a parameter related to the state in which a disease is treated, for example, severity of symptoms, by administration of the composition according to an embodiment. The disease may refer to autoimmune diseases and/or graft-versus-host disease.

In an example, the autoimmune diseases and/or graft-versus-host disease may be caused by overexpression and/or hyperactivation of T cells.

The autoimmune disease may be, but is not limited to, one or more selected from the group consisting of rheumatoid arthritis, multiple sclerosis, systemic lupus erythematosus, type 1 diabetes, Crohn's disease, scleroderma, Sjogren's syndrome, psoriasis, inflammatory bowel disease, ulcerative colitis, ankylosing spondylitis, interstitial lung disease, uveitis, optic neuritis, peripheral neuropathies, sarcoidosis, antiphospholipid syndrome, inflammatory myopathies, Behcet's disease, alopecia totalis/universalis, pemphigus vulgaris, myasthenia gravis, Graves' disease, Hashimoto's thyroiditis, Guillain-Barre syndrome, Celiac disease, and pernicious anemia.

The graft-versus-host disease (GVHD) may be, but is not limited to, one or more selected from the group consisting of acute graft-versus-host disease and chronic graft-versus-host disease.

The pharmaceutical composition may further comprise a pharmaceutically acceptable carrier, diluent, and/or excipient. The pharmaceutically acceptable carrier, diluent, and/or excipient may be any one selected from those commonly used for antibody formulation. For example, the pharmaceutically acceptable carrier includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

The pharmaceutical composition may further comprise one or more selected from the group consisting of lubricants, wetting agents, sweeteners, flavor enhancers, emulsifiers, suspending agents, and preservatives.

The pharmaceutical composition may be administered to a subject orally or parenterally. Parenteral administration may be intravenous injection, subcutaneous injection, intramuscular injection, intraperitoneal injection, endothelial administration, topical administration, intranasal administration, intrapulmonary administration, or rectal administration. Oral administration leads to digestion of proteins or peptides. Thus, the active ingredient of a composition for oral administration may be coated or formulated to prevent digestion in the stomach. In addition, the pharmaceutical composition may be administered using any device that enables the active ingredient to be delivered to target cells.

In the present specification, the term "pharmaceutically effective amount" may refer to an amount of the active ingredient (the anti-CD40L/anti-CD28 bispecific antibody, anti-CD40L antibody, and/or anti-CD28 antibody) which allows it to exert a pharmaceutically meaningful effect in prevention, amelioration, or treatment of autoimmune diseases and/or graft-versus-host disease. The appropriate dosage of a pharmaceutical composition, expressed as the pharmaceutically effective amount of an antibody or the amount of an antibody, may be variously prescribed depending on various factors such as age, weight, gender, pathological condition, diet, excretion rate, patient's response sensitivity, formulation type, administration time, administration route, and administration method. For example, the pharmaceutically effective amount of an antibody or the appropriate dosage of a pharmaceutical composition may be in a range of about 0.001 to about 1000 mg (amount of antibody)/kg (body weight), about 0.01 to about 100 mg/kg, or 0.1 to 50 mg/kg per day for an adult.

As used herein, the pharmaceutically effective amount refers to an amount or dosage of an active ingredient capable of achieving a desired effect. The active ingredient may be the anti-CD40L/anti-CD28 bispecific antibody, anti-CD40L antibody, and/or anti-CD28 antibody. The amount or dosage of the active ingredient in the pharmaceutical composition may be variously prescribed depending on factors such as formulation method, administration mode, patient's age, body weight, gender, pathological condition, diet, administration time, administration interval, administration route, excretion rate, and response sensitivity. For example, in a case where the active ingredient is an antibody, a single dosage thereof may be, but is not limited to, 0.001 to 1000 mg/kg, 0.01 to 100 mg/kg, 0.01 to 50 mg/kg, 0.01 to 20 mg/kg, 0.01 to 10 mg/kg, 0.01 to 5 mg/kg, 0.1 to 100 mg/kg, 0.1 to 50 mg/kg, 0.1 to 20 mg/kg, 0.1 to 10 mg/kg, 0.1 to 5 mg/kg, 1 to 100 mg/kg, 1 to 50 mg/kg, 1 to 20 mg/kg, 1 to 10 mg/kg, or 1 to 5 mg/kg.

In an example, the pharmaceutical composition may be administered to a subject once a day or divided into two or more doses per day; and in an example, it may be administered at intervals of 1 hour, 2 hours, 6 hours, 12 hours, 24 hours, 2 days, 3 days, one week, two weeks, one month, and/or three months.

The dosage and/or dosing frequency are not intended to limit the scope of the present disclosure in any way.

In another example, the amount of the active ingredient in the pharmaceutical composition may be, but is not limited to, 0.01 wt% to 99.9 wt%, 0.01 wt% to 90 wt%, 0.01 wt% to 80 wt%, 0.01 wt% to 70 wt%, 0.01 wt% to 60 wt%, 0.01 wt% to 50 wt%, 0.01 wt% to 40 wt%, 0.01 wt% to 30 wt%, 1 wt% to 99.9 wt%, 1 wt% to 90 wt%, 1 wt% to 80 wt%, 1 wt% to 70 wt%, 1 wt% to 60 wt%, 1 wt% to 50 wt%, 1 wt% to 40 wt%, 1 wt% to 30 wt%, 5 wt% to 99.9 wt%, 5 wt% to 90 wt%, 5 wt% to 80 wt%, 5 wt% to 70 wt%, 5 wt% to 60 wt%, 5 wt% to 50 wt%, 5 wt% to 40 wt%, 5 wt% to 30 wt%, 10 wt% to 99.9 wt%, 10 wt% to 90 wt%, 10 wt% to 80 wt%, 10 wt% to 70 wt%, 10 wt% to 60 wt%, 10 wt% to 50 wt%, 10 wt% to 40 wt%, or 10 wt% to 30 wt%, based on the total weight of the pharmaceutical composition.

Furthermore, the pharmaceutical composition may further comprise, in addition to the active ingredient, a pharmaceutically acceptable carrier and/or adjuvant. The pharmaceutically acceptable carrier may refer to a carrier which is commonly used in formulation of a drug comprising a protein, nucleic acid or cell, does not irritate an organism, and does not inhibit biological activity and/or properties of the active ingredient. In an example, the carrier may be at least one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but not limited thereto. The pharmaceutical composition may further comprise one or more selected from the group consisting of diluents, excipients, lubricants, wetting agents, sweeteners, flavorings, emulsifiers, suspending agents, and preservatives, which are commonly used in preparation of a pharmaceutical composition.

In an example, the pharmaceutically acceptable carrier, which is commonly used in formulation, includes, but is not limited to, saline, sterile water, Ringer's solution, buffered saline, cyclodextrin, dextrose solution, maltodextrin solution, glycerol, ethanol, and liposomes, and may further comprise other conventional additives such as antioxidants and buffers, if necessary. In addition, the composition may be formulated into injectable preparations, such as aqueous solutions, suspensions, and emulsions, or into pills, capsules, granules, or tablets, by further adding diluents, dispersants, surfactants, binders, lubricants, and the like. Regarding suitable pharmaceutically acceptable carriers and formulation methods, formulation may be achieved depending on the respective ingredients by using methods disclosed in Remington's Pharmaceutical Sciences (19th edition, 1995). In an embodiment, the pharmaceutical composition may comprise, as a pharmaceutically acceptable carrier, a binder such as lactose, saccharose, sorbitol, mannitol, starch, amylopectin, cellulose, or gelatin; an excipient such as dicalcium phosphate; a disintegrating agent such as corn starch or sweet potato starch; a lubricant such as magnesium stearate, calcium stearate, sodium stearyl fumarate, or polyethylene glycol wax; a sweetener; a flavoring agent; a syrup; a liquid carrier such as fatty oil; a sterile aqueous solution; propylene glycol; polyethylene glycol; injectable esters such as ethyl oleate; a suspending agent; an emulsifier; a lyophilized preparation; an external preparation; a stabilizer; a buffer; animal oils; vegetable oils; waxes; paraffin; starch; tragacanth; cellulose derivatives; polyethylene glycol; silicone; bentonite; silica; talc; zinc oxide, or a suitable combination thereof.

The subject to which the pharmaceutical composition as provided herein is administered may be a mammal, including humans, dogs, cats, horses, cattle, pigs, goats, rabbits, mice, rats, and the like, or a cell or tissue isolated therefrom, or a culture thereof. In an example, the subject may be an individual (a mammal such as a human) in need of prevention, amelioration, and/or treatment of or having an autoimmune disease and/or a graft-versus-host disease as described above, or a cell or tissue isolated therefrom, or a culture thereof.

The pharmaceutical composition may be administered by oral or parenteral administration, or by being brought in contact with cells, tissues, or body fluids. Specifically, for parenteral administration, the pharmaceutical composition may be administered via subcutaneous injection, intramuscular injection, intravenous injection, intraperitoneal injection, endothelial administration, topical administration, intranasal administration, intrapulmonary administration, and rectal administration. For oral administration, since proteins or peptides are subject to digestion, an oral composition must be formulated so that an active agent is coated or protected from degradation in the stomach. For intranasal administration, the pharmaceutical composition may be administered via nasal spray, where the composition is diluted and absorbed into the nasal cavity through an atomizer or spray system. Examples of nasal spray agents or respiratory formulations for the nasal spray include aerosols.

In addition, the pharmaceutical composition may be formulated in the form of a solution in an oil or aqueous medium, an injection, a suspension, a syrup, an emulsion, a liniment, a patch, an extract, a powder, a granule, a tablet, a capsule, an aerosol, or the like, and may further comprise a dispersant or stabilizer for formulation.

In an embodiment, the pharmaceutical composition may be formulated into a troch, a lozenge, a tablet, a watersoluble suspension, an oil-based suspension, a compounding powder, a granule, an emulsion, a hard capsule, a soft capsule, a syrup, or an elixir. In another embodiment, the pharmaceutical composition may be formulated into an injectable solution, a suppository, a powder for respiratory inhalation, an aerosol for spraying, an ointment, a powder for application, an oil, or a cream. In yet another embodiment, the pharmaceutical composition may be formulated into an injectable solution. Specifically, a therapeutically effective amount of an antiviral peptide may be mixed with a stabilizer or buffer in water to prepare a solution or suspension, and then the resultant may be formulated into a unit dosage form in an ampoule or vial. In another embodiment, the pharmaceutical composition may be blended with an additive such as a propellant to prepare a water-dispersible concentrate or a wettable powder, and then the resultant may be formulated into an aerosol. In yet another embodiment, in a case where the pharmaceutical composition is formulated for transdermal use, a therapeutically effective amount of an antiviral peptide may be added to a carrier such as animal oils, vegetable oils, waxes, paraffin, starch, tragacanth, cellulose derivatives, polyethylene glycol, silicon, bentonite, silica, talc, or zinc oxide to prepare an ointment, a cream, a powder for application, an oil, an external skin preparation, or the like.

### ADVANTAGEOUS EFFECTS OF INVENTION

The anti-CD40L/anti-CD28 bispecific antibody of the present disclosure comprises an anti-CD40L scFv and an anti-CD28 scFv, which allows it to exhibit an excellent therapeutic effect on an autoimmune disease and/or a graft-versus-host disease, together with excellent physical properties of the bispecific antibody, while preventing T cell activation caused by CD28 dimerization and without showing thromboembolic adverse events.

### BRIEF DESCRIPTION OF DRAWINGS

FIG. 1 illustrates the genetic information of 49 clones that bind to human CD40L in an enzyme-linked immunosorbent assay.
FIGS. 2a and 2b illustrate the results obtained by identifying, through flow cytometry, that the expressed and purified 15 anti-CD40L antibodies bind to the human CD40L-expressing Jurkat D1.1 (hCD40L+) cell line.
FIG. 3 illustrates the results obtained by identifying, through flow cytometry, that the anti-CD40L antibodies (1G-2, 1F-7, 3B-7) bind to human CD40L recombinant protein and inhibit its binding to Raji (CD40L+) cell line.
FIG. 4 illustrates that when the 17 anti-CD40L scFv antibodies were compared with a positive control (hu5C8 clone) in terms of the ability to inhibit expression of B-cell co-stimulatory molecules, 1G-2 and 3F-9 clones exhibited a better inhibitory ability than the positive control.
FIG. 5 illustrates that among the 17 clones, the anti-CD40L antibody (1G-2) exhibited an inhibitory ability similar to the positive control when its dose-response (5 or 10 µg/ml) was checked.
FIG. 6 illustrates that treatment with a high concentration (5 µg/ml) of the anti-CD40L antibody (1G-2) resulted in over 50% inhibition of B-cell proliferation.
FIG. 7 illustrates that the anti-CD40L antibody (1G-2) significantly decreases antibody production in a concentration-dependent manner.
FIG. 8 illustrates the results, showing that the expression of co-stimulatory molecules decreases as the concentration of the anti-CD40L antibody (1G-2) increases.
FIG. 9 illustrates that IL-12 production by dendritic cells is effectively inhibited by treatment with the anti-CD40L antibody (1G-2).
FIG. 10 illustrates the results obtained by measuring the antigen binding affinity of 1G-2 clone.
FIG. 11 illustrates that when the 15 anti-CD40L scFv antibodies were compared with a positive control (hu5C8) in terms of the ability to inhibit expression of co-stimulatory molecules on Daudi cells, 1H-8 and 3E-3 clones exhibited an inhibitory ability similar to the positive control.
FIG. 12 illustrates the results obtained by evaluating a T-cell epitope content using immunoinformatics tools, through in silico antibody sequence analysis by Abzena Ltd (UK), to analyze immunogenicity of the selected chicken-derived anti-CD40L antibodies (1G-2, 1H-8, 3E-3).
FIGS. 13a to 13c illustrate the results obtained by selecting 41, 40, and 38 clones, which exhibit higher affinity than the existing wild-type antibody clone, respectively, during de-immunization process of the antibodies (1G-2, 1H-8, and 3E-3), and acquiring genetic information thereof through sequencing.
FIG. 14 illustrates the result obtained by allowing antibodies to be overexpressed through transformation of human embryonic kidney 293F (HEK293F) cells, and then purifying the recombinant antibodies from the cell culture using affinity chromatography.
FIG. 15 illustrates the results obtained by identifying, through flow cytometry, that the three anti-CD40L antibodies (3E-3, de-immunized 2E9F, and 2E9F VH G49S clones) bind to the human CD40L-expressing Jurkat D1.1 (hCD40L+) cell line.
FIG. 16 illustrates the result obtained by identifying, through an enzyme-linked immunosorbent assay, that the three anti-CD40L antibodies bind to human CD40L (a recombinant protein in the form of human CD40L-isoleucine zipper-hemagglutinin).
FIGS. 17a and 17b illustrate the results, showing that both 2E9F and 2E9F VH G49S clones inhibit CD40/CD40L interaction.
FIG. 18 illustrates the results obtained by selecting 79 clones, which exhibit higher affinity than the existing wild-type antibody clone, during humanization process of 3E-3 clone, and acquiring genetic information thereof through sequencing.
FIG. 19 illustrates the result of alignment between the sequence having the fully humanized framework region and the sequence of the acquired clone #91.
FIG. 20 illustrates the result obtained by making 8 combination clones, which comprise human- and chicken-derived residues, and cloning the combination clones into a phagemid vector.
FIG. 21 illustrates the result, showing that the 8 combination clones exhibit a binding ability to human CD40L recombinant protein in a phage enzyme immunoassay.
FIG. 22 illustrates the results obtained by identifying, through flow cytometry, that the expressed and purified 8 antibody clones bind to the human CD40L-overexpressing L cell line.
FIG. 23 illustrates the results, showing that among the 8 prepared clones (LSS, LSG, LIS, LIG, TSS, TSG, TIS, and TIG clones), TSS, TSG, TIS, and TIG clones exhibit a superior binding inhibitory ability.
FIG. 24 illustrates the results, showing that when the TSS, TSG, TIS, and TIG clones were evaluated at different concentrations (2, 4, 6, 8, 10 µg/ml) for their ability to inhibit expression of B-cell co-stimulatory molecules (CD80, CD86), the TSG clone exhibited the highest inhibitory ability.
FIG. 25 illustrates a schematic diagram for binding affinity measurement using Biacore.
FIGS. 26a and 26b illustrate the results obtained by measuring CD40L binding affinity using the multi-kinetics method and the single-kinetics method, respectively.
FIG. 27 illustrates the result obtained by selecting 49 clones, which bind to human CD28, and analyzing genetic information thereof.
FIG. 28 illustrates the results, showing that the expressed and purified 12 anti-CD28 antibodies bind to the Jurkat E6.1 (Human CD28+) cell line.
FIG. 29 illustrates the result, showing that when the 12 anti-CD28 antibodies were compared with a positive control in the Jurkat E6.1 cell line in terms of the ability to inhibit IL-2 secretion, J6-2 and J6-3 clones exhibited an inhibitory ability similar to the positive control.
FIG. 30 illustrates the results, showing that among the 12 anti-CD28 antibodies, J6-3 clone exhibited a higher IL-2 inhibitory ability than a positive control (FR104) and exhibited an IFN gamma secretion inhibitory ability similar to the positive control.
FIG. 31 illustrates the results, showing that binding affinity for CD80/CD86 proteins was significantly decreased in the cells treated with J6-3 clone compared to the cells not treated with J6-3 clone.
FIG. 32 illustrates the result, showing that 4-25, 6-15, and 6-23 clones exhibited a binding ability similar to a positive control (FR104).
FIG. 33 illustrates the result obtained by analyzing immunogenicity of J6-3 clone, through in silico antibody sequence analysis by Abzena Ltd (UK), to check its immunogenicity.
FIG. 34 illustrates a schematic diagram for comparing a human germline sequence having the highest homology to J6-3 clone for humanization simultaneously with de-immunization.
FIG. 35 illustrates the results obtained by performing sequencing of genetic information of various clones, which bind to human CD28, in an enzyme-linked immunosorbent assay.
FIG. 36 illustrates the results, showing that clone #95 (G12 clone) was ultimately obtained through sequencing following an enzyme-linked immunosorbent assay.
FIG. 37 illustrates the result obtained by analyzing G12 clone, showing that there are two immunogenic sites in G12 clone.
FIG. 38 illustrates the result, showing that a final clone has been established by completing de-immunization and humanization of the original J6-3 clone.
FIG. 39 illustrates the results obtained by identifying, through flow cytometry, that the expressed and purified anti-CD28 antibodies (J6-3 clone and humanized 2-G12 clone) bind to the Jurkat E6.1 (hCD28⁺) cell line.
FIG. 40 illustrates the results obtained by identifying, through an enzyme-linked immunosorbent assay, that the J6-3 clone and the humanized 2-G12 clone bind to human CD28 recombinant protein (hCD28).
FIG. 41 illustrates the results, showing that when the humanized 2-G12 clone was checked for its ability to inhibit cytokine secretion by CD4 T cells, the 2-G12 clone exhibited a better IL-2 (interleukin-2) secretion inhibitory ability than a positive control (FR104), while exhibiting an IFN gamma inhibitory ability similar to a positive control (FR104).
FIG. 42 illustrates a schematic diagram in which the humanized anti-CD40L scFv antibody, TSG clone, prepared in Examples 1-4 and the humanized anti-CD28 scFv antibody, 2-G12 clone, prepared in Examples 2-3, are cloned into a bispecific antibody format comprising a Ck linker domain, and then expressed in a mammalian expression system.
FIG. 43 illustrates the result, showing that the recombinant antibodies were overexpressed through transformation of human embryonic kidney 293F (HEK293F) cells, and then the antibodies were successfully purified from a cell culture using affinity chromatography.
FIG. 44 illustrates the result, showing that the bispecific antibody (TSGxHu2G12 clone) prepared in Example 3.2.1 has been successfully purified.
FIG. 45 illustrates a graph, showing that the bispecific antibody (TSGxHu2G12 clone) exhibits CD28 binding affinity equivalent to TSGx2-G12.
FIG. 46 illustrates a graph, showing that the bispecific antibody (TSGxHu2G12 clone) exhibits binding affinity to cell surface CD28 equivalent to TSGx2-G12.
FIG. 47 illustrates a schematic diagram of the experiment for identifying the ability to inhibit CD28-CD80 interaction in Example 3.2.4.
FIG. 48 illustrates a graph, showing that the bispecific antibody (TSGxHu2G12) comprising Hu2G12 clone inhibits binding of recombinant CD80 protein at a 1000 nM antibody concentration to an extent similar to the bispecific antibody (TSGx2-G12) comprising 2-G12 clone.
FIG. 49 illustrates a graph, showing that among the six anti-CD40L/anti-CD28 bispecific antibodies prepared by performing an additional humanization process in Example 3-3, the clone (TSGxHu2G12-S42Q) with an S42Q mutation introduced into the light chain (V_{L}) and the clone (TSGxHu2G12-S76N) with an S76N mutation introduced into the heavy chain (V_{H}) exhibit binding affinity to CD28 which is equivalent to the anti-CD40L/anti-CD28 bispecific antibody (TSGx2-G12, Table 16) prepared in Example 3-1.
FIG. 50 illustrates a graph, showing that TSGxHu2G12-S42Q and TSGxHu2G12-S76N clones exhibit equivalent binding affinity to cell surface CD28 compared to TSGx2-G12 clone.
FIG. 51 illustrates a schematic diagram of the experiment for identifying the ability to inhibit CD28-CD80 interaction in Example 3.3.4.
FIG. 52 illustrates a graph, showing that TSGxHu2G12-S42Q and TSGxHu2G12-S76N clones exhibit an ability to inhibit binding of recombinant CD80 protein which is equivalent to TSGx2-G12 clone.
FIG. 53 illustrates a schematic diagram, showing the positive feedback mechanism in which activated T cells activate APCs, and the activated APCs in turn increase activity of T cells.
FIG. 54 illustrates a schematic diagram for the experimental process of Examples 3-4.
FIG. 55 illustrates a graph, showing that the bispecific antibody (TSGx2-G12) and the CD28 single-inhibitory antibodies (aCot-Ck-aCD28; aCot-Ck-2-G12) exhibit an IFN-γ inhibitory effect.
FIG. 56 illustrates a schematic diagram, showing the phenomenon in which in a case where an autoantigen-specific T cell recognizes an autoantigen presented by an antigen-presenting cell and receives a TCR signal (signal 1), if the T cell does not receive co-stimulation (signal 2) from the antigen-presenting cell, the T cell does not become activated even upon subsequent re-stimulation with the autoantigen.
FIG. 57 illustrates a schematic diagram for the experimental process of Examples 3-5.
FIG. 58 illustrates the experimental result, showing that in the group treated with the anti-CD40L/anti-CD28 bispecific antibody, the amount of IFN-γ was significantly decreased compared to a negative control, and the CD4 T cells treated with the bispecific antibody did not respond even upon antigen re-stimulation.
FIG. 59 illustrates the experimental result, showing that the anti-CD40L/anti-CD28 bispecific antibody of the present disclosure exhibits a superior T cell inhibitory ability regardless of the order of the anti-CD40L antibody and the anti-CD28 antibody.
FIG. 60 illustrates schematic diagrams for the linked inhibition and the separate inhibition.
FIGS. 61 and 62 illustrate the experimental results, showing that CD40L and CD28 are co-localized at the same position only in the group treated with the anti-CD40L-Ck-CD28 bispecific antibody, which suggests that the anti-CD40L/anti-CD28 bispecific antibody of the present disclosure acts in a linked inhibition mode.
FIGS. 63 and 64 illustrate the experimental results, showing that only in the group treated with the anti-CD40L-Ck-CD28 bispecific antibody, CD28-mNeonGreen fluorescence in the cell membrane region was significantly changed, and thus the FRET efficacy value had statistical significance compared to the other groups, which suggests that the anti-CD40L/anti-CD28 bispecific antibody of the present disclosure acts in a linked inhibition mode.
FIGS. 65 and 66 illustrate the results, showing that the group treated with the anti-CD40L/anti-CD28 bispecific antibody had no weight loss during the experimental period and did not develop any disease in evaluation of disease severity. Furthermore, all mice survived throughout the experimental period only in the group treated with the bispecific antibody, indicating that disease progression was completely suppressed.
FIG. 67 illustrates an exemplary form of the anti-CD40L/anti-CD28 bispecific antibody of the present disclosure (anti-CD40L scFv - Ck - anti-CD28 scFv).
FIG. 68 illustrates a graph, showing that among the anti-CD40L antibodies for mice that bind to mouse CD40L, 1-1-H, 1-12C, 1-12-H, 2-12-C, and 3-11-C clones effectively bind to mouse CD40L expressed on the cell surface in a concentration-dependent manner.
FIG. 69 illustrates graphs, showing that in a case of allowing the PV1x2-12-C antibody to bind to activated mouse CD4 T cells and then measuring binding of the antibody by flow cytometry using a fluorescently labeled anti-Ck antibody, the PV1x2-12-C antibody binds to the activated mouse CD4 T cells.
FIG. 70 illustrates experimental results, showing that, similarly to the experimental results of the anti-CD40L/anti-CD28 bispecific antibody that underwent the humanization process in Example 3, the anti-CD40L/anti-CD28 bispecific antibody for mice inhibited T cell activity by blocking CD40L and CD28 signaling on CD4 T cells.
FIG. 71 illustrates a graph, showing that the group (PV1x2-12-C) treated with the anti-CD40L/anti-CD28 bispecific antibody for mice exhibited significantly lower clinical severity of the disease, which indicates that the bispecific antibody provided by the present disclosure effectively suppresses an autoimmune demyelinating disease.

### MODES FOR CARRYING OUT INVENTION

The present disclosure will be described in more detail by way of the following examples. However, these are merely intended to illustrate the present disclosure, and the scope of the present disclosure is not limited by these examples.

### Example 1. Anti-CD40L antibody

### Example 1-1. Selection of chicken-derived anti-CD40L antibody (lead antibody)

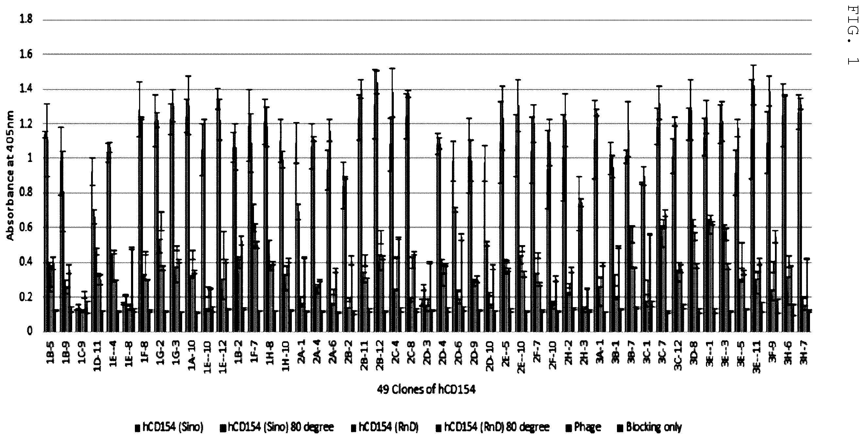

Chickens (white leghorn chicken, 3 animals, Biopoa Co., Ltd.) were immunized with human CD40L recombinant protein (R&D Systems, Minneapolis, MN, USA), and antibody libraries in an scFv format were then constructed using a previously reported method. The constructed three chicken antibody libraries had complexity of 2.4 x10⁹, 2.4 x10⁹, and 2.7 x10⁹, respectively. Bio-panning against human CD40L was performed using the chicken antibody libraries, and 49 clones that bind to human CD40L were selected by an enzyme-linked immunosorbent assay, with the genetic information thereof being obtained through sequencing (FIG. 1).

To produce 15 antibody clones, which bind to human CD40L recombinant protein, in the form of an scFv-Ck-scFv-HIS-HA fusion protein [anti-CD40L scFv antibody (N'-VL-linker (SEQ ID NO: 93)-VH-C') - Ck (SEQ ID NO: 95) - anti-cotinine scFv antibody (N'-VL-linker (SEQ ID NO: 93)-VH-C') - HIS-HA], cloning was performed into an animal cell expression vector (pCEP4 vector, Invitrogen, Carlsbad, CA, USA). The expression vector was used to transform human embryonic kidney 293F (HEK293F) cells (Invitrogen, Carlsbad, CA, USA), and then the antibodies were purified from the cell culture using affinity chromatography.

To check the effect of the anti-CD40L antibodies in a bispecific antibody format in which two monovalent antibodies are linked, the following experiment was conducted by linking the anti-CD40L scFv antibody to an anti-cotinine scFv antibody that binds to cotinine, a substance not present in the body. The anti-cotinine antibody was obtained from Korean Laid-Open Patent Publication (KR 10-2019-0023084 A) and used. The CDR and variable region sequences of the anti-cotinine antibody are as set forth in SEQ ID NOs: 120-126 and SEQ ID NOs: 127-128.

**[Table 7]**

| Clone | Transfection volume (mL) | Quantification | |
|---|---|---|---|
| | | µg | mg/L |
| 2E-5 | 100 | 1007 | 10.1 |
| 3E-1 | 100 | 931 | 9.3 |
| 3E-11 | 100 | 952 | 9.5 |
| 3C15 | 100 | 656 | 6.6 |
| 3A-1 | 100 | 467 | 4.7 |
| 3B-1 | 100 | 385 | 3.9 |
| 3H-7 | 100 | 389 | 3.9 |
| 1B-5 | 100 | 456 | 4.6 |
| 1E-12 | 100 | 2212 | 22.1 |
| 1F-8 | 100 | 4632 | 46.3 |
| 1H-8 | 100 | 6792 | 67.9 |
| 2B-11 | 100 | 1854 | 18.5 |
| 3C-7 | 100 | 1488 | 14.9 |
| 3E-3 | 100 | 5032 | 50.3 |
| 3F-9 | 100 | 1088 | 10.9 |

### 1.1.1. Identification of binding to human CD40L

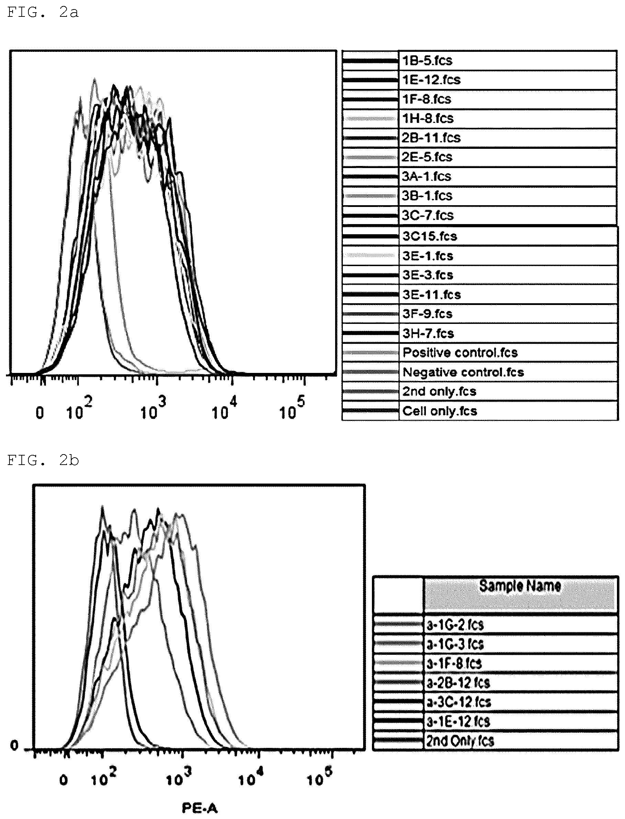

Binding of the expressed and purified 15 anti-CD40L antibodies to the human CD40L-expressing Jurkat D1.1 (hCD40L+) cell line (ATCC, Manassas, VA, USA) was identified through flow cytometry (FIGS. 2a and 2b).

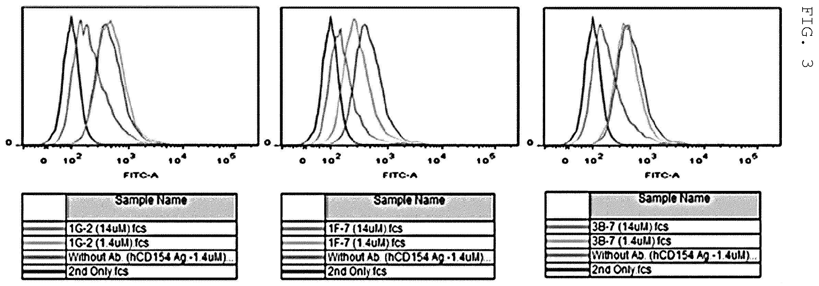

In addition, it was identified through flow cytometry that the anti-CD40L antibodies (1G-2, 1F-7, 3B-7) bind to human CD40L recombinant protein and inhibit its binding to the Raji (CD40L+) cell line (ATCC, Manassas, VA, USA) (FIG. 3).

### 1.1.2. Inhibition of B cell and dendritic cell activation

It is known that CD40L, which is expressed on the surface of T cells, binds to CD40 on the surface of antigen-presenting cells (APCs) such as B cells and dendritic cells, thereby activating the antigen-presenting cells. Therefore, it was evaluated whether the anti-CD40L antibodies have an ability to inhibit CD40L/CD40 interaction to suppress B cell and dendritic cell activation.

For the purpose of obtaining human B cells and monocytes, leukapheresis was performed on healthy adults to obtain leukocytes. B cells and monocytes were then isolated from the obtained leukocytes using the MACS cell separation method (Miltenyi Biotec, 130-050-201). The monocytes were differentiated into dendritic cells using granulocyte-macrophage colony-stimulating factor (GM-CSF) and interleukin-4 (IL-4).

The mouse-derived fibroblast cell line expressing human CD40L (CD154L cells) (Sim et al. Arthritis Research & Therapy (2015) 17:190 DOI 10.1186/s13075-015-0687-1) was co-cultured with the B cells or dendritic cells to establish a system in which CD40 stimulation is transmitted to the B cells and dendritic cells. Then, it was checked whether the prepared anti-CD40L antibodies inhibit activation of the B cells or dendritic cells. Here, the CD154L cells were treated with 60 Gy of radiation to inhibit cell proliferation. A previously known anti-CD40L antibody (hu5C8 clone) (Karpusas et al. Structure of CD40 Ligand in Complex with the Fab Fragment of a Neutralizing Humanized Antibody Structure. 2001 Apr 4;9(4):321-9.) was expressed and purified in an scFv form, and used as a positive control.

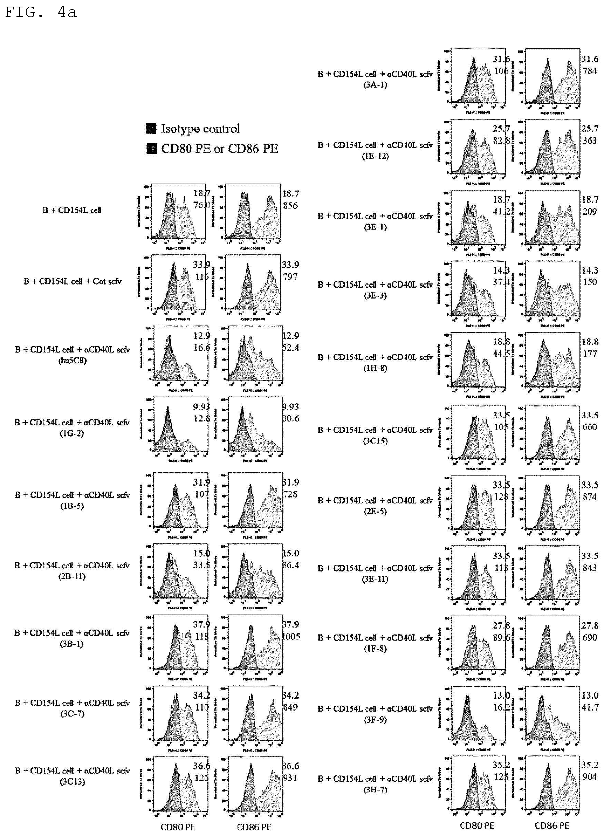

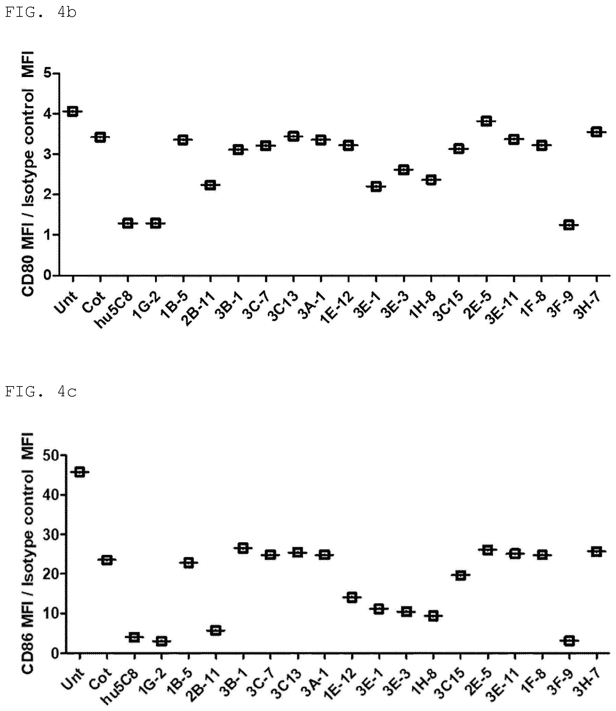

After co-culturing the B cells, the CD154L cells, and the anti-CD40L antibody (5 µg/ml), flow cytometry was used to identify how much an increase in expression of co-stimulatory molecules (CD80, CD86) caused by B cell activation was inhibited by the anti-CD40L antibody. The results are shown in FIGS. 4a to 4c. (FIGS. 4b and 4c quantitatively represent the results of FIG. 4a).

As can be seen from FIGS. 4a, 4b, and 4c, comparison of the 17 anti-CD40L scFv antibodies with the positive control (hu5C8 clone) in terms of the ability to inhibit expression of the B-cell co-stimulatory molecules showed that 1G-2 and 3F-9 clones exhibited a better inhibitory ability than the positive control (hu5C8) (FIG. 4c).

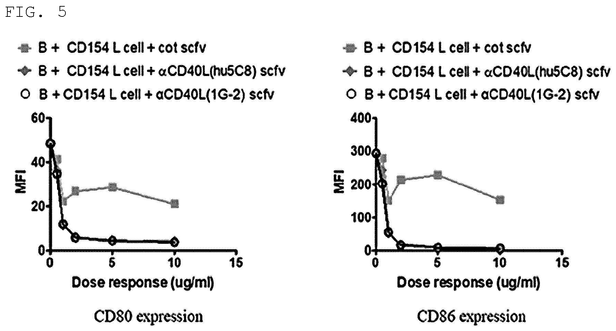

In addition, among the 17 clones, the anti-CD40L antibody (1G-2) was examined for its dose-response (5 or 10 µg/ml). As a result, 1G-2 exhibited an inhibitory ability similar to the positive control (hu5C8) (FIG. 5).

### 1.1.3. Inhibition of B cell proliferation

When B cells and CD154L cells are co-cultured, CD40 on the B cells is stimulated so that the B cells proliferate. Here, the extent of cell proliferation can be identified by treating the proliferating cells with Edu and detecting them with an anti-Edu antibody.

To examine the B cell proliferation inhibitory ability of the anti-CD40L antibody (1G-2), Edu (Thermo Fisher Scientific, C10637) was added to the co-culture system (culture of B cells, CD154L cells, and anti-CD40L antibody), and the amount of Edu incorporated into the DNA of the proliferating B cells was analyzed using an anti-Edu antibody (Thermo Fisher Scientific, C10637) on a flow cytometer to measure the degree of B cell proliferation.

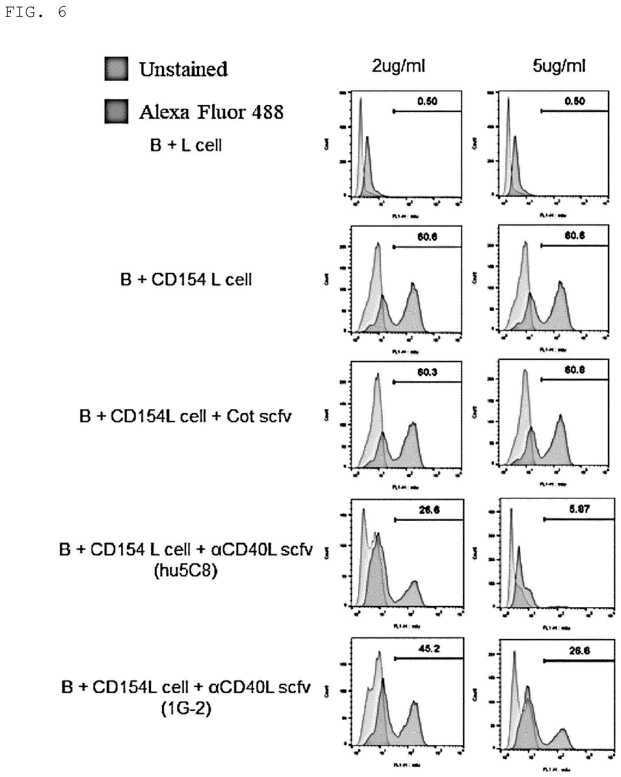

As a result, upon treatment with a high concentration (5 µg/ml), the anti-CD40L antibody (1G-2) showed more than 50% inhibition of B cell proliferation (FIG. 6).

### 1.1.4. Inhibition of antibody production by B cells

When B cells and CD154L cells are co-cultured, the B cells secrete IgM upon CD40 stimulation. The amount of secreted IgM can be measured to assess the degree of B cell activation.

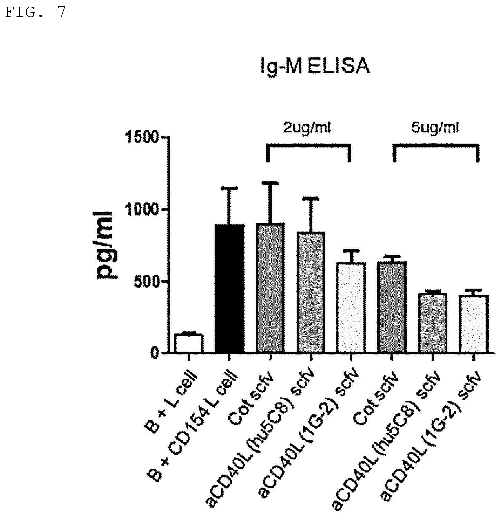

Therefore, it was checked whether antibody production by the B cells is inhibited upon treatment with the anti-CD40L antibody (1G-2) using an IgM ELISA. The results are shown in FIG. 7.

As shown in FIG. 7, it was identified that the anti-CD40L antibody (1G-2) significantly decreased antibody production in a concentration-dependent manner (FIG. 7).

### 1.1.5. Inhibition of expression of co-stimulatory molecules on dendritic cells

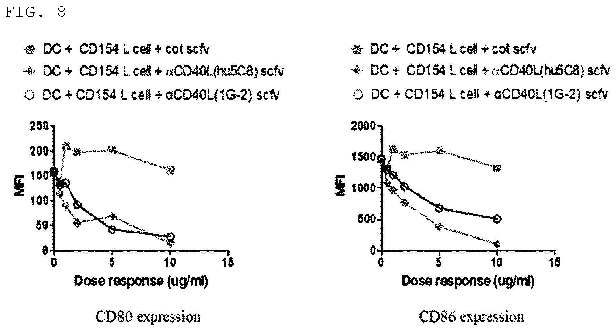

To verify whether the anti-CD40L antibody inhibits dendritic cell (DC) activation along with inhibition of B cell activation, the dendritic cells, the CD154L cells, and the anti-CD40L antibody were co-cultured, and then the expression level of co-stimulatory molecules (CD80, CD86) on the dendritic cells was identified using flow cytometry. The results are shown in FIG. 8.

As shown in FIG. 8, it was identified that the expression of the co-stimulatory molecules (CD80, CD86) decreased as the concentration of the anti-CD40L antibody (1G-2) increased (FIG. 8).

### 1.1.6. Inhibition of cytokine secretion by dendritic cells

When dendritic cells and CD154L cells are co-cultured, the dendritic cells are activated by CD40 stimulation, leading to increased cytokine production. Therefore, the degree of dendritic cell activation can be measured by quantifying the amount of secreted cytokines.

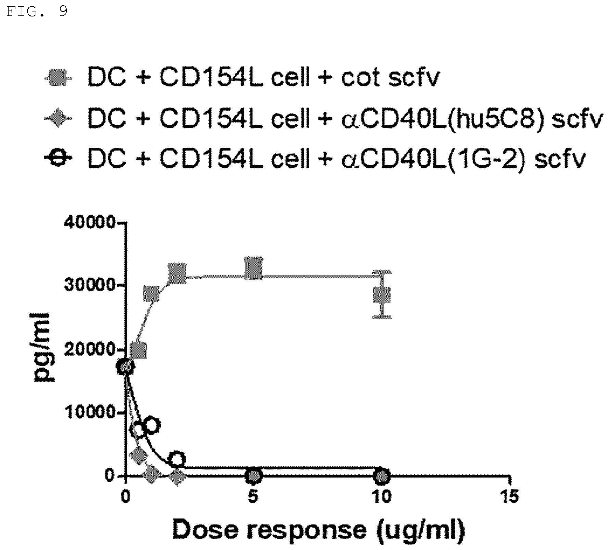

To identify whether treatment with the anti-CD40L antibody inhibits cytokine production by dendritic cells, the amount of IL-12 (interleukin-12) secreted into the supernatant of the co-culture system (culture of dendritic cells, CD154L cells, and anti-CD40L antibody) was measured using an IL-12 ELISA. The results are shown in FIG. 9.

As shown in FIG. 9, it was identified that the treatment with anti-CD40L antibody (1G-2) effectively inhibited IL-12 production (FIG. 9).

### 1.1.7. Identification of binding affinity for CD40L

Through the above Examples 1.1.1. to 1.1.6., it was identified that among the anti-CD40L antibodies, the 1G-2 clone can effectively inhibit the function of CD40L.

To measure the binding affinity between the 1G-2 clone and the CD40L protein, Biacore T200 (GE Healthcare) was used, and the measurement was performed by surface plasmon resonance (SPR) at 25°C. Binding affinity measurement using Biacore T200 was performed through association, dissociation, and regeneration processes by injecting an analyte onto the chip surface on which the ligand is immobilized. Affinity measurement values were calculated using the 1:1 binding model of BIAevaluation software version 1.0, and the binding affinity measurement was performed by immobilizing the antibody on the CM5 chip surface. 2B1-Ck-1G2 and 1G2-Ck-COT were immobilized at 300 RU on the CM5 chip surface at pH 4.5 and pH 5.0, respectively. The 2B1 was used as a partner antibody to identify the effect of 1G2 clone in a bispecific antibody format, similar to the anti-cotinine antibody. The binding affinity was measured by diluting CD40L protein by 1/2 increments from 5 nM, with association/dissociation times of 3 minutes each, at a flow rate of 30 µl/min. In each cycle, 10 mM glycine pH 2.0 containing 500 mM NaCl was used for regeneration at a flow rate of 30 µl/min for 30 seconds.

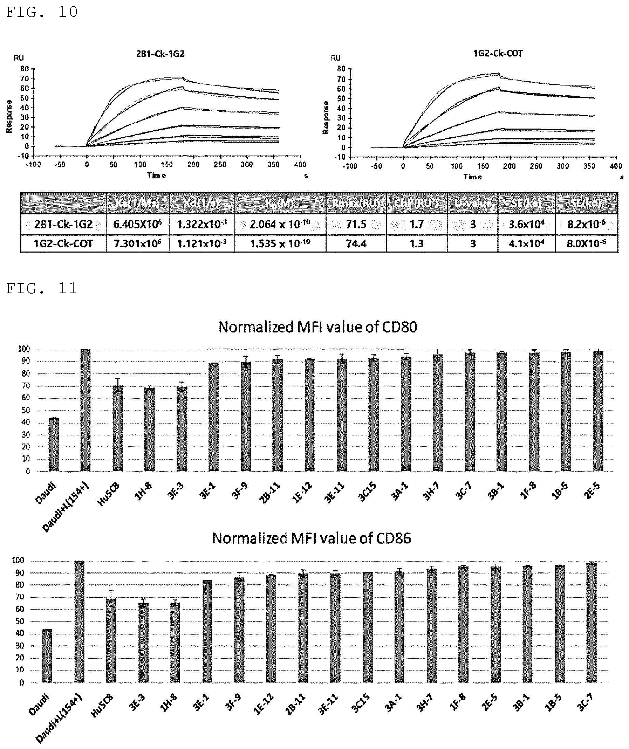

The measurement results for the binding affinity between the 1G-2 clone and the CD40L protein are shown in FIG. 10. The binding affinity measurement showed a KD value of 0.1 to 0.2 nM, indicating a high on rate (FIG. 10).

### Example 1-2. Selection of anti-CD40L antibodies (backup antibodies)

To further select anti-CD40L antibodies having a superior B-cell activation inhibitory ability, the B-cell activation inhibitory ability of 15 antibodies, which bind to human CD40L recombinant protein and have different complementarity determining region (CDR) sequences was evaluated using the human CD40-expressing peripheral blood lymphoma cell line (Daudi cells) (KCLB, Seoul, Republic of Korea) and the human CD40L-expressing mouse fibroblast cell line (CD154L cell).

The Daudi cells, the CD154L cells, and the anti-CD40L antibody (2 µg/ml) were co-cultured, and then the expression level of co-stimulatory molecules (CD80, CD86) on the Daudi cells was checked through flow cytometry. The results are shown in FIG. 11.

Comparison of the 15 anti-CD40L scFv antibodies with the positive control (hu5C8) in terms of the ability to inhibit co-stimulatory molecules on Daudi cells showed that 1H-8 and 3E-3 clones exhibited an inhibitory ability similar to the positive control (FIG. 11).

Considering Examples 1-1 and 1-2 together, 1G-2, 1H-8, and 3E-3 clones were selected as anti-CD40L antibodies. The CDR and variable region sequences of these antibodies are summarized in the table below:

**[Table 8]**

| [3E-3 clone] | |
|---|---|
| Region | Sequence |
| L-CDR1 | SGGYSSYGIYYYG (SEQ ID NO 1) |
| L-CDR2 | NGNKRPS (SEQ ID NO 4) |
| L-CDR3 | GSGDSSSDSGI (SEQ ID NO 7) |
| H-CDR1 | GFTFSSYGMN (SEQ ID NO 10) |
| H-CDR2 | AISNDGSSTSYAPAVKG (SEQ ID NO 13) |
| H-CDR3 | ESTNGGWVADDIDA (SEQ ID NO 16) |
| VL | ALTQPSSVSANLGGTVKITCSGGYSSYGIYYYGWYQQKSPGSAPVTLIYNGNKRP SNIPSRFSGSTSGSTNTLTITGVQVDDEAVYFCGSGDSSSDSGIFGAGTTLTVL (SEQ ID NO 47) |
| Linker | GQSSRSSGGGGSSGGGGS (SEQ ID NO 93) |
| VH | AVTLDESGGGLQTPGGALSLVCKASGFTFSSYGMNWVRQAPGKGLEWVGAISNDG SSTSYAPAVKGRATISRDNGQSTVRLQLNNLRAEDTATYYCAKESTNGGWVADDI DAWGHGTEVIVSS (SEQ ID NO 51) |

**[Table 9]**

| [1G-2 clone] | |
|---|---|
| Region | Sequence |
| L-CDR1 | SGDSSWYG (SEQ ID NO 2) |
| L-CDR2 | ANTNRPS (SEQ ID NO 5) |
| L-CDR3 | GSADSTYTAA (SEQ ID NO 8) |
| H-CDR1 | GFDFSSYDMN (SEQ ID NO 11) |
| H-CDR2 | GIYDDGSGTVYAPAVKG (SEQ ID NO 14) |
| H-CDR3 | GGGADNIDA (SEQ ID NO 17) |
| VL | LTQASSVSANLGGTVEITCSGDSSWYGWYQQKSPGSAPVTLIYANTNRPSDIPSR FSGSTSGSTGTLTITGVQADDEAVYYCGSADSTYTAAFGAGTTLTVL (SEQ ID NO 49) |
| Linker | GQSSRSSGGGGSSGGGGS (SEQ ID NO 93) |
| VH | AVTLDESGGGLQTPGGTLSLVCKASGFDFSSYDMNWVRQAPGKGLEYVAGIYDDG SGTVYAPAVKGRATISRDNGQSTVRLQLNNLRAEDTGTYYCTRGGGADNIDAWGH GTEVIVSS (SEQ ID NO 56) |

**[Table 10]**

| [1H-8 clone] | |
|---|---|
| Region | Sequence |
| L-CDR1 | SGGSGSYG (SEQ ID NO 3) |
| L-CDR2 | WDDERPS (SEQ ID NO 6) |
| L-CDR3 | GGYDDSSYAGI (SEQ ID NO 9) |
| H-CDR1 | GFTFSSYGMQ (SEQ ID NO 12) |
| H-CDR2 | AIDDDGSSTNYGAAVKG (SEQ ID NO 15) |
| H-CDR3 | DGIIYYWNADAGYIDA (SEQ ID NO 18) |
| VL | LTQPSSVSANPGETVKITCSGGSGSYGWYQQKSPGSAPVTLIYWDDERPSDIPSR FSGSKSGSTATLTITGVQAEDEAVYFCGGYDDSSYAGIFGAGTTLTVL    (SEQ ID NO 50) |
| Linker | GQSSRSSGGGGSSGGGGS (SEQ ID NO 93) |
| VH | AVTLDESGGGLHTPGGALSLVCKASGFTFSSYGMQWVRQAPGKGLEFVAAIDDDG SSTNYGAAVKGRATISRDNGQSTVRLQLNNLRAEDTGTYYCAKDGIIYYWNADAG YIDAWGHGTEVIVSS (SEQ ID NO 57) |

In the above tables, the CDR sequences (L-CDR1, L-CDR2, L-CDR3, H-CDR1, H-CDR2, H-CDR3) included in the light chain and heavy chain variable regions are underlined.

### Examples 1-3. De-immunization of antibodies

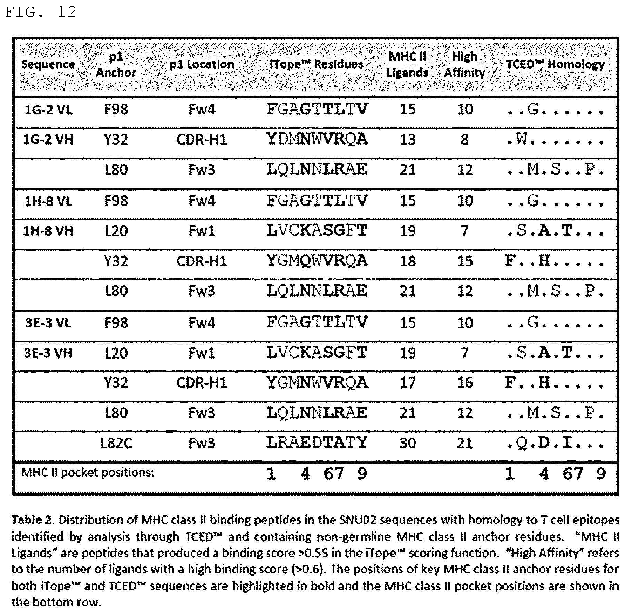

Since antibodies selected from an animal immune library may be immunogenic when administered to humans, the antibodies were optimized to exhibit low immunogenicity while having high affinity for a target antigen. To analyze immunogenicity of the selected chicken-derived anti-CD40L antibodies (1G-2, 1H-8, 3E-3), a T cell epitope content thereof was evaluated using immunology informatics tools, through in silico antibody sequence analysis by Abzena Ltd (UK) (FIG. 12). To select antibodies that exhibit low immunogenicity to humans while having high affinity for CD40L, libraries of non-immunogenic mutant antibodies were constructed by replacing the highly immunogenic regions in the antibody sequences with human germline amino acids.

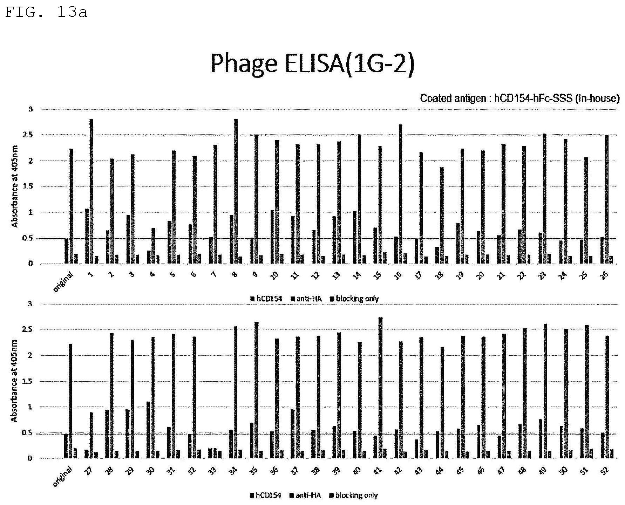

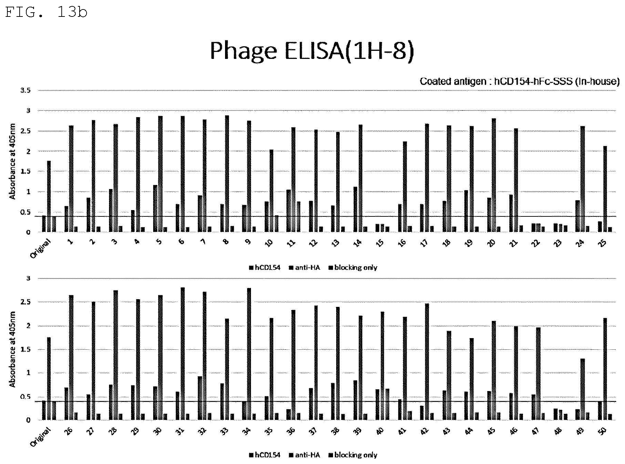

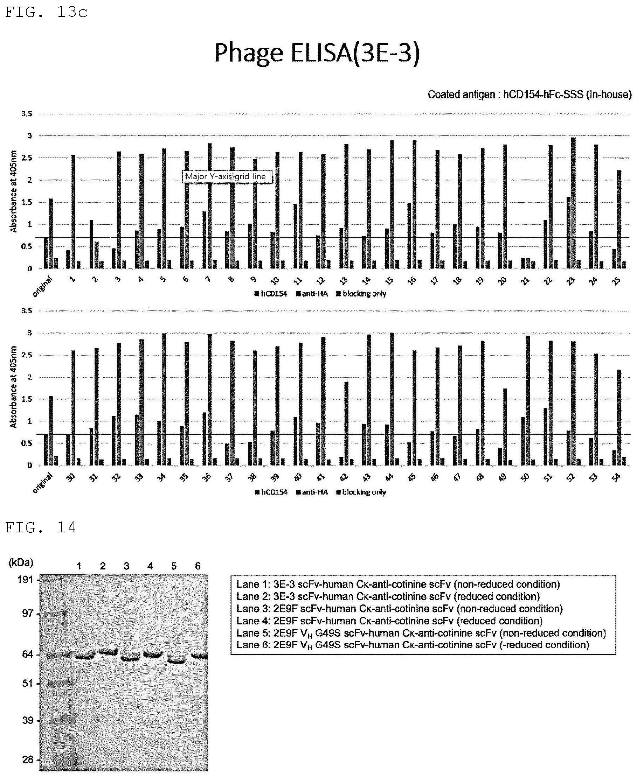

The constructed three non-immunogenic antibody libraries (1G-2, 1H-8, and 3E-3) had complexity of 5.4 x 10⁸, 4.3 x 10⁹, and 2.6 x 10⁸, respectively. Bio-panning was performed against the human CD40L recombinant protein using the constructed non-immunogenic antibody libraries. Through an enzyme-linked immunoassay, 52, 50, and 54 clones, which bind to the human CD40L recombinant protein, were obtained from the respective libraries. In addition, 41, 40, and 38 clones, which showed higher affinity compared to the existing wild-type antibody clones, were selected from the respective libraries, and the genetic information thereof was obtained through sequencing (FIGS. 13a to 13c).

Among the candidate antibodies, for 2E9F derived from the 3E-3 clone, 15 out of its total 16 immunogenic residues were substituted with the same residues as the human germline amino acids, and the remaining unsubstituted V_{H}G49 amino acid residue was substituted with serine, which is identical to the human germline amino acid, thereby synthesizing an antibody gene having no immunogenicity. The selected non-immunogenic antibodies 2E9F and 2E9F V_{H}G49S were cloned into a corresponding mammalian cell expression vector for expression in an scFv-Ck-scFv format. The antibodies were overexpressed through transformation of human embryonic kidney 293F (HEK293F) cells, and the recombinant antibodies were then purified from the cell culture using affinity chromatography (FIG. 14).

### 1.3.1. Identification of binding to human CD40L

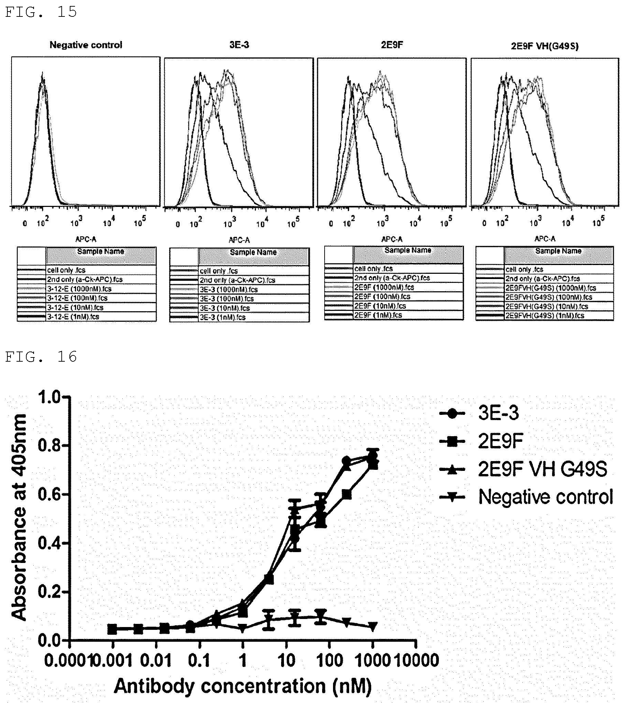

It was identified through flow cytometry that the three anti-CD40L antibodies (3E-3 clone, de-immunized 2E9F clone, and 2E9F VH G49S clone) bound to the human CD40L-expressing Jurkat D1.1 (hCD40L+) cell line (FIG. 15).

In addition, it was identified through an enzyme-linked immunosorbent assay (ELISA) that the three anti-CD40L antibodies bound to human CD40L (a recombinant protein in hCD40L-isoleucine zipper-hemagglutinin format) (R&D Systems, Minneapolis, MN, USA) (FIG. 16).

### 1.3.2. Inhibitory ability of CD40/CD40L binding

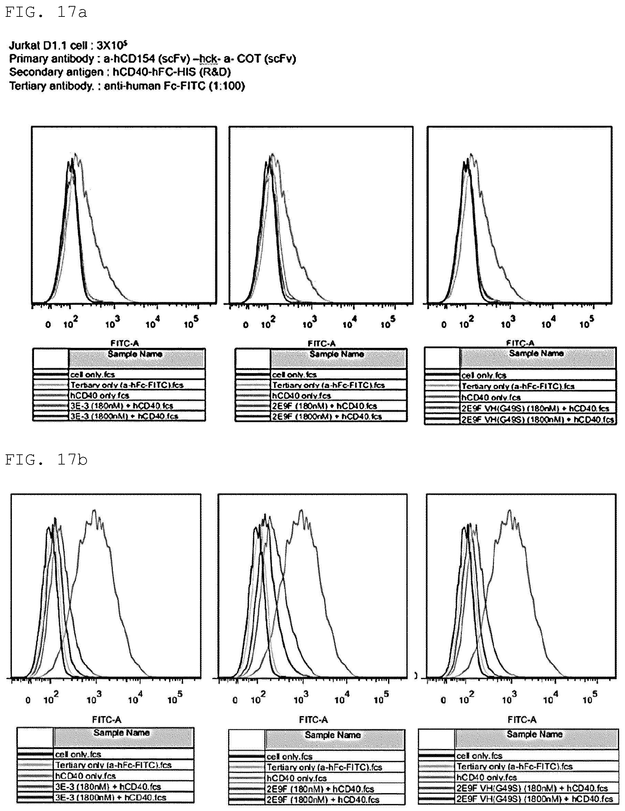

The Jurkat D1.1 (hCD40L+) cell line or the CD154L (hCD40L+) cell line were treated with the de-immunized 2E9F clone and the 2E9F VH G49S clone, followed by treatment with CD40 antigen, and analyzed through flow cytometry. As a result, it was identified that both the 2E9F clone and the 2E9F VH G49S clone inhibited the CD40/CD40L interaction (FIGS. 17a and 17b).

The anti-CD40L antibodies (1G-2, 1H-8) selected in Examples 1-2 were also completely de-immunized using the same method as described above.

### Example 1-4. Humanization of antibodies

To improve antibody safety, humanization was performed using a method of substituting the framework regions, which exclude the complementarity-determining regions (CDRs), of the chicken-derived anti-CD40L antibodies with sequences identical to the human germline sequence. Simultaneously with the antibody humanization, to select antibodies with high affinity for CD40L, mutant antibody libraries containing both human germline amino acids and the amino acid sequences of existing antibodies were constructed using the Trimer controlled library (GENEWIZ, NJ, USA) method.

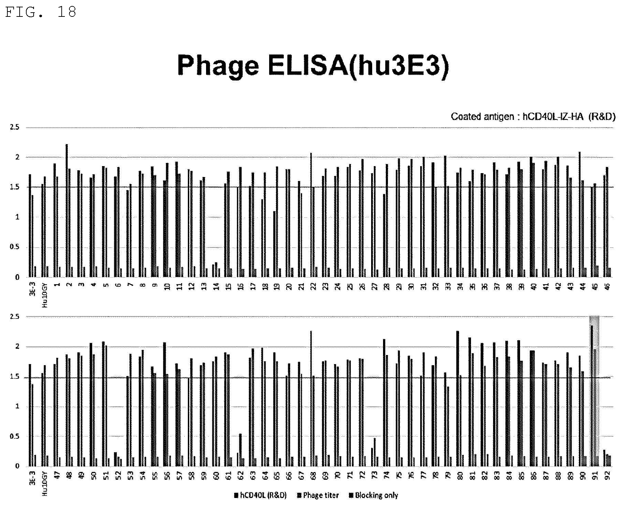

To additionally humanize the remaining 10 chicken-derived residues in the 3E-3 clone, a secondary humanized library was constructed using the Trimer controlled library provided by GENEWIZ. The constructed humanized library had complexity of 5.0 × 10⁸, and the total number of amino acid residues to be substituted was 31. Bio-panning was performed against the human CD40L recombinant protein using the constructed humanized antibody library. Through an enzyme-linked immunoassay, a total of 87 clones, which bind to the human CD40L recombinant protein, were obtained. In addition, 79 clones, which showed higher affinity compared to the existing wild-type antibody clone, were selected, and the genetic information thereof was obtained through sequencing (FIG. 18).

As a result, clone #91 was obtained, in which 28 out of the total 31 residues were substituted with sequences identical to the human germline amino acid sequence.

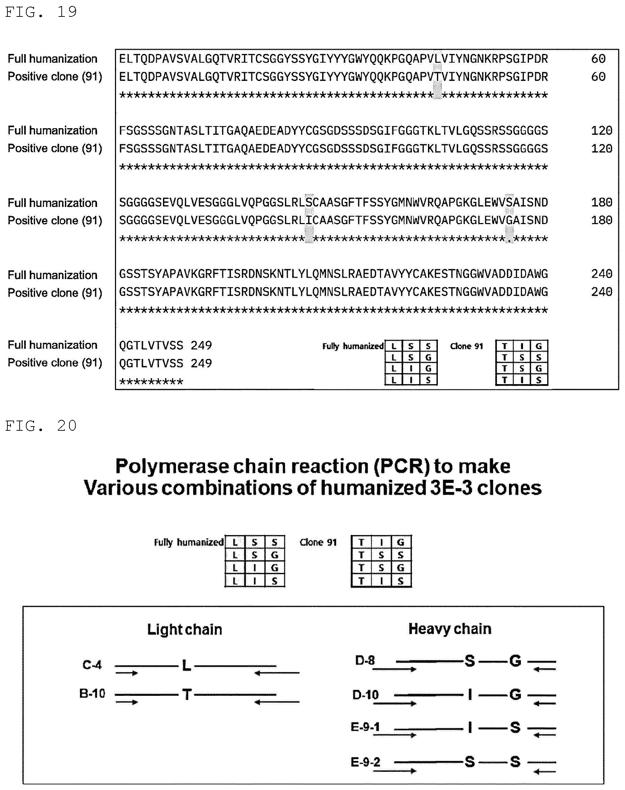

FIG. 19 illustrates the result of alignment between the sequence having the fully humanized framework region and the sequence of the acquired clone #91. The obtained clones were subjected to polymerase chain reaction (PCR) to generate eight clone combinations that comprise both human- and chicken-derived residues for the three non-humanized chicken-derived residues, and the combinations were cloned into a phagemid vector (FIG. 20) (these three chicken-derived residues are highlighted in red in FIG. 19).

The three non-humanized chicken-derived residues correspond to the 12th residue from the N terminus of the L-FR2 region (humanized sequence: L, sequence of clone #91: T), the 21st residue from the N terminus of the H-FR1 region (humanized sequence: S, sequence of clone #91: I), and the 14th residue from the N terminus of the H-FR2 region (humanized sequence: S, sequence of clone #91: G). Combination of the possible cases resulted in the eight clones designated as LSS, LSG, LIS, LIG, TSS, TSG, TIS, and TIG. For example, the "LSS" clone is a clone in which the 12th residue from the N terminus of the L-FR2 region corresponds to "L", the 21st residue from the N terminus of the H-FR1 region corresponds to "S", and the 14th residue from the N terminus of the H-FR2 region corresponds to "S."

### 1.4.1. Identification of binding to human CD40L

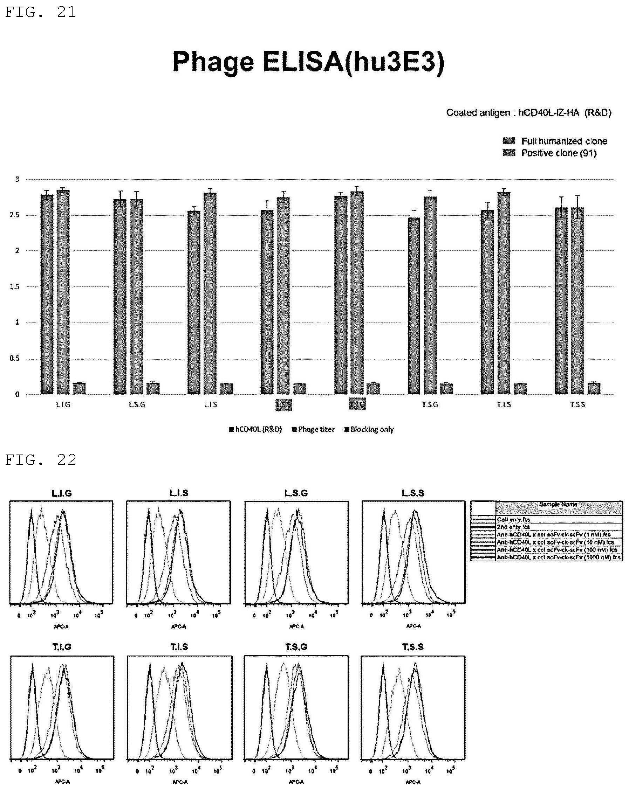

A phage enzyme immunoassay was performed using the 8 clones (LSS, LSG, LIS, LIG, TSS, TSG, TIS, and TIG clones) for which cloning had been completed. As a result, it was identified that all combinations of clones exhibited a binding ability to the human CD40L recombinant protein in the phage enzyme immunoassay (FIG. 21).

### 1.4.2. Identification of binding to L cell line

To express the 8 humanized antibody clones, which exhibited a binding ability to the human CD40L recombinant protein in the phage enzyme immunoassay, in an scFv-Ck-scFv format, they were cloned into the corresponding mammalian cell expression vectors. The antibodies were overexpressed through transformation of human embryonic kidney 293F (HEK293F) cells, and then purified from the cell culture using affinity chromatography. It was identified through flow cytometry that the expressed and purified 8 antibody clones bound to the L cell line overexpressing human CD40L (FIG. 22).

### 1.4.3. Identification of inhibition of binding between human CD40 and L cell line

Flow cytometry was performed to identify whether the anti-CD40L antibodies inhibit binding of human CD40 protein to the L cell line overexpressing hCD40L.

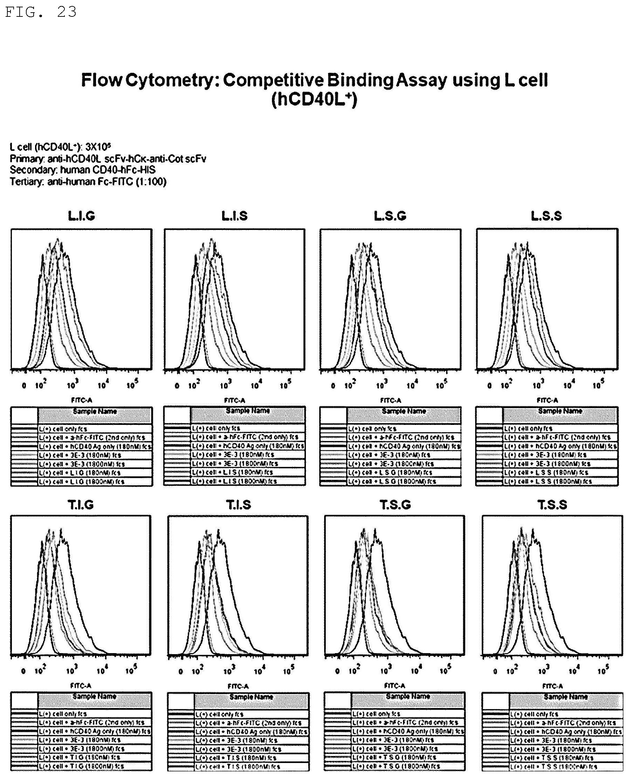

Among the prepared 8 clones (LSS, LSG, LIS, LIG, TSS, TSG, TIS, and TIG clones), it was identified that the TSS, TSG, TIS, and TIG clones exhibited a superior binding inhibitory ability (FIG. 23).

### 1.4.4. Identification of inhibition of expression of B-cell co-stimulatory molecules

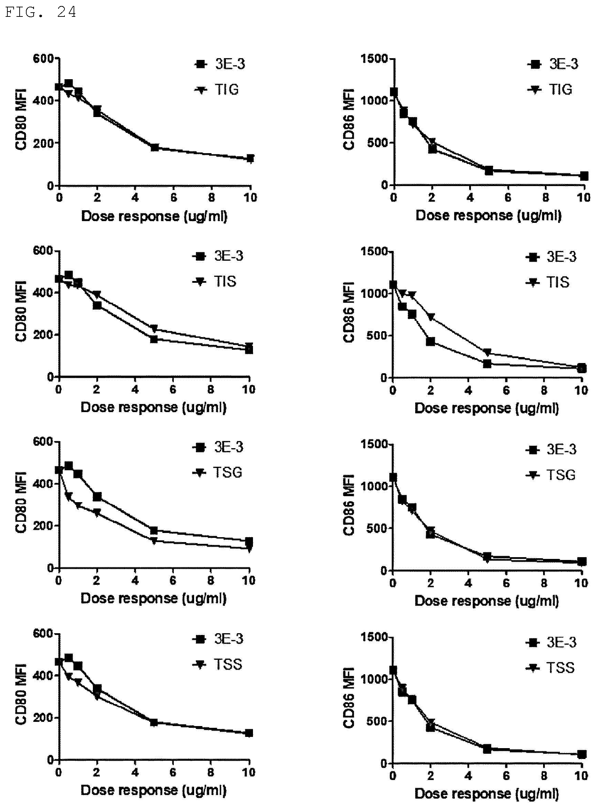

The TSS, TSG, TIS, and TIG clones were examined at various concentrations (2, 4, 6, 8, 10 µg/ml) for their ability to inhibit expression of B-cell co-stimulatory molecules (CD80, CD86). As a result, it was identified that the TSG clone exhibited the highest ability to inhibit expression of B-cell co-stimulatory molecules (FIG. 24). Therefore, the TSG clone was finally selected as the lead antibody (hu3E-3) among the humanized antibodies, with the TSS, TIS, and TIG clones kept as backup antibodies. The fully humanized TSG clone contains only two chicken-derived residues in the framework region, with all other regions substituted with sequences identical to the human germline sequence.

Among the 8 clones, the CDR and variable region sequences of the TSG clone are illustratively summarized in the table below:

**[Table 11]**

| [TSG clone (hu3E-3 clone)] | |
|---|---|
| Region | Sequence |
| L-CDR1 | SGGYSSYGIYYYG (SEQ ID NO 1) |
| L-CDR2 | NGNKRPS (SEQ ID NO 4) |
| L-CDR3 | GSGDSSSDSGI (SEQ ID NO 7) |
| H-CDR1 | GFTFSSYGMN (SEQ ID NO 10) |
| H-CDR2 | AISNDGSSTSYAPAVKG (SEQ ID NO 13) |
| H-CDR3 | ESTNGGWVADDIDA (SEQ ID NO 16) |
| VL | ELTQDPAVSVALGQTVRITCSGGYSSYGIYYYGWYQQKPGQAPV[T]VIYNGNKR PSGIPDRFSGSSSGNTASLTITGAQAEDEADYYCGSGDSSSDSGIFGGGTKLTVL (SEQ ID NO 48) |
| Linker | GQSSRSSGGGGSSGGGGS (SEQ ID NO 93) |
| VH | EVQLVESGGGLVQPGGSLRL[S]CAASGFTFSSYGMNWVRQAPGKGLEWV[G]AI SNDGSSTSYAPAVKGRFTISRDNSKNTLYLQMNSLRAEDTAVYYCAKESTNGGWV ADDIDAWGQGTLVTVSS (SEQ ID NO 53) |

In the table above, the CDR sequences (L-CDR1, L-CDR2, L-CDR3, H-CDR1, H-CDR2, H-CDR3) included in the light chain and heavy chain variable regions are underlined. In addition, the 12th residue from the N terminus of the L-FR2 region, the 21st residue from the N terminus of the H-FR1 region, and the 14th residue from the N terminus of the H-FR2 region are indicated by brackets and bold underlining.

### 1.4.5. Measurement of binding affinity of anti-CD40L antibody

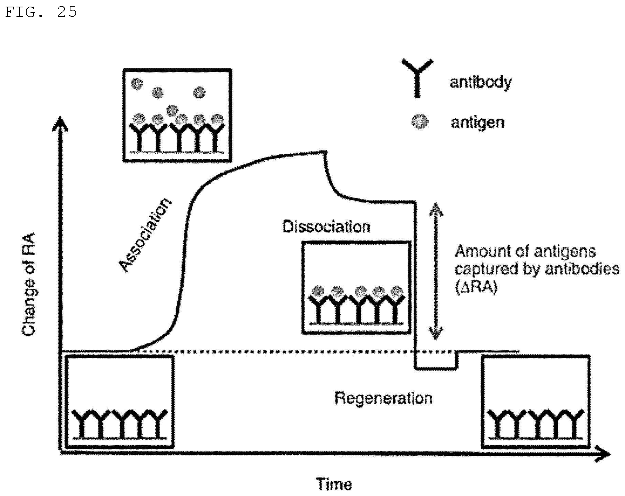

The binding affinity of the 3 anti-CD40L antibodies (1H-8 clone, TSG clone, and 3E-3 clone) to CD40L was measured. To measure the antigen-antibody binding affinity, Biacore T200 (GE Healthcare) was used, and measurement was performed at 25°C using surface plasmon resonance (SPR). The binding affinity measurement using Biacore was performed through association, dissociation, and regeneration processes by injecting an analyte onto the chip surface on which the ligand is immobilized (see the schematic diagram in FIG. 25). Affinity measurement values were calculated using the 1:1 binding model of BIAevaluation software version 1.0.

The binding affinity measurement was performed by immobilizing CD40L (rhCD40 Ligand, R&D Systems, 6420-CL/CF) protein onto the CM5 chip surface. The rhCD40 Ligand was immobilized onto the CM5 chip surface at a level of 100 RU under pH 5.5 conditions. HBS-EP (GE Healthcare, BR-10-0669) was used as the sample and system buffer. The binding affinity was measured for the 3 bispecific antibodies in scFv-Ck-scFv format [structure of anti-CD40L scFv antibody (N'-VL-VH-C')-Ck-anti-cotinine antibody (N'-VL-VH-C'), see Example 1-1], with association/dissociation times set to 4 min/3 min, respectively, at a flow rate of 30 µl/min. In each cycle, 10 mM glycine (pH 2.0) was used for regeneration at a flow rate of 30 µl/min for 30 seconds.

Table 12 below shows the conditions for measuring the binding affinity of the anti-CD40L antibodies.

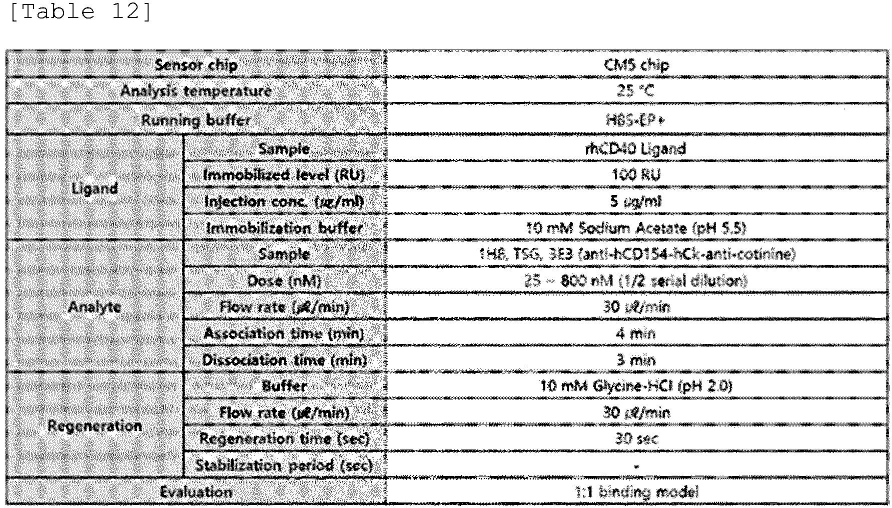

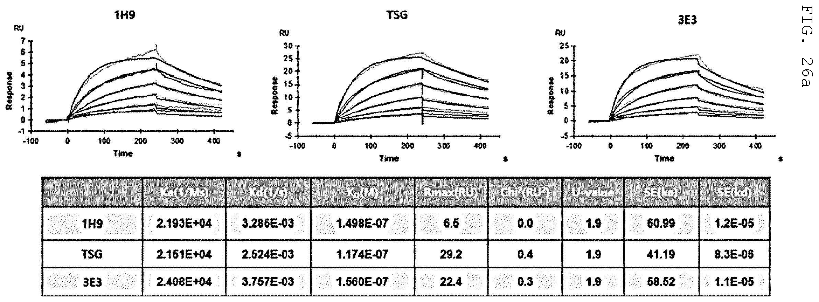

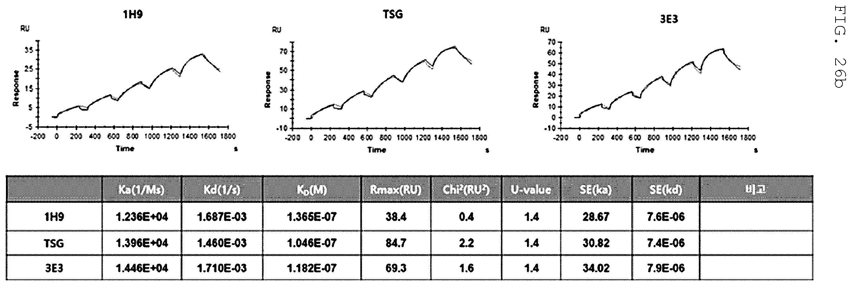

The results obtained by measuring CD40L binding affinity using the multi-kinetics method showed that the antibody regeneration was not stable. Thus, additional experiments were performed using the single kinetics method that does not involve the regeneration step. The results are shown in FIGS. 26a and 26b, respectively.

It is known that the K_{D} value does not vary depending on the single kinetics and multi-kinetics methods. In this example, it was also identified that there was no significant difference in the K_{D} values of the 1H8 clone, TSG clone, and 3E-3 clone between the two methods.

### Example 2. Anti-CD28 antibody

### Example 2-1. Selection of anti-CD28 antibody (lead antibody)

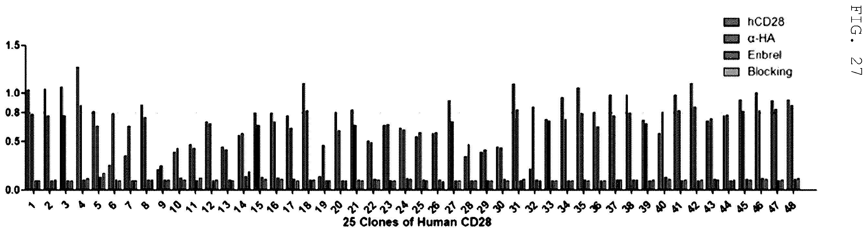

An antibody library in an scFv form was constructed by immunizing chickens (white leghorn chicken, 3 animals, Biopoa Co., Ltd.) with human CD28 recombinant protein (R&D Systems, Minneapolis, MN, USA). The constructed three chicken antibody libraries had complexity of 1.5 x 10⁹, 1.3 x 10⁹, 1.3 x 10⁹, and 9.4 x 10⁸, respectively. Bio-panning against human CD28 was performed using the chicken antibody libraries. Subsequently, 49 clones that bind to human CD28 were selected in an enzyme-linked immunosorbent assay, and the genetic information thereof was obtained through sequencing (FIG. 27).

To produce 12 antibody clones that bind to human CD28 recombinant protein in scFv-Ck-scFv-HIS-HA fusion protein format [anti-CD28 scFv antibody (N'-VL-linker-VH-C') - Ck (SEQ ID NO: 95) - anti-cotinine scFv antibody (N'-VL-linker-VH-C') - HIS-HA, see Example 1-1], cloning was performed into an animal cell expression vector. The expression vector was used to transform human embryonic kidney 293F (HEK293F) cells, and then the antibodies were purified from the cell culture using affinity chromatography.

**[Table 13]**

| | Transfection volume (mL) | Quantification | |
|---|---|---|---|
| | | µg | mg/L |
| FR104 | 50 | 47 | 0.94 |
| 2-7 | 50 | 895 | 17.9 |
| 4-6 | 50 | 1219 | 24.4 |
| 4-14 | 50 | 532 | 10.6 |
| 4-8 | 50 | 1149 | 23 |
| 4-77 | 50 | 250 | 5 |
| 6-1 | 50 | 763 | 15.2 |
| 6-8 | 50 | 177 | 3.54 |
| 6-2 | 50 | 207 | 4.14 |
| 6-3 | 50 | 718 | 14.3 |
| 6-5 | 50 | 220 | 4.4 |
| 6-18 | 50 | 1149 | 29.6 |
| 6-73 | 50 | 447 | 8.94 |

### 2.1.1. Identification of binding to human CD28

It was identified through flow cytometry whether the expressed and purified anti-CD28 antibodies bound to the Jurkat E6.1 (Human CD28+) cell line (ATCC, Manassas, VA, USA). As a positive control, an antibody that specifically binds to CD28 (FR104 clinical candidate from OSE Immunotherapies, J Immunol. 2016. 197(12):4593-4602) was cloned and purified in the same format for comparison.

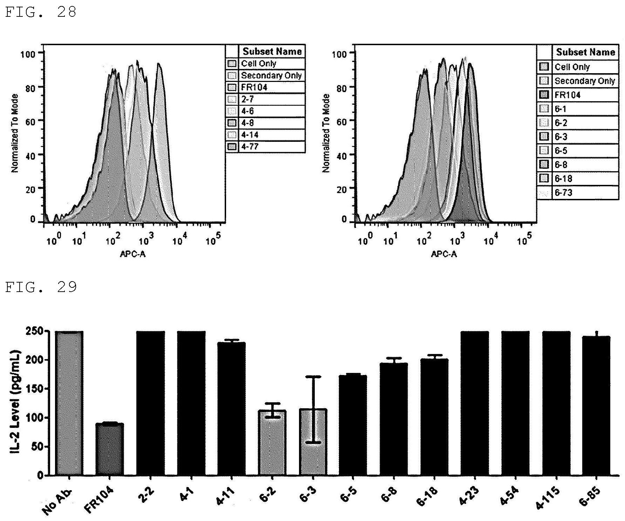

As a result, it was identified that the expressed and purified 12 anti-CD28 antibodies bound to the Jurkat E6.1 (Human CD28+) cell line (FIG. 28).

### 2.1.2. Ability to inhibit cytokine secretion in Jurkat E6.1 cell line (in vitro)

For T cells to be activated, the T cell receptor (TCR) on the T cell surface must recognize an antigen presented on the surface of an antigen-presenting cell (APC). Here, it is known that T cells are activated only when they receive a signal (signal 1) transmitted through the TCR along with a costimulatory signal (signal 2) transmitted by binding of B7 molecules (B7.1 [CD80], B7.2 [CD86]) on the APC surface to CD28 on the T cell surface (Cancer Res. 2001 61(5): 1976-82).

Therefore, it was evaluated whether the anti-CD28 antibodies could inhibit human T cell activation by blocking CD28-B7 binding. When the Jurkat E6.1 cell line, which is a CD28-positive T cell line, are co-cultured with the TCR-stimulating antibody OKT3-Ck-HA (US 5885573 A) and the Raji cell line, which is a CD80/CD86-positive B cell line, T cells are activated and secrete IL-2 (interleukin-2). It was checked whether treatment of the co-culture system with the anti-CD28 antibody inhibited the IL-2 secretion ability of T cells.

Specifically, the Jurkat E6.1 cell line, the Raji cell line, OKT3 (10 nM), and the anti-CD28 antibody (1 µM) were co-cultured. After 48 hours, the supernatant of the culture was harvested and an IL-2 ELISA was performed. As a positive control, the FR104 antibody, which had been expressed in FR104 scFv-Ck-Cot scFv format [structure of FR104 scFv antibody (N'-VL-VH-C')-Ck (SEQ ID NO: 95)-anti-cotinine antibody (N'-VL-VH-C'), see Example 1-1], was used.

The 12 anti-CD28 antibodies were checked for their ability to inhibit IL-2 secretion in the Jurkat E6.1 cell line. As a result, it was identified that the J6-2 and J6-3 clones exhibited an inhibitory ability similar to the positive control (FIG. 29).

To identify whether the above experimental results could be reproduced in human primary T cells, an additional experiment was conducted. CD4-positive T cells and monocytes were isolated from the leukocytes obtained by leukapheresis from healthy adults using the MACS cell separation method. The isolated monocytes were differentiated into immature dendritic cells by treatment with GM-CSF (granulocyte-macrophage colony-stimulating factor) and IL-4 (interleukin-4). The resulting cells were then matured by treatment with LPS (lipopolysaccharide, 1 µg/ml) for 24 hours and used as antigen-presenting cells for T cell stimulation. Co-culture of the CD4 T cells with the dendritic cells and an anti-CD3 antibody for TCR stimulation induces T cell activation. Here, the anti-CD28 antibody was added thereto to verify its T cell activation inhibitory effect.

Specifically, the CD4 T cells, the dendritic cells, the anti-CD3 antibody (2 µg/ml), and the purified anti-CD28 antibody (10 µg/ml) were co-cultured. After 72 hours, the supernatant was collected, and IL-2 and IFN-gamma (interferon gamma) ELISAs were performed.

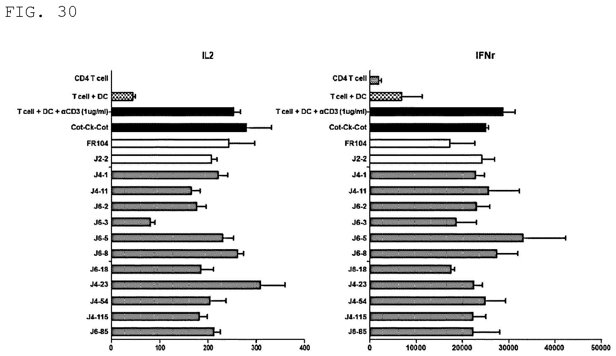

As a result, among the 12 anti-CD28 antibodies, the J6-3 clone exhibited a higher IL-2 inhibitory ability than the positive control (FR104) and exhibited an IFN gamma secretion inhibitory ability similar to the positive control (FIG. 30).

### 2.1.3. Identification of competitive binding with CD80/CD86 proteins

To further identify binding specificity of the J6-3 clone to CD28 on the T cell surface, a competitive binding assay was performed using recombinant CD80 and CD86 proteins.

All CD28 expressed on the surface of Jurkat E6.1 cells was masked by treating the Jurkat E6.1 cells with the J6-3 clone for 1 hour (1500 nM). Subsequently, the Jurkat E6.1 cells were washed, and then treated with recombinant CD80 and CD86 proteins (500 nM) to determine whether the J6-3 clone inhibited binding of these recombinant proteins. The binding degree of the CD80/CD86 proteins, which are in the form of Fc-fusion proteins, was analyzed by a flow cytometer using a fluorescently labeled anti-Fc secondary antibody.

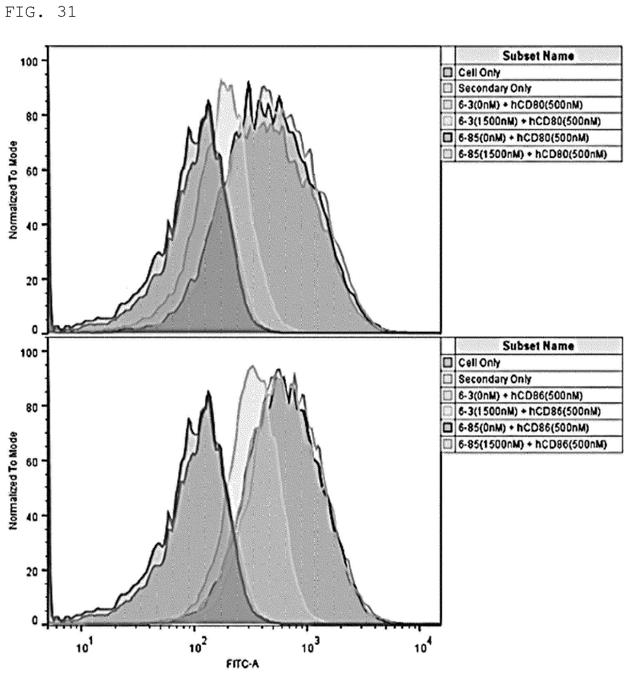

As a result, it was identified that the binding affinity of the CD80/CD86 proteins was significantly decreased in the cells treated with the J6-3 clone compared to the cells not treated with the J6-3 clone. On the contrary, such a decrease in the binding affinity of the CD80/CD86 proteins was not observed in the cells treated with a negative control antibody (J6-85 clone), which demonstrates specificity of the J6-3 clone (FIG. 31).

Accordingly, it was identified that the J6-3 clone competitively bound to CD28 on the cell surface with the CD80/CD86 proteins.

The CDR and variable region sequences of the J6-3 clone are summarized in the table below:

**[Table 14]**

| [J6-3 clone] | |
|---|---|
| Region | Sequence |
| L-CDR1 | SGGGSNNYG (SEQ ID NO 65) |
| L-CDR2 | YNDKRPS (SEQ ID NO 66) |
| L-CDR3 | GSRDSSHDGI (SEQ ID NO 67) |
| H-CDR1 | GFTFSTFNMF (SEQ ID NO 68) |
| H-CDR2 | QISSNDGSWTGYGAAVKG (SEQ ID NO 70) |
| H-CDR3 | AAGGYNVCGYGCGGDYIDA (SEQ ID NO 71) |
| VL | MTQPSSVSANPGETVKITCSGGGSNNYGWYQQKSPGSAPVTVIYYNDKRPSGIPS RFSGSTSGSTGTLTITGVRAEDEAVYFCGSRDSSHDGIFGAGTTLTVL (SEQ ID NO 87) |
| Linker | GQSSRSSGGGGSSGGGGS (SEQ ID NO 93) |
| VH | AVTLDESGGGLQTPGGALSLVCKASGFTFSTFNMFWVRQAPGKGLEYVAQISSND GSWTGYGAAVKGRATISRDNGQSTVRLQLNNLRAEDTATYYCAKAAGGYNVCGYG CGGDYIDAWGHGTEVIVSSTS (SEQ ID NO 89) |

In the table above, the CDR sequences (L-CDR1, L-CDR2, L-CDR3, H-CDR1, H-CDR2, H-CDR3) included in the light chain and heavy chain variable regions are underlined.

### Example 2-2. Selection of anti-CD28 antibodies (backup antibodies)

To obtain additional backup antibodies, besides the J6-3 clone, from the anti-CD28 antibody library, clones that bind to CD28 were acquired through screening. Multiple additional antibody clones that have binding affinity to CD28 were selected, and gene sequence analysis was completed. The recombinant proteins were purified in the same format as the previously described J6-3 clone. The anti-CD28 antibody (at 1,000 nM) in scFv-Ck-scFv format [anti-CD28 scFv (N'-VH-VL-C')-Cκ-anti-cotinine scFv (N'-VH-VL-C'), see Example 1-1] was reacted with the CD28-positive Jurkat E6.1 cell line, and its binding to CD28 was checked through flow cytometry.

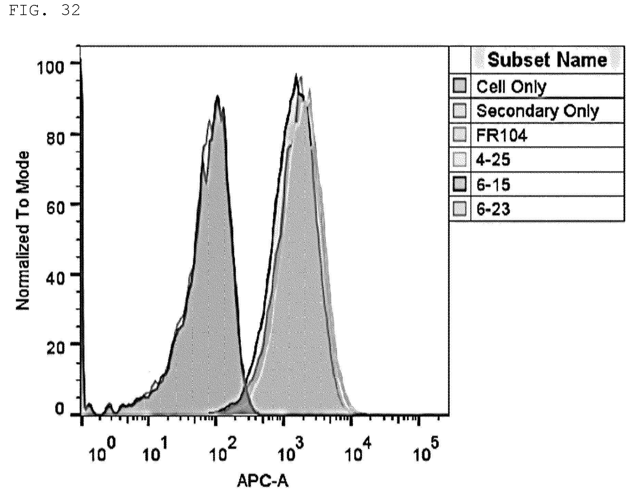

As a result, it was identified that 4-25, 6-15, and 6-23 clones bound similarly to the positive control (FR104) (FIG. 32).

Therefore, the three backup antibody clones (4-25, 6-15, and 6-23 clones) were obtained which bind to the Jurkat E6.1 cell line similarly to the positive control.

### Example 2-3. De-immunization of antibodies and preparation of humanized antibodies (1)

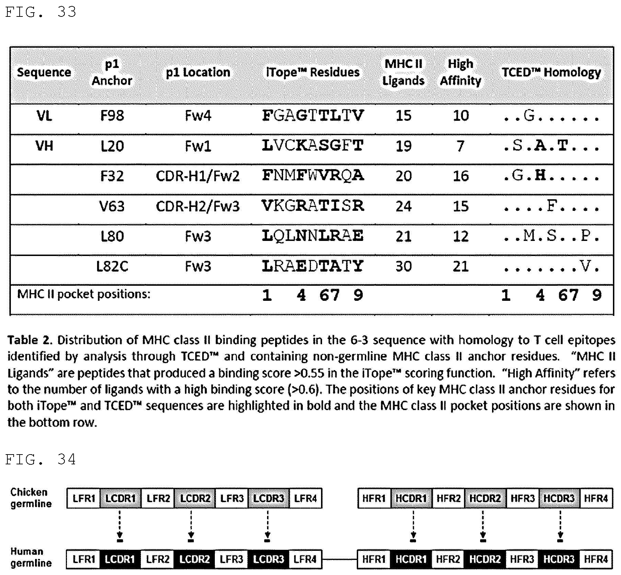

To check immunogenicity of the J6-3 clone selected through flow cytometry and interleukin-2 inhibition assay, immunogenicity analysis was performed through in silico antibody sequence analysis by Abzena Ltd (UK) (FIG. 33).

After checking the immunogenicity, the J6-3 clone selected in Example 2-1 was compared to the human germline sequence having the highest homology thereto for simultaneous de-immunization and humanization (see the schematic diagram in FIG. 34). A library was constructed using degenerate codons to replace the immunogenic portions within the complementary-determining regions (CDRs) with human germline sequences.

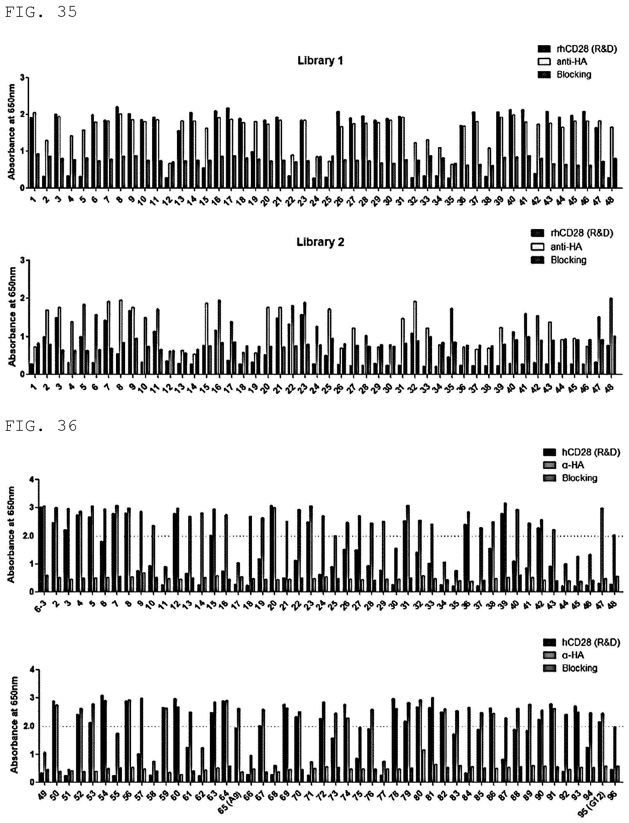

Bio-panning against human CD28 was performed using the constructed random library containing the human germline sequences. Sequencing was performed to obtain the genetic information of various clones that bound to human CD28 in an enzyme-linked immunosorbent assay (ELISA) (FIG. 35).

The sequencing failed to select the clones that perfectly matched the previous immunogenicity analysis results; however, it was identified that a large number of complementarity-determining regions had changed to the human germline sequences compared to the existing J6-3 clone.

The clone with the greatest number of substitutions compared to the J6-3 clone was selected, and a library was constructed therefrom using the same procedure as described above, considering only the unchanged regions. The newly constructed library was subjected to bio-panning again, followed by an enzyme-linked immunosorbent assay. Then, clone #95 (G12 clone) was finally obtained by sequencing (FIG. 36).

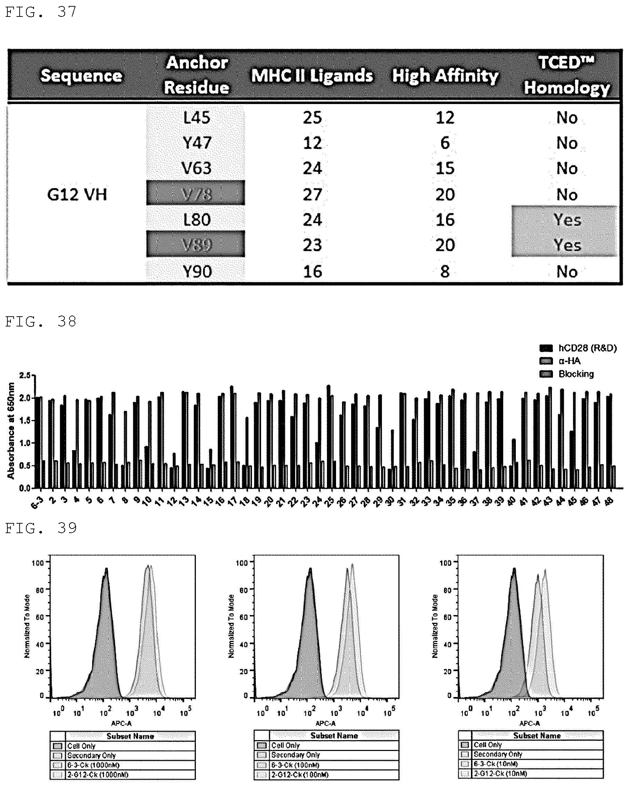

It was checked again whether the selected G12 clone was de-immunized. Analysis of the G12 clone revealed the presence of immunogenicity at a total of two sites (FIG. 37). A library was then constructed to change these sites to the human germline sequences. The library was subjected to bio-panning again, followed by an enzyme-linked immunosorbent assay. Then, sequencing was performed to establish a clone which is a de-immunized and humanized version of the original J6-3 clone (FIG. 38).

The clone obtained by the above de-immunization and humanization process was designated as 2-G12 clone. The CDR and variable region sequences of the 2-G12 clone are summarized in the table below:

**[Table 15]**

| [2-G12 clone] | |
|---|---|
| Region | Sequence |
| L-CDR1 | SGGGSNNYG (SEQ ID NO 65) |
| L-CDR2 | YNDKRPS (SEQ ID NO 66) |
| L-CDR3 | GSRDSSHDGI (SEQ ID NO 67) |
| H-CDR1 | GFTFSSYAMH (SEQ ID NO 69) |
| H-CDR2 | QISSNDGSWTGYGAAVKG (SEQ ID NO 70) |
| H-CDR3 | AAGGYNVCGYGCGGDYIDA (SEQ ID NO 71) |
| VL | LTQPSSVSANPGETVKITCSGGGSNNYGWYQQKPGSAPVTVIYYNDKRPSGIPSRF SGSTSGSTGTLTITGVRAEDEAVYFCGSRDSSHDGIFGGGTKLTVL (SEQ ID NO 88) |
| Linker | GQSSRSSGGGGSSGGGGS (SEQ ID NO 93) |
| VH | PVKLVESGGGLVTPGGSLRLSCSASGFTFSSYAMHWVRQAPGKGLEYVGQISSNDG SWTGYGAAVKGRFTISRDNSKSTLYLQMNSLRAEDTAVYYCAKAAGGYNVCGYGCG GDYIDAWGHGTQVTVSSTS (SEQ ID NO 90) |

In the table above, the CDR sequences (L-CDR1, L-CDR2, L-CDR3, H-CDR1, H-CDR2, H-CDR3) included in the light chain and heavy chain variable regions are underlined.

### 2.3.1. Binding ability to human CD28

To express the humanized antibody clone (2-G12), which exhibited a binding ability to the human CD28 recombinant protein in the phage enzyme immunoassay, in an scFv-Ck-scFv format [anti-CD28 scFv antibody (N'-VH-VL-C')-Ck-anti-cotinine antibody (N'-VH-VL-C')], the clone was cloned into the corresponding mammalian cell expression vector. The antibodies were overexpressed through transformation of human embryonic kidney 293F (HEK293F) cells, and the recombinant antibodies were then purified from the cell culture using affinity chromatography.

It was identified through flow cytometry that the expressed and purified anti-CD28 antibodies (J6-3 clone and humanized 2-G12 clone) bound to the Jurkat E6.1 (hCD28⁺) cell line (FIG. 39).

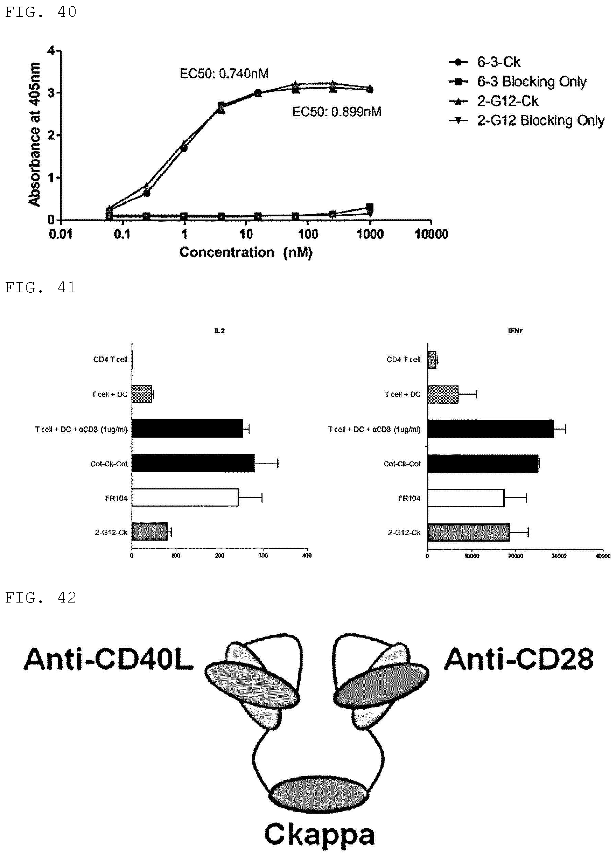

In addition, it was identified through an enzyme-linked immunosorbent assay that the J6-3 clone and the humanized 2-G12 clone bound to the human CD28 recombinant protein (hCD28) (R&D Systems, Minneapolis, MN, USA) (FIG. 40).

### 2.3.2. Ability to inhibit cytokine secretion in CD4 T cells

It was checked whether the humanized 2-G12 clone exhibits an ability to inhibit cytokine secretion in CD4 T cells. As a result, it was identified that the 2-G12 clone exhibited a superior IL-2 (interleukin-2) secretion inhibitory ability compared to the positive control (FR104) and exhibited an IFN gamma inhibitory ability similar to the positive control (FR104) (FIG. 41).

### Example 2-4. Preparation of humanized antibodies (2)

To improve antibody stability, additional humanization was performed by substituting the chicken-derived amino acid sequences remaining in the framework region of the 2-G12 clone with sequences identical to the human germline sequence. As a result, the Hu2G12 clone was obtained, in which 13 out of the 23 chicken-derived residues in the original 2-G12 framework region were additionally substituted with human germline sequences. The substituted residues are A13V, N14S, E17Q, V19A, K20R, S60D, T76S, and R79Q in the light chain (V_{L}) , and P1E, K3Q, T13Q, H105Q, and Q108E in the heavy chain (V_{H}).

The CDR and variable region sequences of the Hu2G12 clone are summarized in Table 16 below:

**[Table 16]**

| [Hu2G12 clone] | |
|---|---|
| Region | Sequence |
| L-CDR1 | SGGGSNNYG (SEQ ID NO 65) |
| L-CDR2 | YNDKRPS (SEQ ID NO 66) |
| L-CDR3 | GSRDSSHDGI (SEQ ID NO 67) |
| H-CDR1 | GFTFSSYAMH (SEQ ID NO 69) |
| H-CDR2 | QISSNDGSWTGYGAAVKG (SEQ ID NO 70) |
| H-CDR3 | AAGGYNVCGYGCGGDYIDA (SEQ ID NO 71) |
| VL | LTQPSSVSVSPGQTARITCSGGGSNNYGWYQQKPGSAPVTVIYYNDKRPS GIPERFSGSTSGSTGTLTISGVQAEDEAVYFCGSRDSSHDGIFGGGTKLT VL (SEQ ID NO 98) |
| Linker | GQSSRSSGGGGSSGGGGS (SEQ ID NO 93) |
| VH | EVQLVESGGGLVQPGGSLRLSCSASGFTFSSYAMHWVRQAPGKGLEYVGQ ISSNDGSWTGYGAAVKGRFTISRDNSKSTLYLQMNSLRAEDTAVYYCAKA AGGYNVCGYGCGGDYIDAWGQGTLVTVSSTS (SEQ ID NO 99) |

In the table above, the CDR sequences (L-CDR1, L-CDR2, L-CDR3, H-CDR1, H-CDR2, H-CDR3) included in the light chain and heavy chain variable regions are underlined, and the 10 non-humanized residues are indicated in bold.

### Example 2-5. Preparation of humanized antibodies (3)

To further humanize the 10 non-humanized residues in the Hu2G12 clone prepared in Example 2-4, among the remaining 10 chicken-derived residues, individual single mutations to the human germline sequence were introduced at S42Q and T46L in the light chain (V_{L}), and at G49S, S76N, and K94R in the heavy chain (V_{H}), thereby producing a total of 5 clones and a fully humanized V_{H} clone in which all 3 residues of the heavy chain (V_{H}) were substituted.

The clones were designated, respectively, as Hu2G12-S42Q (S42Q mutation in V_{L}), Hu2G12-T46L (T46L mutation in V_{L}), Hu2G12-G49S (G49S mutation in V_{H}), Hu2G12-S76N (S76N mutation in V_{H}), Hu2G12-K94R (K94R mutation in V_{H}), and Hu2G12-G49S/S76N/K94R (G49S, S76N, K94R mutations in V_{H}).

In an example, the CDR and variable region sequences of the Hu2G12-S42Q and Hu2G12-S76N clones are summarized in Tables 17 and 18 below:

**[Table 17]**

| [Hu2G12-S42Q clone] | |
|---|---|
| Region | Sequence |
| L-CDR1 | SGGGSNNYG (SEQ ID NO 65) |
| L-CDR2 | YNDKRPS (SEQ ID NO 66) |
| L-CDR3 | GSRDSSHDGI (SEQ ID NO 67) |
| H-CDR1 | GFTFSSYAMH (SEQ ID NO 69) |
| H-CDR2 | QISSNDGSWTGYGAAVKG (SEQ ID NO 70) |
| H-CDR3 | AAGGYNVCGYGCGGDYIDA (SEQ ID NO 71) |
| VL | LTQPSSVSVSPGQTARITCSGGGSNNYGWYQQKPGQAPVTVIYYNDKRPSGI PERFSGSTSGSTGTLTISGVQAEDEAVYFCGSRDSSHDGIFGGGTKLTVL (SEQ ID NO 100) |
| Linker | GQSSRSSGGGGSSGGGGS |
| VH | EVQLVESGGGLVQPGGSLRLSCSASGFTFSSYAMHWVRQAPGKGLEYVGQIS SNDGSWTGYGAAVKGRFTISRDNSKSTLYLQMNSLRAEDTAVYYCAKAAGGY |
| | NVCGYGCGGDYIDAWGQGTLVTVSSTS (SEQ ID NO 99) |

**[Table 18]**

| [Hu2G12-S76N clone] | |
|---|---|
| Region | Sequence |
| L-CDR1 | SGGGSNNYG (SEQ ID NO 65) |
| L-CDR2 | YNDKRPS (SEQ ID NO 66) |
| L-CDR3 | GSRDSSHDGI (SEQ ID NO 67) |
| H-CDR1 | GFTFSSYAMH (SEQ ID NO 69) |
| H-CDR2 | QISSNDGSWTGYGAAVKG (SEQ ID NO 70) |
| H-CDR3 | AAGGYNVCGYGCGGDYIDA (SEQ ID NO 71) |
| VL | LTQPSSVSVSPGQTARITCSGGGSNNYGWYQQKPGSAPVTVIYYNDKRPSG IPERFSGSTSGSTGTLTISGVQAEDEAVYFCGSRDSSHDGIFGGGTKLTVL (SEQ ID NO 98) |
| Linker | GQSSRSSGGGGSSGGGGS |
| VH | EVQLVESGGGLVQPGGSLRLSCSASGFTFSSYAMHWVRQAPGKGLEYVGQI SSNDGSWTGYGAAVKGRFTISRDNSKNTLYLQMNSLRAEDTAVYYCAKAAG GYNVCGYGCGGDYIDAWGQGTLVTVSSTS (SEQ ID NO 101) |

### Example 3. Anti-CD40L/anti-CD28 bispecific antibody

### Example 3-1. Anti-CD40L/anti-CD28 bispecific antibody (1)

An anti-CD40L/anti-CD28 bispecific antibody was prepared in which the respective anti-CD40L antibody and anti-CD28 antibody are combined in a monovalent antibody form.

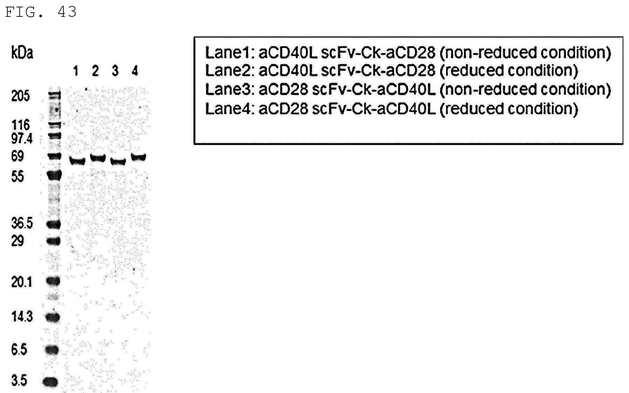

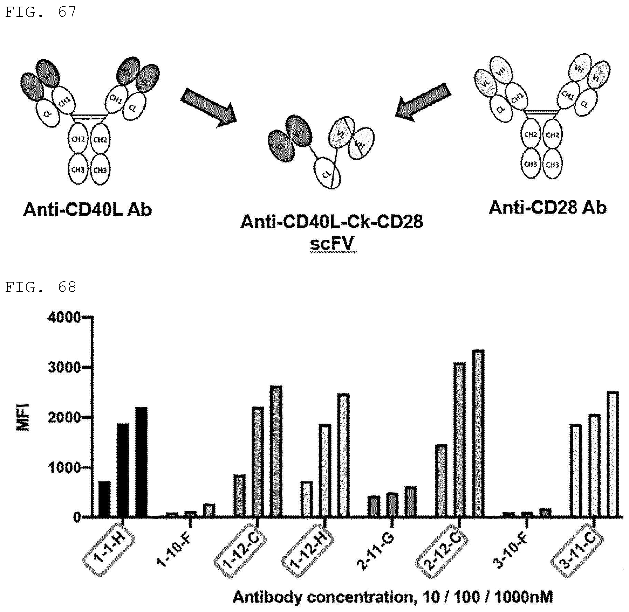

To prepare an exemplary anti-CD40L/anti-CD28 bispecific antibody in the above form, the humanized anti-CD40L scFv antibody, TSG clone, prepared in Example 1-4 and the humanized anti-CD28 scFv antibody, 2-G12 clone, prepared in Example 2-3 were cloned into a bispecific antibody format comprising a Ck linker domain, and then expressed in a mammalian expression system (see the schematic diagrams in FIGS. 42 and 67). Specifically, the antibody was overexpressed through transformation of human embryonic kidney 293F (HEK293F) cells, and then the recombinant antibody was successfully purified from the cell culture using affinity chromatography (FIG. 43).

FIG. 43 illustrates the SDS-PAGE results for the anti-CD40L/anti-CD28 bispecific antibody.

The complete sequences of the anti-CD40L/anti-CD28 bispecific antibodies are shown in Tables 19 and 20 below. The bispecific antibody in Table 19 comprises, from the N terminus, the anti-CD40L antibody (TSG clone), the Ck domain (SEQ ID NO: 95), and the anti-CD28 antibody (2-G12 clone) in that order. The bispecific antibody in Table 20 comprises, from the N terminus, the anti-CD28 antibody (2-G12 clone), the Ck domain (SEQ ID NO: 95), and the anti-CD40L antibody (TSG clone) in that order. The bispecific antibody in Table 19 was designated as TSGx2-G12, and the bispecific antibody in Table 20 was designated as 2-G12xTSG.

**[Table 19]**

| [N'-(TSG scFv) - linker-Ck-linker - (2-G12 scFv)-C'; TSGx2-G12] | | |
|---|---|---|
| Antibody | Region | Sequence |
| TSG | VL | ELTQDPAVSVALGQTVRITCSGGYSSYGIYYYGWYQQKPGQAPVTV IYNGNKRPSGIPDRFSGSSSGNTASLTITGAQAEDEADYYCGSGDS SSDSGIFGGGTKLTVL (SEQ ID NO 48) |
| | Linker | GQSSRSSGGGGSSGGGGS (SEQ ID NO 93) |
| | VH | EVQLVESGGGLVQPGGSLRLSCAASGFTFSSYGMNWVRQAPGKGLE WVGAISNDGSSTSYAPAVKGRFTISRDNSKNTLYLQMNSLRAEDTA VYYCAKESTNGGWVADDIDAWGQGTLVTVSS (SEQ ID NO 53) |
| Linker-Ck-Linker | Linker | GQAGQ *[Sfil* site] - GGGGSGGGGSGGGGS (SEQ ID NO 94) - KL [*HindIII* site] |
| | Ck | RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSLPVTKSFNRGES (SEQ ID NO 95) |
| | Linker | LE *[Xhol* site] - GGGGSGGGGSGGGGS (SEQ ID NO 94) - TG *[Agel* site] |
| 2-G12 | VL | LTQPSSVSANPGETVKITCSGGGSNNYGWYQQKPGSAPVTVIYYND KRPSGIPSRFSGSTSGSTGTLTITGVRAEDEAVYFCGSRDSSHDGI FGGGTKLTVL (SEQ ID NO 88) |
| | Linker | GQSSRSSGGGGSSGGGGS (SEQ ID NO 93) |
| | VH | PVKLVESGGGLVTPGGSLRLSCSASGFTFSSYAMHWVRQAPGKGLE |
| | | YVGQISSNDGSWTGYGAAVKGRFTISRDNSKSTLYLQMNSLRAEDT AVYYCAKAAGGYNVCGYGCGGDYIDAWGHGTQVTVSSTS (SEQ ID NO 90) |
| Full sequence [N' - (TSG scFv) - linker-Ck-linker - (2-G12 scFv) - C'] | ELTQDPAVSVALGQTVRITCSGGYSSYGIYYYGWYQQKPGQAPVTVIYNGNKR PSGIPDRFSGSSSGNTASLTITGAQAEDEADYYCGSGDSSSDSGIFGGGTKLT VLGQSSRSSGGGGSSGGGGSEVQLVESGGGLVQPGGSLRLSCAASGFTFSSYG MNWVRQAPGKGLEWVGAISNDGSSTSYAPAVKGRFTISRDNSKNTLYLQMNSL RAEDTAVYYCAKESTNGGWVADDIDAWGQGTLVTVSSGQAGQGGGGSGGGGSG GGGSKLRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSLPVTK SFNRGESLEGGGGSGGGGSGGGGSTGLTQPSSVSANPGETVKITCSGGGSNNY GWYQQKPGSAPVTVIYYNDKRPSGIPSRFSGSTSGSTGTLTITGVRAEDEAVY FCGSRDSSHDGIFGGGTKLTVLGQSSRSSGGGGSSGGGGSPVKLVESGGGLVT PGGSLRLSCSASGFTFSSYAMHWVRQAPGKGLEYVGQISSNDGSWTGYGAAVK GRFTISRDNSKSTLYLQMNSLRAEDTAVYYCAKAAGGYNVCGYGCGGDYIDAW GHGTQVTVSSTS (SEQ ID NO 96) | |

**[Table 20]**

| [N'-(2-G12 scFv) - linker-Ck-linker - (TSG scFv) -C'; 2-G12xTSG] | | |
|---|---|---|
| Antibody | Region | Sequence |
| 2-G12 | VL | LTQPSSVSANPGETVKITCSGGGSNNYGWYQQKPGSAPVTVIYYND KRPSGIPSRFSGSTSGSTGTLTITGVRAEDEAVYFCGSRDSSHDGI FGGGTKLTVL (SEQ ID NO 88) |
| | Linker | GQSSRSSGGGGSSGGGGS (SEQ ID NO 93) |
| | VH | PVKLVESGGGLVTPGGSLRLSCSASGFTFSSYAMHWVRQAPGKGLE YVGQISSNDGSWTGYGAAVKGRFTISRDNSKSTLYLQMNSLRAEDT AVYYCAKAAGGYNVCGYGCGGDYIDAWGHGT QVTVSSTS (SEQ ID NO 90) |
| Linker-Ck-Linker | Linker | GQAGQ *[Sfil* site] - GGGGSGGGGSGGGGS (SEQ ID NO 94) - KL [*HindIII* site] |
| | Ck | RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSLPVTKSFNRGES (SEQ ID NO 95) |
| | Linker | LE *[XhoI* site] - GGGGSGGGGSGGGGS (SEQ ID NO 94) - TG *[Agel* site] |
| TSG | VL | ELTQDPAVSVALGQTVRITCSGGYSSYGIYYGWYQQKPGQAPVTV IYNGNKRPSGIPDRFSGSSSGNTASLTITGAQAEDEADYYCGSGDS SSDSGIFGGGTKLTVL (SEQ ID NO 48) |
| | Linker | GQSSRSSGGGGSSGGGGS (SEQ ID NO 93) |
| | VH | EVQLVESGGGLVQPGGSLRLSCAASGFTFSSYGMNWVRQAPGKGLE WVGAISNDGSSTSYAPAVKGRFTISRDNSKNTLYLQMNSLRAEDTA VYYCAKESTNGGWVADDIDAWGQGTLVTVSS (SEQ ID NO 53) |
| Full sequence [N'-(2-G12 scFv) - linker-Ck- | LTQPSSVSANPGETVKITCSGGGSNNYGWYQQKPGSAPVTVIYYNDKRPSGIP SRFSGSTSGSTGTLTITGVRAEDEAVYFCGSRDSSHDGIFGGGTKLTVLGQSS RSSGGGGSSGGGGSPVKLVESGGGLVTPGGSLRLSCSASGFTFSSYAMHWVRQ APGKGLEYVGQISSNDGSWTGYGAAVKGRFTISRDNSKSTLYLQMNSLRAEDT AVYYCAKAAGGYNVCGYGCGGDYIDAWGHGTQVTVSSTSGQAGQGGGGSGGGG SGGGGSKLRTVAAPSVFIFPPSDEQLKSGTASVVCLLNNFYPREAKVQWKVDN ALQSGNSQESVTEQDSKDSTYSLSSTLTLSKADYEKHKVYACEVTHQGLSLPV | |
| linker - (TSG scFv) - C'] | TKSFNRGESLEGGGGSGGGGSGGGGSTGELTQDPAVSVALGQTVRITCSGGYS SYGIYYYGWYQQKPGQAPVTVIYNGNKRPSGIPDRFSGSSSGNTASLTITGAQ AEDEADYYCGSGDSSSDSGIFGGGTKLTVLGQSSRSSGGGGSSGGGGSEVQLV ESGGGLVQPGGSLRLSCAASGFTFSSYGMNWVRQAPGKGLEWVGAISNDGSST SYAPAVKGRFTISRDNSKNTLYLQMNSLRAEDTAVYYCAKESTNGGWVADDID AWGQGTLVTVSS (SEQ ID NO 97) | |

### Example 3-2. Anti-CD40L/anti-CD28 bispecific antibody (2)

### 3.2.1. Preparation of anti-CD40L/anti-CD28 bispecific antibody

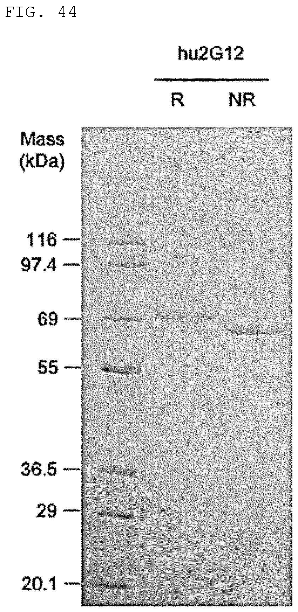

Using the same method as in Example 3-1, the humanized anti-CD40L scFv antibody, TSG clone, prepared in Example 1-4 and the additionally humanized anti-CD28 scFv antibody, Hu2G12 clone, prepared in Example 2-4 were cloned, in a bispecific antibody format comprising a Ck linker domain ([N'-(TSG scFv) - linker-Ck-linker - (Hu2G12 scFv)-C']; TSGxHu2G12), into the mammalian cell expression pCEP4 vector (Invitrogen, USA), and then transfected into Expi293F (Gibco, USA) cells. Subsequently, the recombinant antibody was purified from the culture using affinity chromatography, and SDS-PAGE was performed to identify the purified recombinant antibody (FIG. 44). The bispecific antibody prepared above was designated as TSGxHu2G12.

### 3.2.2. Identification of binding affinity for CD28

It was checked whether the additionally humanized anti-CD28 antibody portion of the bispecific antibody prepared in Example 3-2 preserved its binding affinity for CD28 and its CD28-CD80 (CD28 ligand) interaction inhibitory ability.

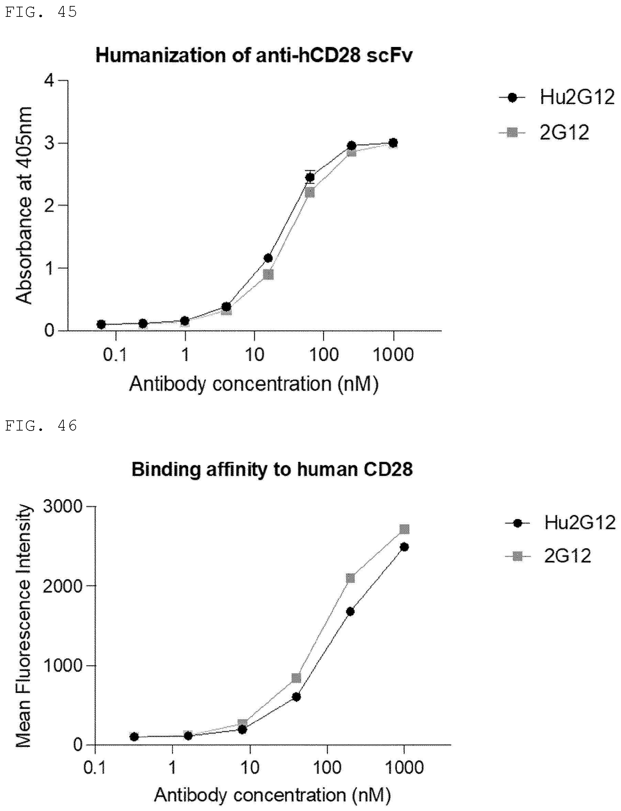

For this purpose, the anti-CD40L/anti-CD28 bispecific antibody (TSGx2-G12, Table 19) prepared in Example 3-1 and the anti-CD40L/anti-CD28 bispecific antibody (TSGxHu2G12) prepared in Example 3-2 were treated with recombinant human CD28 protein (R&D systems, USA), and an enzyme-linked immunosorbent assay (ELISA) was performed to check their binding affinity. Specifically, a 96-well plate (Corning, USA) was coated with the recombinant CD28 protein (in a form conjugated to human Fc region), and then blocking was performed using 3% BSA/PBS (w/v). Subsequently, the TSGx2-G12 and TSGxHu2G12 were incubated for 2 hours, followed by washing 3 times using 0.05% PBST(v/v). Then, treatment with anti-hCk-HRP (Millipore, USA) was performed for 1 hour. After the reaction was completed, washing was additionally performed 3 times. Color development was carried out using ABTS (Thermo Scientific, USA), and absorbance was measured at 405 nm using a microplate reader (Thermo Scientific, USA). The results are shown in FIG. 45.

As can be seen in FIG. 45, TSGxHu2G12 exhibited CD28 binding affinity equivalent to TSGx2-G12.

### 3.2.3. Identification of binding affinity for cell surface CD28

The TSGxHu2G12 clone was reacted with the CD28-positive Jurkat E6.1 cell line, and checked through flow cytometry for its binding ability to CD28 expressed on the cell surface. Specifically, the 2-G12 and Hu2G12 clones were reacted with the Jurkat E6.1 cell line for 1 hour, followed by washing 3 times using 1% BSA/PBS (w/v) with 0.02% NaN₃ (v/v). Then, treatment with anti-hCk-APC (AbCam, UK) was performed and the reaction was allowed to proceed for 1 hour. After the reaction was completed, washing was additionally performed 3 times, and fluorescence was detected using a flow cytometer (Becton Dickinson, USA). The results are shown in FIG. 46.

As shown in FIG. 46, it was identified that the bispecific antibody (TSGxHu2G12) comprising the Hu2G12 clone exhibited binding affinity to an extent similar to the bispecific antibody (TSGx2-G12) comprising the 2-G12 clone (FIG. 46).

### 3.2.4. Identification of CD28-CD80 interaction inhibitory ability

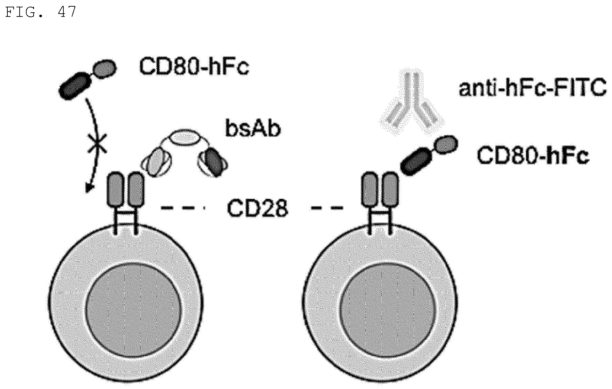

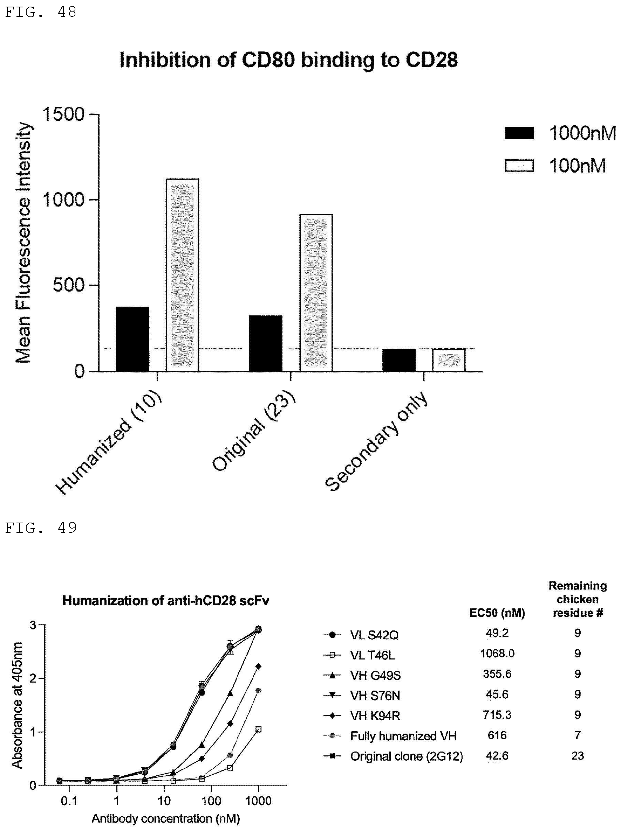

To evaluate the CD28-CD80 interaction inhibitory ability of the TSGxHu2G12 clone, 2G-12 and Hu2G12 clones (100 nM or 1000 nM) were allowed to bind to the CD28-positive Jurkat E6.1 cell line for 1 hour, followed by washing 3 times using 1% BSA/PBS (w/v) with 0.02% NaN₃ (v/v). Then, incubation with 1 mg of recombinant CD80 protein (Sino Biological, China) was performed for 1 hour. After the reaction, washing was performed 3 times. Subsequently, incubation with anti-hFc-FITC (Invitrogen, USA), which is capable of detecting the recombinant CD80 protein, was performed, followed by washing 3 times. Fluorescence was then measured using a flow cytometer (Becton Dickinson, USA) to check whether the TSGxHu2G12 clone could inhibit binding of the recombinant CD80 protein to CD28. The results are shown in FIG. 48. FIG. 47 illustrates a schematic diagram of the above experiment.

As a result, it was identified that the bispecific antibody (TSGxHu2G12) comprising the Hu2G12 clone inhibited binding of the recombinant CD80 protein at a 1000 nM antibody concentration to an extent similar to the bispecific antibody (TSGx2-G12) comprising the 2-G12 clone (FIG. 48).

### Example 3-3. Anti-CD40L/anti-CD28 bispecific antibody (3)

### 3.3.1. Preparation of anti-CD40L/anti-CD28 bispecific antibody

Using the same method as in Example 3-1, the humanized anti-CD40L scFv antibody, TSG clone, prepared in Example 1-4 and the additionally humanized anti-CD28 scFv antibody, Hu2G12-S42Q, Hu2G12-T46L, Hu2G12-G49S, Hu2G12-S76N, Hu2G12-K94R, or Hu2G12-G49S/S76N/K94R clone, prepared in Example 2-5 were cloned, in a bispecific antibody format comprising a Ck linker domain, into the mammalian cell expression pCEP4 vector (Invitrogen, USA), and then transfected into Expi293F (Gibco, USA) cells. Subsequently, the recombinant antibody was purified from the culture using affinity chromatography, and SDS-PAGE was performed to identify the purified recombinant antibody. The bispecific antibodies prepared were designated, respectively, as TSGxHu2G12-S42Q, TSGxHu2G12-T46L, TSGxHu2G12-G49S, TSGxHu2G12-S76N, TSGxHu2G12-K94R, and TSGxHu2G12-G49S/S76N/K94R.

### 3.3.2. Identification of binding affinity for CD28

It was checked whether the additionally humanized anti-CD28 antibody portions of the bispecific antibody prepared in Example 3-3 preserved their binding affinity for CD28 and their CD28-CD80 (CD28 ligand) interaction inhibitory ability.

For this purpose, the anti-CD40L/anti-CD28 bispecific antibody (TSGx2-G12, Table 16) prepared in Example 3-1 and the anti-CD40L/anti-CD28 bispecific antibodies (TSGxHu2G12-S42Q, TSGxHu2G12-T46L, TSGxHu2G12-G49S, TSGxHu2G12-S76N, TSGxHu2G12-K94R, TSGxHu2G12-G49S/S76N/K94R) prepared in Example 3-3 were treated with recombinant human CD28 protein (R&D Systems, USA), and an enzyme-linked immunosorbent assay (ELISA) was performed using the same method as in Example 3.2.2 to check their binding affinity. The results are shown in FIG. 49.

As can be seen in FIG. 49, among the six anti-CD40L/anti-CD28 bispecific antibodies prepared by performing the additional humanization process in Example 3-3, the clone (TSGxHu2G12-S42Q) in which the S42Q mutation was introduced into the light chain (V_{L}) and the clone (TSGxHu2G12-S76N) in which the S76N mutation was introduced into the heavy chain (V_{H}) exhibited binding affinity to CD28 equivalent to the anti-CD40L/anti-CD28 bispecific antibody (TSGx2-G12, Table 16) prepared in Example 3-1.

### 3.3.3. Identification of binding affinity for cell surface CD28

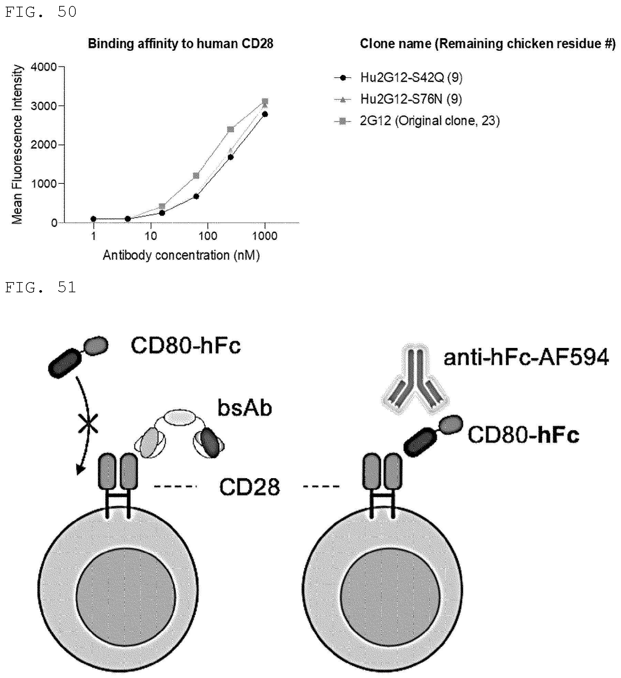

The TSGxHu2G12-S42Q and TSGxHu2G12-S76N clones, which exhibited CD28 binding affinity equivalent to the TSGx2-G12 clone in Example 3.3.2, were reacted with THE CD28-positive Jurkat E6.1 cell line, and checked through flow cytometry for their binding ability to CD28 expressed on the cell surface. Specifically, the TSGx2-G12, TSGxHu2G12-S42Q, and TSGxHu2G12-S76N clones were reacted with the Jurkat E6.1 cell line for 1 hour, followed by washing 3 times using 1% BSA/PBS (w/v) with 0.02% NaN₃ (v/v). Then, treatment with anti-hCk-APC (AbCam, UK) was performed and the reaction was allowed to proceed for 1 hour. After the reaction was completed, washing was additionally performed 3 times, and fluorescence was detected using a flow cytometer (Becton Dickinson, USA). The results are shown in FIG. 50.

As can be seen in FIG. 50, the TSGxHu2G12-S42Q and TSGxHu2G12-S76N clones exhibited slightly decreased binding affinity to the cell surface CD28 compared to the TSGx2-G12 clone, but still showed binding affinity equivalent thereto.

### 3.3.4. Identification of CD28-CD80 interaction inhibitory ability

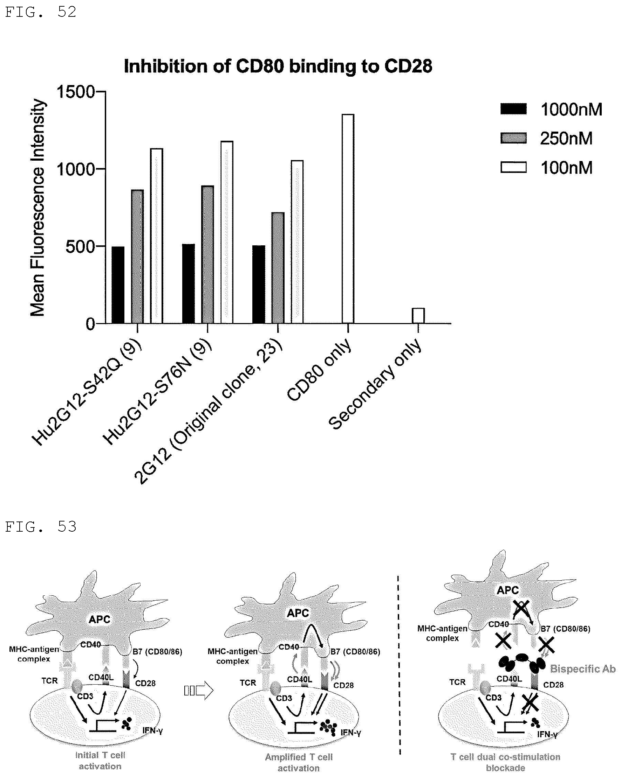

To evaluate the CD28-CD80 interaction inhibitory ability of the anti-CD40L/anti-CD28 bispecific antibody, the TSGx2-G12, TSGxHu2G12-S42Q, or TSGxHu2G12-S76N clone (at 100 nM, 250 nM, or 1000 nM) was allowed to bind to the CD28-positive Jurkat E6.1 cell line for 1 hour, followed by washing 3 times using 1% BSA/PBS (w/v) with 0.02% NaN₃ (v/v). Then, incubation with 1 mg of recombinant CD80 protein (Sino Biological, China) was performed for 1 hour. After the reaction, washing was performed 3 times. Subsequently, incubation with anti-hFc-Alexa Fluor 594 (Jackson Laboratory, USA), which is capable of detecting the recombinant CD80 protein, was performed, followed by washing 3 times. Fluorescence was then measured using a flow cytometer (Becton Dickinson, USA), and the results are shown in FIG. 52. FIG. 51 illustrates a schematic diagram of the above experiment.

As can be seen in FIG. 52, the TSGxHu2G12-S42Q and TSGxHu2G12-S76N clones exhibited an ability to inhibit binding of recombinant CD80 protein to an extent equivalent to the TSGx2-G12 clone.

### Example 3-4. In vitro activity (T cell inhibitory ability) of anti-CD40L/anti-CD28 bispecific antibody

For T cells to be activated, the T cell receptor (TCR) on the T cell surface must recognize an antigen presented on the surface of an antigen-presenting cell (APC). Here, it is known that T cells are activated only when they receive a signal (signal 1) transmitted through the TCR along with a costimulatory signal (signal 2) transmitted by binding of B7 molecules (B7.1 [CD80], B7.2 [CD86]) on the APC surface to CD28 on the T cell surface.

The activated T cells induce CD40L expression. When CD40L on the CD4 T cells binds to CD40 on the APC surface, CD40 transmits an activation signal to the antigen-presenting cell. As a result, the expression of MHC molecules and costimulatory molecules (such as CD80, CD86, and 41BBL) on the APC surface increases, and the expression of T cell-stimulating cytokines such as IL-12 also increases. A positive feedback mechanism is known in which such activated APCs in turn increase T cell activity (see the schematic diagram in FIG. 53).

Accordingly, it was evaluated whether the anti-CD40L/anti-CD28 bispecific antibody could simultaneously bind to CD40L and CD28 on CD4 T cells to block CD40L-CD40 and CD28-B7 binding, thereby inhibiting activation of CD4 T cells.

CD4-positive T cells and monocytes were isolated from the leukocytes obtained by leukapheresis from healthy adults using the MACS cell separation method (CD14 microbead, 130-050-201, Miltenyi Biotech). The monocytes were differentiated into dendritic cells by being treated with GM-CSF (20 ng/ml, JW Creagene, rhGM-CSF) and IL-4 (20 ng/ml, JW Creagene, rhIL-4) for 5 days.

CD4-positive T cells were isolated from the leukocyte fraction of the same donor using the MACS cell separation method (CD4 microbead, 130-097-048, Miltenyi Biotech), added to the differentiated dendritic cells, and co-cultured with an anti-CD3 antibody (clone OKT3, 1 µg/ml, 50-561-956, Bio X cell) added for TCR stimulation. To maintain sufficient distance between T cells in the culture space, B cells isolated from the leukocyte fraction of the same donor using the MACS cell separation method (CD19 microbead, 130-050-301, Miltenyi Biotech) were irradiated to prevent proliferation and then added as bystander cells to the culture. The T cell inhibitory efficacy of the bispecific antibody was evaluated by adding the bispecific antibody under the co-culture conditions.

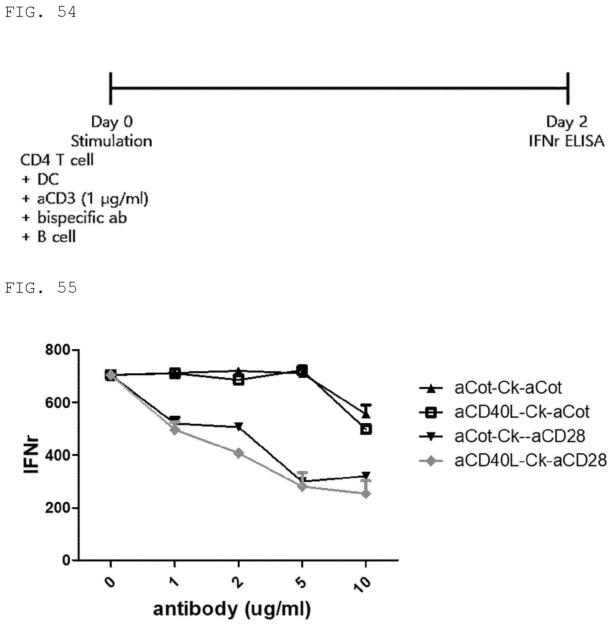

FIG 54 illustrates a schematic diagram of the above experimental process.

The experimental results are shown in FIG. 55. Here, the CD28 single-inhibitory antibodies (aCot-Ck-aCD28; aCot-Ck-2-G12) tested concurrently also exhibited an IFN-γ inhibitory effect to an extent similar to the bispecific antibody (TSGx2-G12); however, no IFN-γ inhibitory effect was observed for the CD40L single-inhibitory antibodies (aCD40L-Ck-aCot; TSG-Ck-aCot). Therefore, the T cell activation inhibitory potency of the bispecific antibody under these experimental conditions is considered to be primarily due to its inhibitory effect on CD28. This is interpreted as indicating that, because CD40L is expressed only in activated T cells, no CD40L inhibitory effect was observed under the 48-hour culture conditions for observing the initial activation of T cells.

### Example 3-5. In vitro activity (induction of T cell immune tolerance) of anti-CD40L/anti-CD28 bispecific antibody

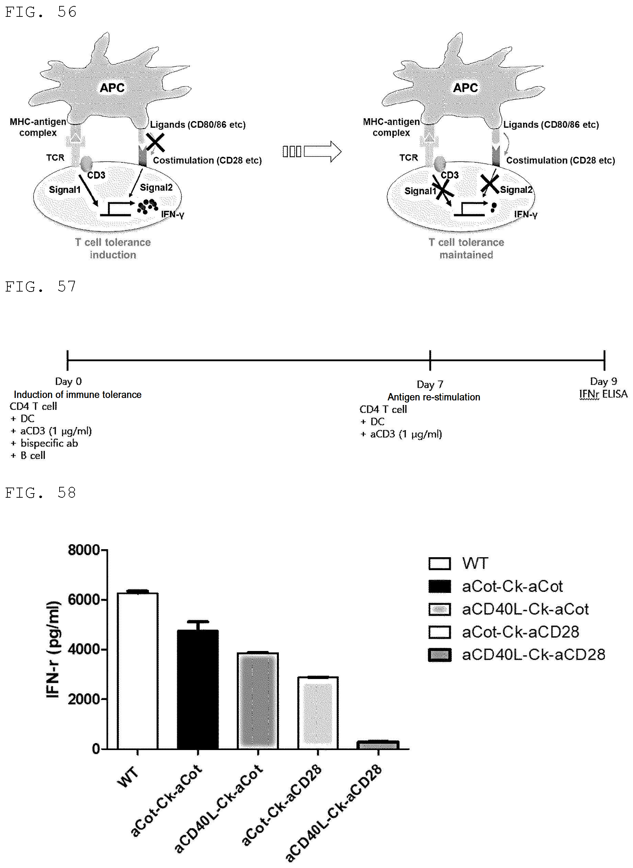

Immunological tolerance refers to a mechanism by which the human immune system prevents autoimmune diseases, which is the phenomenon in which, when an autoantigen-specific T cell recognizes an autoantigen presented by an antigen-presenting cell and receives a TCR signal (signal 1), if the T cell does not receive a co-stimulatory signal (signal 2) from the antigen-presenting cell, it remains inactivated even upon subsequent re-stimulation with the autoantigen (see the schematic diagram in FIG. 56).

In this example, it was identified that the anti-CD40L antibody (TSG clone), the anti-CD28 antibody (2-G12 clone), and the anti-CD40L/anti-CD28 bispecific antibody (TSGx2-G12 clone) promoted immune tolerance which is a state where the respective antibodies simultaneously blocked CD40L and CD28 co-stimulation during the activation stage of T cells, and subsequently these T cells failed to activate even if they receive activation stimulation again.

Similar to the experiment for the initial T cell activation stage, human CD4 T cells, dendritic cells, and irradiated B cells were co-cultured with an anti-CD3 antibody to induce CD4 T cell activation. At the same time, the bispecific antibody was simultaneously added thereto to block co-stimulation (induction of immune tolerance). After 7 days, these CD4 T cells were re-stimulated with new dendritic cells and anti-CD3 antibody. Then, it was evaluated whether the T cells show unresponsiveness. T cell unresponsiveness was evaluated by measuring, with an ELISA assay, IFN-γ secretion in the culture supernatant at 48 hours after antigen re-stimulation.

FIG. 57 illustrates a schematic diagram of the above experimental procedure.

The experimental results are shown in FIG. 58.

For the CD40L single antibody or the CD28 single antibody, the IFN-γ secretion level was partially decreased compared to the negative control antibody, indicating that these single antibodies also have an immune tolerance-inducing effect. In the group treated with the anti-CD40L/anti-CD28 bispecific antibody, the amount of IFN-γ was significantly decreased compared to the negative control, indicating that the CD4 T cells treated with the bispecific antibody did not respond even upon antigen re-stimulation (FIG. 58). Therefore, it was identified that the bispecific antibody possesses a significant immune tolerance-inducing effect.

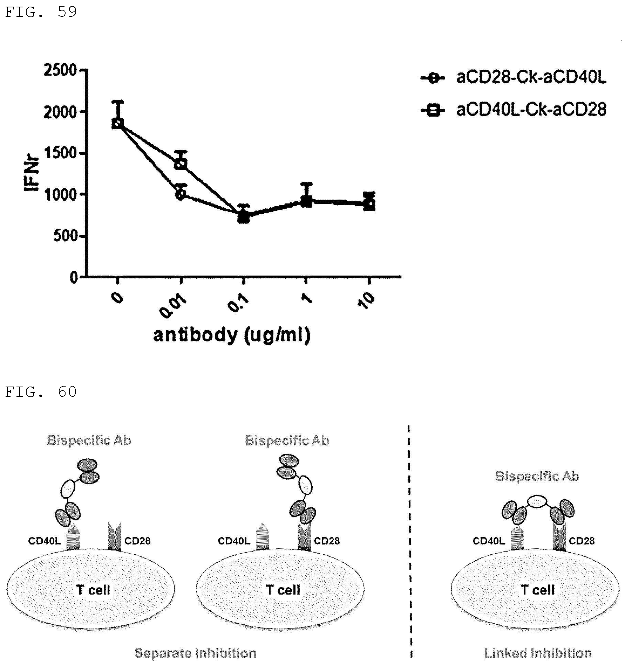

Additionally, various formats of the anti-CD40L/anti-CD28 bispecific antibody (anti-CD40L-Ck-CD28 bispecific antibody and anti-CD28-Ck-CD40L bispecific antibody) were compared in terms of the efficacy. Two formats of the anti-CD40L/anti-CD28 bispecific antibody were prepared (anti-CD40L antibody - anti-CD28 antibody and anti-CD28 antibody - anti-CD40L antibody). Then, the same treatment as described above was performed using these bispecific antibodies. After 48 hours, their IFN-γ production inhibitory ability was measured by ELISA. As a result, it was identified that both bispecific antibodies effectively inhibited T cell activation (FIG. 59), indicating that they exhibited an excellent T cell inhibitory ability regardless of the order of the anti-CD40L antibody and the anti-CD28 antibody.

### Examples 3-6. Identification of cis-interaction of anti-CD40L/anti-CD28 bispecific antibody

The anti-CD40L/anti-CD28 bispecific antibody provided in this application, as a single antibody molecule, can not only independently bind to CD40L and CD28 molecules expressed on T cells, thereby inhibiting the function of each molecule, but can also simultaneously bind to CD40L and CD28 molecules expressed on a single T cell (cis-interaction) to inhibit both molecules at the same time. This linked inhibition can demonstrate a superior effect compared to separate inhibition. FIG. 60 illustrates a schematic diagram of the simultaneous inhibition and the separate inhibition.

In this example, a Fφrster resonance energy transfer (FRET) assay was performed to check whether the anti-CD40L/anti-CD28 bispecific antibody functions in a linked inhibition mode through cis-interaction on T cells.

The fluorescent protein-tagged CD28 recombinant receptor [N'-Extracellular domain-Transmembrane domain-Cytoplasmic domain-linker-mNeongreen-C' format] (Addgene, [#113400] pEF6a-CD28-PafA) and CD40L recombinant receptor [N'-mScarlet-linker-Cytoplasmic domain-Transmembrane domain-Extracellular domain-C' format] (Addgene, [#52337] CD154-His-bio) were cloned into the pRK5 expression vector. Subsequently, 0.5 mg and 1 mg of the DNA, respectively, were co-transfected into human embryonic kidney 293A (HEK293A) cells, which had been cultured for 24 hours, using Lipofectamine 2000 (Invitrogen, USA). After 24 hours, the cells were treated with PMA (phorbol myristate acetate) and cultured for additional 12 hours to induce cell activation. The activated HEK293A cells were then treated with the anti-CD40L-Ck-CD28 bispecific antibody in an scFv-Ck-scFv format, the anti-CD40L antibody (anti-CD40L scFv-Ck-cotinine scFv) or the anti-CD28 antibody (anti-cotinine scFv-Ck-CD28 scFv) or a mixture thereof (anti-CD40L antibody + anti-CD28 antibody), and two control antibodies in the same format, each at 75 nM, and observed for 10 minutes.

Subsequently, by using the mCherry filter (Excitation 562/40, dichroic mirror 593, Emission 641/75) and adjusting the neutral density (ND) filter that can control light intensity, the fluorescence of CD40L-mScarlet was photobleached by being irradiated for 1 minute with an ND4 filter. After photobleaching, the mCherry fluorescence change was approximately -80% on average. The Fφrster resonance energy transfer (FRET) level [(FITC intensity after photobleaching - FITC intensity before photobleaching)/FITC intensity after photobleaching] was calculated by measuring the mNeonGreen fluorescence change level (FITC intensity) in the cell membrane region.

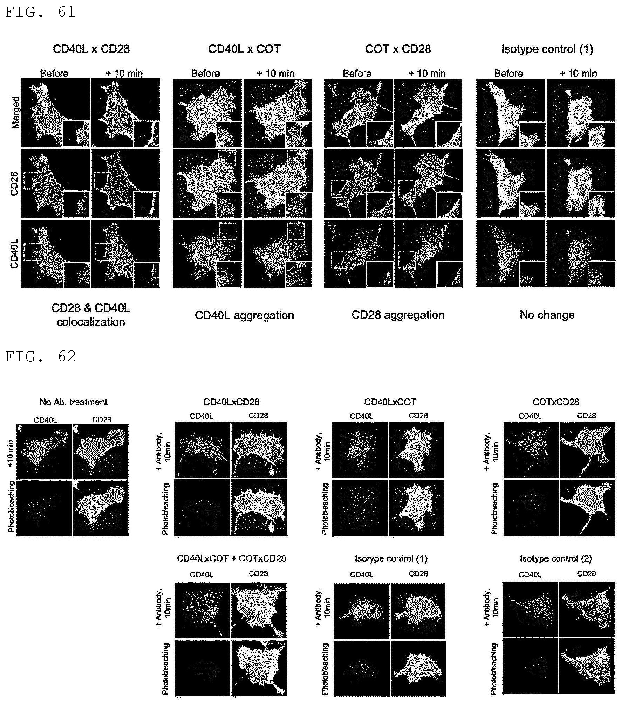

The experimental results are shown in FIGS. 61 and 62.

As can be seen from the experimental results, it was identified that CD40L and CD28 were co-localized in the same location only in the group treated with the anti-CD40L-Ck-CD28 bispecific antibody.

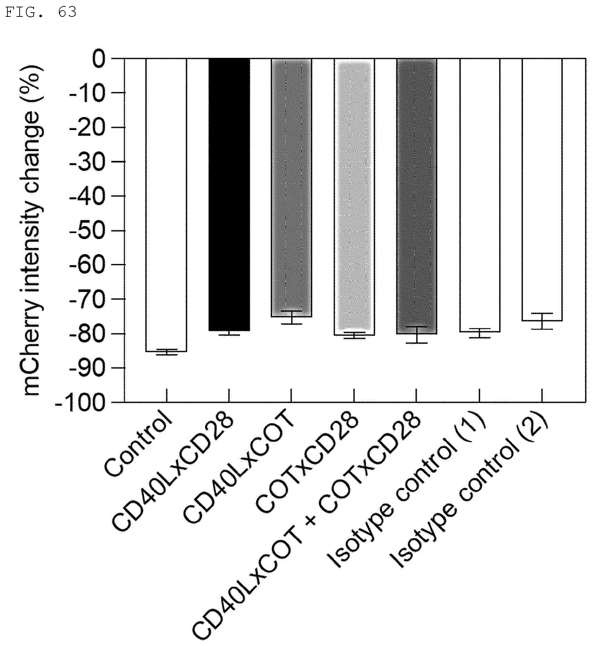

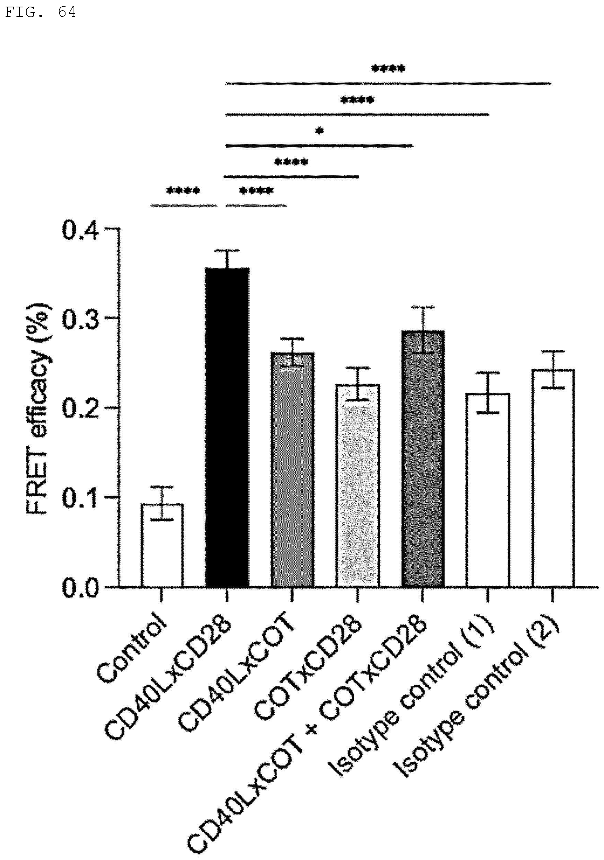

Furthermore, the results obtained by measuring the mNeonGreen fluorescence change level in the cell membrane region after photobleaching and calculating the FRET efficacy are shown in FIGS. 63 and 64. It was identified that only in the group treated with the anti-CD40L-Ck-CD28 bispecific antibody, the CD28-mNeonGreen fluorescence in the cell membrane region significantly changed and thus the FRET efficacy value was statistically significant compared to the other groups. This means that the anti-CD40L-Ck-CD28 bispecific antibody expressed on the cell surface binds to CD40L and CD28 expressed on the same cell surface via cis-interaction.

Therefore, the anti-CD40L-Ck-CD28 bispecific antibody suggested the possibility of acting in a linked inhibition mode through cis-interaction, in addition to separate inhibition of CD40L and CD28.

### Example 3-7. In vivo activity of anti-CD40L/anti-CD28 bispecific antibody

Although in vivo autoimmune disease models mediated by human T cells are difficult to implement in experimental animals such as mice, as a mouse model similar to an autoimmune disease model, a xenogeneic graft-versus-host disease (GVHD) mouse model exists in which an inflammatory immune disease is induced by human T cells. This mouse model induces T cell-mediated inflammatory diseases when human T cells are injected into immunodeficient mice, as the human T cells recognize and attack the mouse's xeno-antigens. This model shares a similar mechanism with an autoimmune disease in that inflammation is induced by T cell hyperactivation. Therefore, the model allows to assess the degree of human T cell hyperactivation by evaluating severity of an inflammatory disease induced in mice, and can also simulate the inhibitory effect on autoimmune diseases by observing the T cell inhibitor-mediated inhibition of human T cell activity and the resulting alleviation of an inflammatory disease. Thus, it can be used as an appropriate model for evaluating the in vivo function of human anti-CD40L antibodies and human CD28 antibodies.

Furthermore, this model can also be used as a model for simulating allogeneic GVHD that can occur in hematologic cancer patients due to transplanted donor T-cells after hematopoietic stem cell transplantation (HSCT). Therefore, in addition to autoimmune diseases, it can be used as a model for evaluating efficacy of an antibody as a therapeutic agent for GVHD caused by T cell hyperactivation.

Therefore, this example was intended to establish a xenogeneic GVHD mouse model using human T cells and verify the inflammatory disease inhibitory effect of the anti-CD40L antibody and the anti-CD28 antibody. NSG mice (immunodeficient mice) (NSG mice (NOD.Cg-Prkdcscid I12rgtm1Wjl/SzJ), Jackson Laboratory, #005557) were injected intravenously with peripheral blood mononuclear cells (PBMCs, 1x10⁷ cells) isolated from the peripheral blood of healthy adults. Subsequently, disease progression was monitored by scoring disease severity based on body weight loss and clinical symptoms in the mice.

The experimental groups were divided as follows: a group without antibody treatment (PBMC), a group (PBMC+aCD40L) treated with anti-CD40L antibody (TSG clone) alone, a group (PBMC+aCD28) treated with anti-CD28 antibody (2-G12 clone) alone, a group treated with combination of anti-CD40L antibody and anti-CD28 antibody (PBMC+aCD40L+aCD28), and groups (PBMC+aCD40L-aCD28, PBMC+aCD28-aCD40L) treated with anti-CD40L/anti-CD28 bispecific antibody. The mice were intraperitoneally injected with the antibody three times a week at a dose of 400 µg per injection over a total of nine times.

The body weight data were recorded until the mice died.

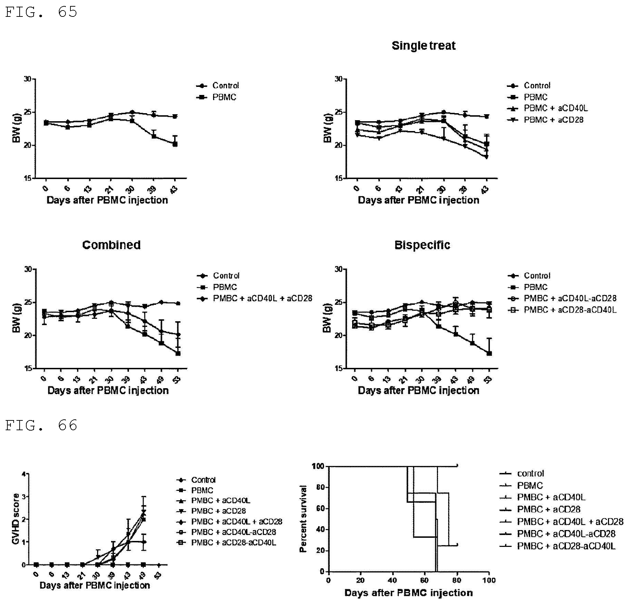

The experimental results are shown in FIGS. 65 and 66.

It was identified that the group (negative control, PBMC) without antibody treatment showed body weight loss after 30 days, indicating disease onset.

The groups treated with either anti-CD40L antibody or anti-CD28 antibody alone showed weight loss to an extent similar to the negative control and showed disease progression similar to the negative control in disease severity assessment. All mice died within a period similar to the negative control. This indicates that disease progression could not be suppressed by inhibition of a single co-stimulatory molecule alone under these GVHD model conditions. However, in view of the T cell and antigen-presenting cell activation inhibitory ability of the two single antibodies observed in vitro, the results do not imply a complete lack of efficacy in vivo.

The groups treated with combined anti-CD40L and anti-CD28 antibodies showed delayed weight loss compared to the control, and showed delayed disease progression compared to the control in disease severity assessment. Furthermore, it was identified that the survival period of the mice increased compared to the groups treated with each antibody alone, indicating that combined therapy with the two antibodies can more effectively suppress disease progression. These results demonstrate partial efficacy of the single antibodies and prove that the combined therapy with the two antibodies can induce a synergistic effect. However, under the current antibody dosing regimen, disease progression could not be completely suppressed. Thus, the mice experienced weight loss and eventually died.

In contrast, it was identified that the groups treated with the anti-CD40L/anti-CD28 bispecific antibody showed no weight loss during the experimental period, and did not exhibit disease onset in disease severity assessment. Furthermore, all mice survived throughout the experimental period, indicating that disease progression was completely suppressed only in the groups treated with the bispecific antibody. These results explain superiority of the bispecific antibody format, where two antibody portions are simultaneously present within a single molecule, compared to combined treatment of the two single antibodies. The results also demonstrate conceptual originality of the linked inhibition for both CD40L and CD28. However, these results do not negate partial efficacy of each single antibody or individual efficacy when each single antibody is prepared in a format other than the scFv form.

### Example 4. Anti-CD40L/anti-CD28 bispecific antibody for mice

### Example 4-1. Preparation of anti-CD40L/anti-CD28 bispecific antibody for mice

As previously explained, autoimmune disease animal models utilizing hyperactivation of human T cells are theoretically difficult to implement in experimental animals. In this example, to further support the anti-CD40L/anti-CD28 bispecific antibody provided by the present disclosure and its therapeutic effect on autoimmune diseases, a surrogate antibody against mouse CD28 and mouse CD40L (anti-CD40L/anti-CD28 bispecific antibody) was administered to an autoimmune disease mouse model to check its therapeutic effect on autoimmune diseases.

The present disclosure newly proposes an anti-CD40L/anti-CD28 bispecific antibody in which the respective anti-CD40L antibody and anti-CD28 antibody are combined in a monovalent antibody form, and a use thereof for treating autoimmune diseases. Therefore, as shown below, the preparation example of the anti-CD40L/anti-CD28 bispecific antibody for mice, in which the respective antibodies are combined in a monovalent antibody form, and its excellent therapeutic effect on autoimmune diseases should be construed as supporting the anti-CD40L/anti-CD28 bispecific antibody provided by the present disclosure and its therapeutic effect on autoimmune diseases.

### 4.1.1. Anti-mouse CD28 antibody

The anti-mouse CD28 antibody utilized the scFv sequence of the PV-1 clone (Plos One 2017; 12(3):e0171822, Novel CD28 antagonist mPEG PV1-Fab' mitigates experimental autoimmune uveitis by suppressing CD4+ T lymphocyte activation and IFN-γ production). The CDR and variable region sequences of the PV-1 clone (scFv) are shown in Table 21 below.

**[Table 21]**

| [PV1 (anti-mCD28) ] | |
|---|---|
| Region | Sequence |
| LCDR1 | RSSQSLYYSGIKKNLLA (SEQ ID NO 102) |
| LCDR2 | FTSTRLP (SEQ ID NO 103) |
| LCDR3 | QQGISTPLT (SEQ ID NO 104) |
| HCDR1 | DGYMN (SEQ ID NO 105) |
| HCDR2 | RIDPDSGNTRYNQKFQG (SEQ ID NO 106) |
| HCDR3 | DGTFYGTYGYWYFDF (SEQ ID NO 107) |
| VL | DIVMTQSPYSLAVSAGEKVTMSCRSSQSLYYSGIKKNLLAWYQQKPGQSPKL LIYFTSTRLPGVPDRFTGSGSGTDYTLTITSVQAEDMGHYFCQQGISTPLTF GDGTKLEIR (SEQ ID NO 108) |
| Linker | GQSSRSSGGGGSSGGGGS (SEQ ID NO 93) |
| VH | QVQLKQSGAELVKPGASVKISCKTSGYTFTDGYMNWVEQKPGQGLEWIGRID |
| | PDSGNTRYNQKFQGKATLTRDKSSSTVYMDLRSLTSEDSAVYYCARDGTFYG TYGYWYFDFWGQGTQVTVSS (SEQ ID NO 109) |

### 4.1.2. Anti-mouse CD40L antibody

The anti-mouse CD40L antibody was newly prepared from chickens immunized with mouse CD40L protein and used.

Chickens were immunized 4 times with mouse CD40L recombinant protein (Sino Biological, China), and an antibody library in an scFv form was constructed using a previously reported method. The constructed three chicken antibody libraries had complexity of 5.0x10⁹, 5.8x10⁹, and 6.3x10⁹, respectively. Bio-panning against mouse CD40L (R&D Systems, USA) was performed using the chicken antibody libraries, and 16 clones that bind to mouse CD40L were selected by an enzyme-linked immunosorbent assay, with their genetic information being obtained through sequencing.

Among the 16 antibody clones that bind to mouse CD40L recombinant protein, to produce 8 clones with different HCDR3 sequences in an scFv-hFc fusion protein format [anti-mouse CD40L scFv (N'-VL-linker-VH-C')-human Fc], cloning was performed into the mammalian cell expression pCEP4 vector (Invitrogen, USA). After transfection into Expi293F (Gibco, USA) cells, the antibodies were purified from the cell culture by performing affinity chromatography. SDS-PAGE was performed to identify the purified recombinant antibodies.

It was identified through flow cytometry that the expressed 8 anti-mouse CD40L antibodies bound to the L (mCD154+) cell line (L4.5 cell line, DSMZ, Cat No. ACC 772) expressing mouse CD40L. Specifically, the anti-mouse CD40L antibody was bound to the L (mCD154+) cell line at concentrations of 10, 100, and 1000 nM. The cells were then washed 3 times using 1% BSA/PBS (w/v) with 0.02% NaN₃ (v/v). Subsequently, anti-hFc-FITC (Invitrogen, USA), which had been diluted 1:100, was bound thereto, followed by washing 3 times. Fluorescence was detected using a flow cytometer (BD Bioscience, USA). The results were presented by comparing mean fluorescence intensity (MFI) values. It was identified that the 1-1-H, 1-12C, 1-12-H, 2-12-C, and 3-11-C clones effectively bound to mouse CD40L expressed on the cell surface in a concentration-dependent manner (FIG. 68).

From the above results, the 2-12-C clone, which exhibited the best binding affinity for mouse CD40L, was selected. The CDR and variable region sequences of the 2-12-C clone are shown in Table 22 below.

**[Table 22]**

| [2-12-C (anti-mCD40L) ] | |
|---|---|
| Region | Sequence |
| LCDR1 | SGGYSDYYG (SEQ ID NO 110) |
| LCDR2 | YNDKRPS (SEQ ID NO 111) |
| LCDR3 | GGYDGSTDAGI (SEQ ID NO 112) |
| HCDR1 | GFTFNNNGMA (SEQ ID NO 113) |
| HCDR2 | GISSGSYTYYGTAVKG (SEQ ID NO 114) |
| HCDR3 | DGSDYNWNSGADSIDA (SEQ ID NO 115) |
| VL | LTQPSSVSANPGGTVEITCSGGYSDYYGWYQQKAPGSAPVTVIYYNDKRPSN IPSRFSGSTSGSTGTLTITGVQAEDEAIYYCGGYDGSTDAGIFGAGTTLTVL (SEQ ID NO 116) |
| Linker | GQSSRSSSGGGSSGGGGS (SEQ ID NO 118) |
| VH | AVTLDESEGGLQTPGGALSLVCKASGFTFNNNGMAWVRQAPGKGLEFVAGIS SGSYTYYGTAVKGRATISRDNGQSTVRLQLNNLRAEDTATYFCAKDGSDYNW NSGADSIDAWGHGTEVIVSS (SEQ ID NO 117) |

### 4.1.3. Anti-CD40L/anti-CD28 bispecific antibody for mice

The anti-mouse CD28 scFv antibody, PV1 clone, and the newly developed anti-mouse CD40L scFv antibody, 2-12-C clone, were cloned, in a bispecific antibody format comprising a human Ck linker domain [anti-mouse CD28 scFv antibody (N'-VL-VH-C')-Ck-anti-mouse CD40L scFv antibody (N'-VL-VH-C')], into the mammalian cell expression pCEP4 vector (Invitrogen, USA). After transfection into Expi293F cells (Gibco, USA), the recombinant antibody was purified from the culture using affinity chromatography, and SDS-PAGE was performed to identify the purified recombinant antibody.

The prepared anti-CD40L/anti-CD28 bispecific antibody for mice was designated as PV1x2-12-C clone, and the respective sequences are shown in Table 23 below.

**[Table 23]**

| [PV1(anti-mCD28) x 2-12-C(anti-mCD40L)] | | |
|---|---|---|
| Antibody | Region | Sequence |
| PV1 | VL | DIVMTQSPYSLAVSAGEKVTMSCRSSQSLYYSGIKKNLLAWYQ QKPGQSPKLLIYFTSTRLPGVPDRFTGSGSGTDYTLTITSVQA EDMGHYFCQQGISTPLTFGDGTKLEIR (SEQ ID NO 108) |
| | Linker | GQSSRSSGGGGSSGGGGS (SEQ ID NO 93) |
| | VH | QVQLKQSGAELVKPGASVKISCKTSGYTFTDGYMNWVEQKPGQ GLEWIGRIDPDSGNTRYNQKFQGKATLTRDKSSSTVYMDLRSL TSEDSAVYYCARDGTFYGTYGYWYFDFWGQGTQVTVSS (SEQ ID NO 109) |
| Linker-Ck-Linker | Linker | GQAGQ *[Sfil* site] - GGGGSGGGGSGGGGS (SEQ ID NO 94) - KL [*HindIII* site] |
| | Ck | RTVAAPSVFIFPPSDEQLKSGTASVVCLLNNFYPREAKVQWKV DNALQSGNSQESVTEQDSKDSTYSLSSTLTLSKADYEKHKVYA CEVTHQGLSLPVTKSFNRGES (SEQ ID NO 95) |
| | Linker | LE *[Xhol* site] - GGGGSGGGGSGGGGS (SEQ ID NO 94) - TG *[Agel* site] |
| 2-12-C | VL | LTQPSSVSANPGGTVEITCSGGYSDYYGWYQQKAPGSAPVTVI YYNDKRPSNIPSRFSGSTSGSTGTLTITGVQAEDEAIYYCGGY DGSTDAGIFGAGTTLTVL (SEQ ID NO 116) |
| | Linker | GQSSRSSSGGGSSGGGGS (SEQ ID NO 118) |
| | VH | AVTLDESEGGLQTPGGALSLVCKAGFTFNNNGMAWVRQAPGKG LEFVAGISSGSYTYYGTAVKGRATISRDNGQSTVRLQLNNLRA |
| | | EDTATYFCAKDGSDYNWNSGADSIDAWGHGTEVIVSS (SEQ ID NO 117) |
| Full sequence [N'-(PV1 scFv) - linker-Ck-linker - (2-12-C scFv) - C'] | DIVMTQSPYSLAVSAGEKVTMSCRSSQSLYYSGIKKNLLAWYQQKPGQSPK LLIYFTSTRLPGVPDRFTGSGSGTDYTLTITSVQAEDMGHYFCQQGISTPL TFGDGTKLEIRGQSSRSSGGGGSSGGGGSQVQLKQSGAELVKPGASVKISC KTSGYTFTDGYMNWVEQKPGQGLEWIGRIDPDSGNTRYNQKFQGKATLTRD KSSSTVYMDLRSLTSEDSAVYYCARDGTFYGTYGYWYFDFWGQGTQVTVSS GQAGQGGGGSGGGGSGGGGSKLRTVAAPSVFIFPPSDEQLKSGTASVVCLL NNFYPREAKVQWKVDNALQSGNSQESVTEQDSKDSTYSLSSTLTLSKADYE KHKVYACEVTHQGLSLPVTKSFNRGESLEGGGGSGGGGSGGGGSTGLTQPS SVSANPGGTVEITCSGGYSDYYGWYQQKAPGSAPVTVIYYNDKRPSNIPSR FSGSTSGSTGTLTITGVQAEDEAIYYCGGYDGSTDAGIFGAGTTLTVLGQS SRSSSGGGSSGGGGSAVTLDESEGGLQTPGGALSLVCKASGFTFNNNGMAW VRQAPGKGLEFVAGISSGSYTYYGTAVKGRATISRDNGQSTVRLQLNNLRA EDTATYFCAKDGSDYNWNSGADSIDAWGHGTEVIVSS (SEQ ID NO 119) | |

### Example 4-2. Identification of binding ability to mouse CD4 T cells (in vitro)

An experiment was conducted to evaluate T cell binding ability of the anti-CD40L/anti-CD28 bispecific antibody (PV1x2-12-C) for mice prepared in Example 4-1. CD4 T cells were isolated from the mouse spleen and lymph nodes using the MACS method (mouse CD4 microbead, 130-117-043 Miltenyi Biotech). Then, to induce CD40L expression on the CD4 T cells, the cells were stimulated in vitro for 48 hours with plate-bound aCD3 (clone 145-2C11, BE0001-1, Bio X Cell, 10 µg/ml) and aCD28 (clone 37.51, 553294 BD Bioscience, 2 µg/ml).

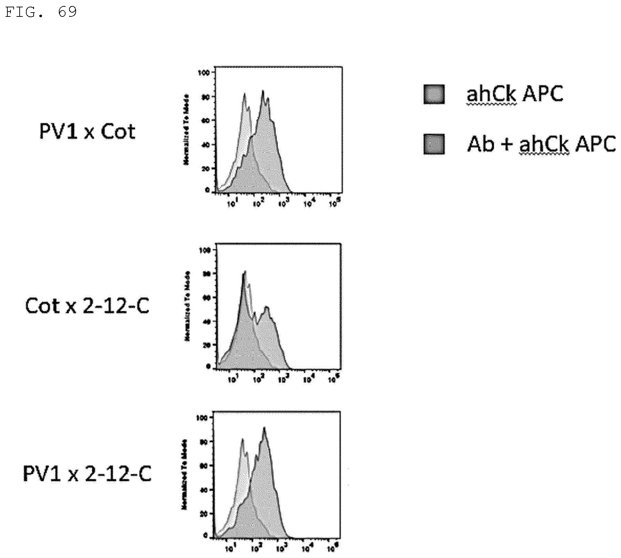

The PV1x2-12-C antibody was bound to the activated CD4 T cells, and then antibody binding was measured by flow cytometry using a fluorescently labeled anti-Ck antibody. As a result, it was identified that the PV1x2-12-C antibody bound to the activated mouse CD4 T cells (FIG. 69).

### Example 4-3. Identification of T-cell inhibitory ability (in vitro)

It was evaluated whether the anti-CD40L/anti-CD28 bispecific antibody (PV1x2-12-C) for mice could simultaneously bind to CD40L and CD28 on CD4 T cells to block CD40L-CD40 and CD28-B7 binding, and consequently inhibit activation of CD4 T cells.

Mouse bone marrow cells were isolated and cultured for 5 days with GMCSF (20 ng/ml, JW Creagene, rmGM-CSF) and IL-4 (20 ng/ml, JW Creagene, rmIL-4) to differentiate into dendritic cells. CD4 T cells were isolated from the mouse spleen and lymph nodes using the MACS method. The mouse CD4 T cells and the dendritic cells were then co-cultured, and the anti-mouse CD3 antibody (1 µg/ml) was added thereto for TCR stimulation. T cell inhibitory efficacy of the bispecific antibody was evaluated by adding the anti-CD40L/anti-CD28 bispecific antibody (PV1x2-12-C) for mice to the above co-culture conditions.

After 48 hours, the supernatant was separated and the T-cell activation cytokines, IFN-γ (IFN-γ ELISA set, BDB555138, BD Bioscience), and IL-2 (IL-2 ELISA set, BDB555148, BD Bioscience) were measured using an ELISA method. The results showed that secretion of IFN-γ and IL-2 in the group treated with PV1x2-12-C was significantly decreased compared to the negative control.

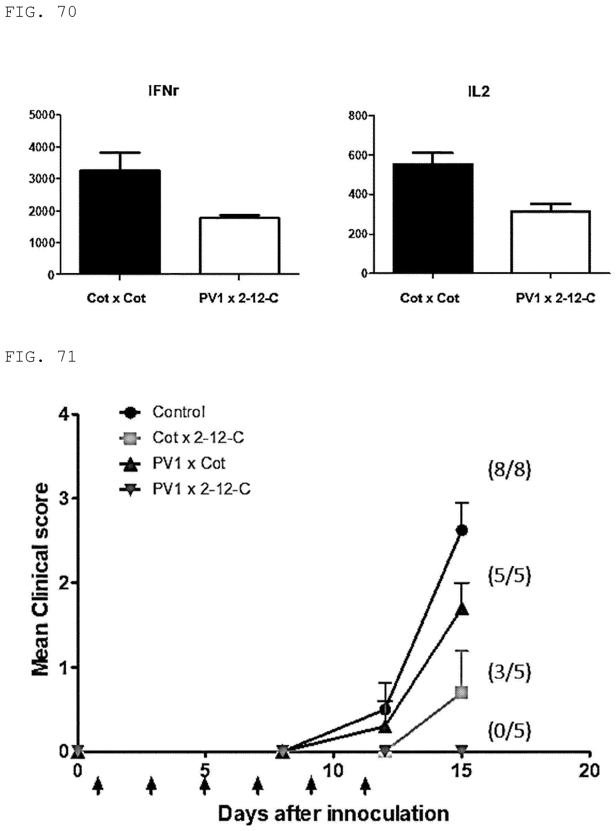

This suggests that, similarly to the experimental results of the anti-CD40L/anti-CD28 bispecific antibody that underwent humanization in Example 3, the anti-CD40L/anti-CD28 bispecific antibody for mice inhibited T cell activation by blocking CD40L and CD28 signaling on CD4 T cells (FIG. 70).

### Example 4-4. Evaluation of autoimmune disease suppressing ability

Since it was identified in Example 4-3 that the anti-CD40L/anti-CD28 bispecific antibody (PV1x2-12-C) for mice inhibited T cells to an extent similar to the anti-CD40L/anti-CD28 bispecific antibody that underwent humanization, an experiment was conducted to check whether the anti-CD40L/anti-CD28 bispecific antibody for mice can inhibit autoimmune T cells in vivo and thus suppress autoimmune diseases.

The experimental autoimmune encephalomyelitis (EAE) mouse model, which is an animal model for the central nervous system autoimmune disease multiple sclerosis, was established and efficacy of the anti-CD40LxCD28 antibody was verified. An autoimmune demyelination disease was induced in WT C57Bl/6 mice (C57BL/6, Orient Bio) by subcutaneous injection of the myelin oligodendrocyte glycoprotein (MOG) peptide (Peptron, 200 µg/mouse) along with a large amount of Complete Freunds Adjuvant (Incomplete Freud's Adjuvant (IFA) (F5506, Sigma-Aldrich) + killed Mycobacterium tuberculosis (231141, BD Biosciences), 1000 µg/animal) and pertussis toxin (P7208, Sigma-Aldrich). An experiment was then conducted to identify that the disease progression could be suppressed by intraperitoneal injection of the anti-CD40L/anti-CD28 bispecific antibody for mice (PV1x2-12-C). As single antibody controls, bispecific antibodies (Cotx2-12-C; PV1xCot, see Example 1-1) were used in which the respective single antibodies (PV1 and 2-12-C clones, scFv) were linked to an anti-cotinine antibody scFv (Korean Laid-Open Patent Publication KR 10-2019-0023084 A).

Each mouse received 200 µg of antibody via intraperitoneal injection 6 times at two-day intervals, and the disease progression was monitored over time. The results are shown in Figure 71.

As shown in FIG. 71, it was found that the control without antibody treatment exhibited disease progression as evidenced by an increase in clinical severity (mean clinical score) over time. It was identified that the groups treated with the single antibody controls (Cotx2-12-C; PV1xCot) exhibited partially suppressed disease progression compared to the control, indicating partial efficacy of the single antibodies. It was identified that the group treated with the bispecific antibody (PV1x2-12-C) exhibited significantly lower clinical severity, indicating that the bispecific antibody effectively suppresses autoimmune demyelinating diseases.

These experimental results should be construed as supporting excellent effects of the anti-CD40L/anti-CD28 bispecific antibody newly proposed herein, in which an antigen-binding fragment of an anti-CD40L antibody lacking an Fc region is linked to an antigen-binding fragment of an anti-CD28 antibody in a monovalent antibody form, wherein the excellent effects include the ability to effectively suppress autoimmune diseases by simultaneously inhibiting two co-stimulatory molecules without causing adverse effects such as thrombosis.

From the above description, those skilled in the art to which the present disclosure pertains will understand that the present disclosure may be implemented in other specific forms without changing the technical ideas or essential features. In this regard, the examples as described above should be understood as being illustrative in all respects and not restrictive. The scope of the present disclosure should be interpreted as including all modifications or variations derived from the meaning and scope of the appended claims and their equivalents, rather than the foregoing description.

## Claims

1. An anti-CD40L/anti-CD28 bispecific antibody, comprising:
(1) an antigen-binding fragment of an anti-CD40L antibody lacking an Fc region; and
(2) an antigen-binding fragment of an anti-CD28 antibody in a monovalent antibody form.

2. The anti-CD40L/anti-CD28 bispecific antibody of Claim 1, wherein
(1) the antigen-binding fragment of the anti-CD40L antibody is in the form of an anti-CD40L scFv, Fv, (scFv)₂, Fab, Fab' or F(ab')₂, or a single domain antibody lacking an Fc region, and
(2) the antigen-binding fragment of the anti-CD28 antibody is in the form of an anti-CD28 scFv, Fv, Fab or Fab', or a single domain antibody in a monovalent antibody form.

3. The anti-CD40L/anti-CD28 bispecific antibody of Claim 1, wherein
(1) the antigen-binding fragment of the anti-CD40L antibody is an anti-CD40L scFv, and
(2) the antigen-binding fragment of the anti-CD28 antibody is an anti-CD28 scFv.

4. The anti-CD40L/anti-CD28 bispecific antibody of Claim 1, wherein the anti-CD40L/anti-CD28 bispecific antibody is in a form in which an anti-CD40L scFv and an anti-CD28 scFv are linked to each other via a constant region (C kappa) of a light chain of an IgG antibody.

5. The anti-CD40L/anti-CD28 bispecific antibody of Claim 1, wherein
1) the antigen-binding fragment of the anti-CD40L antibody comprises:
L-CDR1 comprising the amino acid sequence of SEQ ID NO: 1 or 2;
L-CDR2 comprising the amino acid sequence of SEQ ID NO: 4 or 5;
L-CDR3 comprising the amino acid sequence of SEQ ID NO: 7 or 8;
H-CDR1 comprising the amino acid sequence of SEQ ID NO: 10 or 11;
H-CDR2 comprising the amino acid sequence of SEQ ID NO: 13 or 14; and
H-CDR3 comprising the amino acid sequence of SEQ ID NO: 16 or 17; and
2) the antigen-binding fragment of the anti-CD28 antibody comprises:
L-CDR1 comprising the amino acid sequence of SEQ ID NO: 65;
L-CDR2 comprising the amino acid sequence of SEQ ID NO: 66;
L-CDR3 comprising the amino acid sequence of SEQ ID NO: 67;
H-CDR1 comprising the amino acid sequence of SEQ ID NO: 68 or 69;
H-CDR2 comprising the amino acid sequence of SEQ ID NO: 70; and
H-CDR3 comprising the amino acid sequence of SEQ ID NO: 71.

6. The anti-CD40L/anti-CD28 bispecific antibody of Claim 5, wherein
1) the antigen-binding fragment of the anti-CD40L antibody comprises:
(1) L-CDR1 comprising the amino acid sequence of SEQ ID NO: 1, L-CDR2 comprising the amino acid sequence of SEQ ID NO: 4, L-CDR3 comprising the amino acid sequence of SEQ ID NO: 7, H-CDR1 comprising the amino acid sequence of SEQ ID NO: 10, H-CDR2 comprising the amino acid sequence of SEQ ID NO: 13, and H-CDR3 comprising the amino acid sequence of SEQ ID NO: 16; or
(2) L-CDR1 comprising the amino acid sequence of SEQ ID NO: 2, L-CDR2 comprising the amino acid sequence of SEQ ID NO: 5, L-CDR3 comprising the amino acid sequence of SEQ ID NO: 8, H-CDR1 comprising the amino acid sequence of SEQ ID NO: 11, H-CDR2 comprising the amino acid sequence of SEQ ID NO: 14, and H-CDR3 comprising the amino acid sequence of SEQ ID NO: 17;
2) the antigen-binding fragment of the anti-CD28 antibody comprises:
L-CDR1 comprising the amino acid sequence of SEQ ID NO: 65; L-CDR2 comprising the amino acid sequence of SEQ ID NO: 66; L-CDR3 comprising the amino acid sequence of SEQ ID NO: 67; H-CDR1 comprising the amino acid sequence of SEQ ID NO: 68 or 69; H-CDR2 comprising the amino acid sequence of SEQ ID NO: 70; and H-CDR3 comprising the amino acid sequence of SEQ ID NO: 71.

7. The anti-CD40L/anti-CD28 bispecific antibody of Claim 5, wherein
1) the antigen-binding fragment of the anti-CD40L antibody comprises:
(1) a light chain variable region comprising the amino acid sequence of SEQ ID NO: 47, 48, 49, or 128; and
(2) a heavy chain variable region comprising the amino acid sequence of SEQ ID NO: 51, 52, 53, 54, 55, or 56; and
2) the antigen-binding fragment of the anti-CD28 antibody comprises:
(1) a light chain variable region comprising the amino acid sequence of SEQ ID NO: 87, 88, 98, or 100; and
(2) a heavy chain variable region comprising the amino acid sequence of SEQ ID NO: 89, 90, 99, or 101.

8. The anti-CD40L/anti-CD28 bispecific antibody of Claim 5, wherein
1) the antigen-binding fragment of the anti-CD40L antibody comprises:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48 or 128; and a heavy chain variable region comprising the amino acid sequence of SEQ ID NO: 52, 53, 54, or 55; or
(3) a light chain variable region comprising the amino acid sequence of SEQ ID NO: 49; and a heavy chain variable region comprising the amino acid sequence of SEQ ID NO: 56, and
2) the antigen-binding fragment of the anti-CD28 antibody comprises:
(1) a light chain variable region comprising the amino acid sequence of SEQ ID NO: 87; and a heavy chain variable region comprising the amino acid sequence of SEQ ID NO: 89;
(2) a light chain variable region comprising the amino acid sequence of SEQ ID NO: 88; and a heavy chain variable region comprising the amino acid sequence of SEQ ID NO: 90;
(3) a light chain variable region comprising the amino acid sequence of SEQ ID NO: 98; and a heavy chain variable region comprising the amino acid sequence of SEQ ID NO: 99;
(4) a light chain variable region comprising the amino acid sequence of SEQ ID NO: 100; and a heavy chain variable region comprising the amino acid sequence of SEQ ID NO: 99; or
(5) a light chain variable region comprising the amino acid sequence of SEQ ID NO: 98; and a heavy chain variable region comprising the amino acid sequence of SEQ ID NO: 101.

9. The anti-CD40L/anti-CD28 bispecific antibody of Claim 5, wherein the anti-CD40L/anti-CD28 bispecific antibody is in a form in which an anti-CD40L scFv and an anti-CD28 scFv are linked to each other via a constant region (C kappa) of a light chain of an IgG antibody.

10. The anti-CD40L/anti-CD28 bispecific antibody of Claim 9, wherein the constant region of the light chain of the IgG antibody comprises the amino acid sequence of SEQ ID NO: 95.

11. A pharmaceutical composition for prevention or treatment of one or more diseases selected from the group consisting of autoimmune diseases and graft-versus-host disease, comprising the anti-CD40L/anti-CD28 bispecific antibody of any one of Claims 1 to 10.

12. The pharmaceutical composition of Claim 11, wherein the autoimmune disease is caused by T cell hyperactivation.

13. The pharmaceutical composition of Claim 11, wherein the autoimmune disease is one or more selected from the group consisting of rheumatoid arthritis, multiple sclerosis, systemic lupus erythematosus, type 1 diabetes, Crohn's disease, scleroderma, Sjogren's syndrome, psoriasis, inflammatory bowel disease, ulcerative colitis, ankylosing spondylitis, interstitial lung disease, uveitis, optic neuritis, peripheral neuropathies, sarcoidosis, antiphospholipid syndrome, inflammatory myopathies, Behcet's disease, alopecia totalis/universalis, pemphigus vulgaris, myasthenia gravis, Graves' disease, Hashimoto's thyroiditis, Guillain-Barre syndrome, Celiac disease, and pernicious anemia.

14. The pharmaceutical composition of Claim 11, wherein the graft-versus-host disease is one or more selected from the group consisting of acute graft-versus-host disease and chronic graft-versus-host disease.

15. An anti-CD40L antibody or an antigen-binding fragment thereof, comprising:
L-CDR1 comprising the amino acid sequence of SEQ ID NO: 1 or 2;
L-CDR2 comprising the amino acid sequence of SEQ ID NO: 4 or 5;
L-CDR3 comprising the amino acid sequence of SEQ ID NO: 7 or 8;
H-CDR1 comprising the amino acid sequence of SEQ ID NO: 10 or 11;
H-CDR2 comprising the amino acid sequence of SEQ ID NO: 13 or 14; and
H-CDR3 comprising the amino acid sequence of SEQ ID NO: 16 or 17.

16. The anti-CD40L antibody or an antigen-binding fragment thereof of Claim 15, wherein
(1) L-CDR1 comprising the amino acid sequence of SEQ ID NO: 1, L-CDR2 comprising the amino acid sequence of SEQ ID NO: 4, L-CDR3 comprising the amino acid sequence of SEQ ID NO: 7, H-CDR1 comprising the amino acid sequence of SEQ ID NO: 10, H-CDR2 comprising the amino acid sequence of SEQ ID NO: 13, and H-CDR3 comprising the amino acid sequence of SEQ ID NO: 16; or
(2) L-CDR1 comprising the amino acid sequence of SEQ ID NO: 2, L-CDR2 comprising the amino acid sequence of SEQ ID NO: 5, L-CDR3 comprising the amino acid sequence of SEQ ID NO: 8, H-CDR1 comprising the amino acid sequence of SEQ ID NO: 11, H-CDR2 comprising the amino acid sequence of SEQ ID NO: 14, and H-CDR3 comprising the amino acid sequence of SEQ ID NO: 17.

17. The anti-CD40L antibody or an antigen-binding fragment thereof of Claim 15, comprising:
a light chain variable region comprising the amino acid sequence of SEQ ID NO: 47, 48, 49, or 128; and
a heavy chain variable region comprising the amino acid sequence of SEQ ID NO: 51, 52, 53, 54, 55, or 56.

18. The anti-CD40L antibody or an antigen-binding fragment thereof of Claim 15, comprising:
(1) a light chain variable region comprising the amino acid sequence of SEQ ID NO: 47; and a heavy chain variable region comprising the amino acid sequence of SEQ ID NO: 51;
(2) a light chain variable region comprising the amino acid sequence of SEQ ID NO: 48 or 128; and a heavy chain variable region comprising the amino acid sequence of SEQ ID NO: 52, 53, 54, or 55; or
(3) a light chain variable region comprising the amino acid sequence of SEQ ID NO: 49; and a heavy chain variable region comprising the amino acid sequence of SEQ ID NO: 56.

19. The anti-CD40L antibody or an antigen-binding fragment thereof of Claim 15, wherein the anti-CD40L antibody or an antigen-binding fragment thereof lacks an Fc region.

20. The anti-CD40L antibody or an antigen-binding fragment thereof of Claim 15, wherein the anti-CD40L antibody or an antigen-binding fragment thereof is in the form of an anti-CD40L scFv, Fv, (scFv)₂, Fab, Fab' or F(ab')₂, or a single domain antibody lacking an Fc region.

21. The anti-CD40L antibody or an antigen-binding fragment thereof of Claim 15, wherein the anti-CD40L antibody or an antigen-binding fragment thereof is an anti-CD40L scFv.

22. A pharmaceutical composition for prevention or treatment of one or more diseases selected from the group consisting of autoimmune diseases and graft-versus-host disease, comprising the anti-CD40L antibody or an antigen-binding fragment thereof of any one of Claims 15 to 21.

23. The pharmaceutical composition of Claim 22, wherein the autoimmune disease is caused by T cell hyperactivation.

24. An anti-CD28 antibody or an antigen-binding fragment thereof, comprising:
L-CDR1 comprising the amino acid sequence of SEQ ID NO: 65;
L-CDR2 comprising the amino acid sequence of SEQ ID NO: 66;
L-CDR3 comprising the amino acid sequence of SEQ ID NO: 67;
H-CDR1 comprising the amino acid sequence of SEQ ID NO: 68 or 69;
H-CDR2 comprising the amino acid sequence of SEQ ID NO: 70; and
H-CDR3 comprising the amino acid sequence of SEQ ID NO: 71.

25. The anti-CD28 antibody or an antigen-binding fragment thereof of Claim 24, comprising:
(1) a light chain variable region comprising the amino acid sequence of SEQ ID NO: 87, 88, 98, or 100; and
(2) a heavy chain variable region comprising the amino acid sequence of SEQ ID NO: 89, 90, 99, or 101.

26. The anti-CD28 antibody or an antigen-binding fragment thereof of Claim 24, comprising:
(1) a light chain variable region comprising the amino acid sequence of SEQ ID NO: 87; and a heavy chain variable region comprising the amino acid sequence of SEQ ID NO: 89;
(2) a light chain variable region comprising the amino acid sequence of SEQ ID NO: 88; and a heavy chain variable region comprising the amino acid sequence of SEQ ID NO: 90;
(3) a light chain variable region comprising the amino acid sequence of SEQ ID NO: 98; and a heavy chain variable region comprising the amino acid sequence of SEQ ID NO: 99;
(4) a light chain variable region comprising the amino acid sequence of SEQ ID NO: 100; and a heavy chain variable region comprising the amino acid sequence of SEQ ID NO: 99; or
(5) a light chain variable region comprising the amino acid sequence of SEQ ID NO: 98; and a heavy chain variable region comprising the amino acid sequence of SEQ ID NO: 101.

27. The anti-CD28 antibody or an antigen-binding fragment thereof of Claim 24, wherein the antigen-binding fragment of the anti-CD28 antibody is in a monovalent antibody form.

28. The anti-CD28 antibody or an antigen-binding fragment thereof of Claim 24, wherein the antigen-binding fragment of the anti-CD28 antibody is in the form of an anti-CD28 scFv, Fv, Fab or Fab', or a single domain antibody in a monovalent antibody form.

29. The anti-CD28 antibody or an antigen-binding fragment thereof of Claim 24, wherein the anti-CD28 antibody or an antigen-binding fragment thereof is an anti-CD28 scFv.

30. A pharmaceutical composition for prevention or treatment of one or more diseases selected from the group consisting of autoimmune diseases and graft-versus-host disease, comprising the anti-CD40L antibody or an antigen-binding fragment thereof of any one of Claims 24 to 29.

31. The pharmaceutical composition of Claim 30, wherein the autoimmune disease is caused by T cell hyperactivation.
